# EUROPEAN PATENT APPLICATION

(11) **EP 3 839 073 A1**
(43) Date of publication of application: **23.06.2021**
(21) Application number: 19219124.5
(22) Date of filing: 20.12.2019
(51) Int. Cl.: C12Q 1/6895

(54) **ENHANCED DISEASE RESISTANCE OF MAIZE TO NORTHERN CORN LEAF BLIGHT BY A QTL ON CHROMOSOME 4**

(71) Applicant: KWS SAAT SE & Co. KGaA, 37574 Einbeck (DE)
(72) Inventor: SCHEUERMANN, Daniela, 37574 Einbeck (DE); PRESTERL, Thomas, 37574 Einbeck (DE); KESSEL, Bettina, 37574 Einbeck (DE); STAHL, Dietmar, 37574 Einbeck (DE); STIRNWEIS, Daniel Fabian, 37085 Göttingen (DE)

(57) **Abstract**

The present invention relates to maize plants having increased pathogen resistance or tolerance, in particular increased resistance or tolerance to pathogens causing Northern Corn Leaf Blight, i.e. *Exserohilum turcicum.* Such maize plants can be characterized as having a particular QTL allele comprising one or more resistance gene, e.g. Zm00001d049159, or particular molecular markers on chromosome 4. The invention further relates to methods for generating such maize plants, as well as methods for identifying such maize plants.

SEQUENCE LISTING

<110>  KWS SAAT SE & Co. KGaA

<120>  ENHANCED DISEASE RESISTANCE OF MAIZE TO NORTHERN CORN LEAF BLIGHT
        BY A QTL ON CHROMOSOME 4

<130>  KWS0337EP

<160>  33

<170>  PatentIn version 3.5

<210>  1
<211>  2718
<212>  DNA
<213>  Artificial Sequence

<220>
<223>  cDNA of Zm00001d049121 of genotype H102

<400>  1
atggcggagg ccatcgtggg gtcgatgctg tggaagctgc agcaggtggc ggtgagtgag      60

gcgcggacgc tggtggccgt gaacgaggac atccggagcc tccgggacaa gctcatgtgg     120

atgcaggcgt tcctgcacga cgtccagccg tcgcgccgcg tgcagcccaa cgagctcatc     180

aaggtgtggc tgcagcagac ccgcgacgcc gtcttcgacg ccgaagacgc cgtcgaccac     240

tttaccaccc aagtggttgt ccgtcgcgac ctctccagca ggatcaggct gatcaatgga     300

aggctggagg gaatcattgc caacaaggac aggtacaggt tgggagagtc tactacgctg     360

ggagcaattt ggaggccatc tagctccacg tcaccgctct cggaaatgat ggacgaggtg     420

gtgctgccac tggtgggacg agaggaactg gtgcgcaatc ttaagaattg gctttataaa     480

ggcagcggga atcataaaaa tgtgatcacc gtgacggggg aaagtggagt cggcaagaca     540

aagctggtga ggaacttgta cgacagcaag cagaccctgt ctcactttga catctacgag     600

tgggtgagct ttgggcccaa tctcagtgcc tctgatgtcc tcaagatcat catcagacgc     660

attacagatg gagaagaatg ttccaaggac aacatagaga ggaagctgcg ggagatactg     720

aaagaaaaga agtatctgtt ggtgatagat gctgagctca gtaactcgga gtggaatcgc     780

attttcgcta tgctccctga ccccaaggat gtggatgtcg atgctgccag cagaatagtg     840

aggatttctc agattccgcc acataagcca cccccacact atcaagaagc caaacttgag     900

gttccgaagt tttatgacca agaagtagtc atcaatctgt tcaaggaaac cttccaatca     960

tgtggcagaa atgaacttcc tgacgaagca ataagagaat acagacagag aatcttggac    1020

aacacaaaag ggttgccgct ggcaatagtt cttctgtcag gccttctgcg taccaaggag    1080

taccctagtg aatgggagac ggtgttcgag cacctggaca ggatgcagtc aaagcagatc    1140

gacaagattc tatccctctg cttcgatgac cttcactatg acctgaaatc gtgcctcctc    1200

tactttgctg cattgccagt gaataccttt atcagggcaa gcaacatcgt gtgcatgtgg    1260

atggcggagg gcttcctggt acccaaggcc acaacggtgg agaaagtagg agagcagtac 1320

ctgatggagc tgatagatag gcgcctcgtc aacttggcgc cagtgggcta caatgttcct 1380

gggtacgagc gtgtagctgt tcagagcaaa gtacacgctt tcctgcagct cgaagcacag 1440

gagcaatgct tcgtggagat ccacagtggt gacgacatcc cggctttgtc ggatgcccga 1500

cgcttgtcac tccagaacca caaagacaag tatgcagcac tgtcacaccc gctgccgaag 1560

ctgcgagcca tcctgtcaaa ctttgagacg gagcaagagg ctgcacaagt tgctggcgag 1620

aagccaccag gtgaagctgg agaagggcaa catggaaatg gatgccggcc tggtttcaac 1680

aaaaagaaaa cgcaagccaa gtcttgtgtg caggacctgc tacggaactc aaggttcctc 1740

cgtgtcatcc atttcaacgg cctcgaggtg gacaaagagc ttcctgacga aatcggcaaa 1800

gttgtgaacc tgcagtacgt ccgagtgacc tcctgctcct tggagaagat gccgccgtcg 1860

attggcaggc tgtgcaacct ccagactctt gatgtccggg gcacctcggt ccgaagcttg 1920

ccagatgggt tctggaggat tcagacgctc cgtcatgtgc tgggcgaccg tctcatcctg 1980

cctaaacgtg ttggtgactt gaggaatctg cagacactca acagcgtgaa gcccgactat 2040

ggtgaccccc atgcctggga cgacaagacc ttcagccaca tgatccgcct cttgtacttg 2100

cacatccggc gcggtgagag tctgggcgtc caggaaatct tctccaaccg caagaacaag 2160

gctgccttgg taaaagcagt atccattctc aagtaccttg tcgtcctcat cataaaagct 2220

cctgtgatcc cctcagaagt gttcactagg tccaacctcc aacgtctcaa ggcaatggaa 2280

ctggtcggga agcttgacct tcctaaaaat ggcatccctg agatggatgt ccgcaatcgc 2340

ctcccaaacc tcaagaagct gaggcttatg aagacaatgg tgtcacagga attcattaac 2400

caacttggag gcctacagtt cctttccacc cttgaactaa ccagcaattc tttcagggag 2460

cttgtcttca cggaaggatt tcgcagcctc aaagaactga cattgcatga ggagacactc 2520

gagaggttgg agatacatga gtcggcactt cccaagcttg gggatctgga tattgtcggc 2580

catagggaca agctccatgt tgttatccat ggtcattccg tgctcgtgca acacattgaa 2640

gcagaggatg aagatatttt taaggttacg gaagtaataa cgacgcaaca agtccaagtg 2700

gaagacaaca aagtctga 2718

<210> 2
<211> 905
<212> PRT
<213> Zea mays

<400> 2

Met Ala Glu Ala Ile Val Gly Ser Met Leu Trp Lys Leu Gln Gln Val
1               5                   10                  15

Ala Val Ser Glu Ala Arg Thr Leu Val Ala Val Asn Glu Asp Ile Arg
            20                  25                  30
Ser Leu Arg Asp Lys Leu Met Trp Met Gln Ala Phe Leu His Asp Val
        35                  40                  45
Gln Pro Ser Arg Arg Val Gln Pro Asn Glu Leu Ile Lys Val Trp Leu
    50                  55                  60
Gln Gln Thr Arg Asp Ala Val Phe Asp Ala Glu Asp Ala Val Asp His
65                  70                  75                  80
Phe Thr Thr Gln Val Val Val Arg Arg Asp Leu Ser Ser Arg Ile Arg
                85                  90                  95
Leu Ile Asn Gly Arg Leu Glu Gly Ile Ile Ala Asn Lys Asp Arg Tyr
            100                 105                 110
Arg Leu Gly Glu Ser Thr Thr Leu Gly Ala Ile Trp Arg Pro Ser Ser
        115                 120                 125
Ser Thr Ser Pro Leu Ser Glu Met Met Asp Glu Val Val Leu Pro Leu
    130                 135                 140
Val Gly Arg Glu Glu Leu Val Arg Asn Leu Lys Asn Trp Leu Tyr Lys
145                 150                 155                 160
Gly Ser Gly Asn His Lys Asn Val Ile Thr Val Thr Gly Glu Ser Gly
                165                 170                 175
Val Gly Lys Thr Lys Leu Val Arg Asn Leu Tyr Asp Ser Lys Gln Thr
            180                 185                 190
Leu Ser His Phe Asp Ile Tyr Glu Trp Val Ser Phe Gly Pro Asn Leu
        195                 200                 205
Ser Ala Ser Asp Val Leu Lys Ile Ile Ile Arg Arg Ile Thr Asp Gly
    210                 215                 220
Glu Glu Cys Ser Lys Asp Asn Ile Glu Arg Lys Leu Arg Glu Ile Leu
225                 230                 235                 240
Lys Glu Lys Lys Tyr Leu Leu Val Ile Asp Ala Glu Leu Ser Asn Ser
                245                 250                 255
Glu Trp Asn Arg Ile Phe Ala Met Leu Pro Asp Pro Lys Asp Val Asp
            260                 265                 270

Val Asp Ala Ala Ser Arg Ile Val Arg Ile Ser Gln Ile Pro Pro His
        275                 280                 285

Lys Pro Pro Pro His Tyr Gln Glu Ala Lys Leu Glu Val Pro Lys Phe
    290                 295                 300

Tyr Asp Gln Glu Val Val Ile Asn Leu Phe Lys Glu Thr Phe Gln Ser
305                 310                 315                 320

Cys Gly Arg Asn Glu Leu Pro Asp Glu Ala Ile Arg Glu Tyr Arg Gln
                325                 330                 335

Arg Ile Leu Asp Asn Thr Lys Gly Leu Pro Leu Ala Ile Val Leu Leu
            340                 345                 350

Ser Gly Leu Leu Arg Thr Lys Glu Tyr Pro Ser Glu Trp Glu Thr Val
        355                 360                 365

Phe Glu His Leu Asp Arg Met Gln Ser Lys Gln Ile Asp Lys Ile Leu
    370                 375                 380

Ser Leu Cys Phe Asp Asp Leu His Tyr Asp Leu Lys Ser Cys Leu Leu
385                 390                 395                 400

Tyr Phe Ala Ala Leu Pro Val Asn Thr Phe Ile Arg Ala Ser Asn Ile
                405                 410                 415

Val Cys Met Trp Met Ala Glu Gly Phe Leu Val Pro Lys Ala Thr Thr
            420                 425                 430

Val Glu Lys Val Gly Glu Gln Tyr Leu Met Glu Leu Ile Asp Arg Arg
        435                 440                 445

Leu Val Asn Leu Ala Pro Val Gly Tyr Asn Val Pro Gly Tyr Glu Arg
    450                 455                 460

Val Ala Val Gln Ser Lys Val His Ala Phe Leu Gln Leu Glu Ala Gln
465                 470                 475                 480

Glu Gln Cys Phe Val Glu Ile His Ser Gly Asp Asp Ile Pro Ala Leu
                485                 490                 495

Ser Asp Ala Arg Arg Leu Ser Leu Gln Asn His Lys Asp Lys Tyr Ala
            500                 505                 510

Ala Leu Ser His Pro Leu Pro Lys Leu Arg Ala Ile Leu Ser Asn Phe
        515                 520                 525

Glu Thr Glu Gln Glu Ala Ala Gln Val Ala Gly Glu Lys Pro Pro Gly
    530                 535                 540

Glu Ala Gly Glu Gly Gln His Gly Asn Gly Cys Arg Pro Gly Phe Asn
545                 550                 555                 560

Lys Lys Lys Thr Gln Ala Lys Ser Cys Val Gln Asp Leu Leu Arg Asn
                565                 570                 575

Ser Arg Phe Leu Arg Val Ile His Phe Asn Gly Leu Glu Val Asp Lys
            580                 585                 590

Glu Leu Pro Asp Glu Ile Gly Lys Val Val Asn Leu Gln Tyr Val Arg
        595                 600                 605

Val Thr Ser Cys Ser Leu Glu Lys Met Pro Pro Ser Ile Gly Arg Leu
    610                 615                 620

Cys Asn Leu Gln Thr Leu Asp Val Arg Gly Thr Ser Val Arg Ser Leu
625                 630                 635                 640

Pro Asp Gly Phe Trp Arg Ile Gln Thr Leu Arg His Val Leu Gly Asp
                645                 650                 655

Arg Leu Ile Leu Pro Lys Arg Val Gly Asp Leu Arg Asn Leu Gln Thr
            660                 665                 670

Leu Asn Ser Val Lys Pro Asp Tyr Gly Asp Pro His Ala Trp Asp Asp
        675                 680                 685

Lys Thr Phe Ser His Met Ile Arg Leu Leu Tyr Leu His Ile Arg Arg
    690                 695                 700

Gly Glu Ser Leu Gly Val Gln Glu Ile Phe Ser Asn Arg Lys Asn Lys
705                 710                 715                 720

Ala Ala Leu Val Lys Ala Val Ser Ile Leu Lys Tyr Leu Val Val Leu
                725                 730                 735

Ile Ile Lys Ala Pro Val Ile Pro Ser Glu Val Phe Thr Arg Ser Asn
            740                 745                 750

Leu Gln Arg Leu Lys Ala Met Glu Leu Val Gly Lys Leu Asp Leu Pro
        755                 760                 765

Lys Asn Gly Ile Pro Glu Met Asp Val Arg Asn Arg Leu Pro Asn Leu

770                 775                 780

Lys Lys Leu Arg Leu Met Lys Thr Met Val Ser Gln Glu Phe Ile Asn
785                 790                 795                 800

Gln Leu Gly Gly Leu Gln Phe Leu Ser Thr Leu Glu Leu Thr Ser Asn
                805                 810                 815

Ser Phe Arg Glu Leu Val Phe Thr Glu Gly Phe Arg Ser Leu Lys Glu
            820                 825                 830

Leu Thr Leu His Glu Glu Thr Leu Glu Arg Leu Glu Ile His Glu Ser
        835                 840                 845

Ala Leu Pro Lys Leu Gly Asp Leu Asp Ile Val Gly His Arg Asp Lys
    850                 855                 860

Leu His Val Val Ile His Gly His Ser Val Leu Val Gln His Ile Glu
865                 870                 875                 880

Ala Glu Asp Glu Asp Ile Phe Lys Val Thr Glu Val Ile Thr Thr Gln
                885                 890                 895

Gln Val Gln Val Glu Asp Asn Lys Val
            900                 905

<210>  3
<211>  1089
<212>  DNA
<213>  Artificial Sequence

<220>
<223>  cDNA of Zm00001d049113 of genotype H102

<400>  3
atggatcagt ccaacattag caacaagcac atgacgatga caaccctaat gctcctcctc      60

tgtgtcatag ccattgcaga ttgcatttgc cacgccgcag tggcagcccg ggtggagcca     120

tccaccaccg tcggcagaac tacaggagga gacgaggcga tgatgatggc gaggtacaag     180

aagtggatgg cgcagtatcg ccggaagtac aaggacgacg ccgagaaagc acaccgtttc     240

caggtattca aggcgaacgc ggagtttatt gacaggtcta acgctggagg caagaagaag     300

tacgtccttg ggaccaacca gtttgccgac ctgaccagca aagagttcgc ggccatgtac     360

accggtttga ggaaaccggc ggcggtgcct tccggggcca agcagatccc tgcaggttcc     420

aagtaccaga attttacgcg cctagatgat gatgtccagg ttgattggag gcagcagggt     480

gctgtcactc ctgtcaagaa ccaaggccaa tgtggctgtt gctgggcgtt ctctgcagta     540

ggtgccatgg aaggtttgat catgataacg acaggaaacc tggtctccct gtcggagcag     600

cagattctag actgcgacga gtcagacggg aaccagggct gcaacggtgg ctacatggac 660

aacgccttcc agtacgtcat caacaatggc ggcgtcacca ctgaggacgc ctacccttac 720

tctgcagtcc aagggacgtg ccaaaacgtc cagccagccg ccaccatcag cggcttccag 780

gacctgccca gcggcgacga gaacgcgctc gccaacgcag tcgccaacca gccggtgtct 840

gttggcgtcg acggcggatc gagtcctttc cagttttacc agggtggcat atacgacggg 900

gatggctgtg gcacggacat gaaccatgca gtgacggcga tcggctacgg cgccgatgac 960

cagggaaccc agtactggat cctcaagaac tcctggggca caggatgggg tgagaacggt 1020

ttcatgcagc tacagatggg cgtcggcgcc tgtggtatct ccacgatggc ctcctaccca 1080

actccatga 1089

<210> 4
<211> 362
<212> PRT
<213> Zea mays

<400> 4

Met Asp Gln Ser Asn Ile Ser Asn Lys His Met Thr Met Thr Thr Leu
1               5                   10                  15

Met Leu Leu Leu Cys Val Ile Ala Ile Ala Asp Cys Ile Cys His Ala
            20                  25                  30

Ala Val Ala Ala Arg Val Glu Pro Ser Thr Thr Val Gly Arg Thr Thr
        35                  40                  45

Gly Gly Asp Glu Ala Met Met Met Ala Arg Tyr Lys Lys Trp Met Ala
    50                  55                  60

Gln Tyr Arg Arg Lys Tyr Lys Asp Asp Ala Glu Lys Ala His Arg Phe
65                  70                  75                  80

Gln Val Phe Lys Ala Asn Ala Glu Phe Ile Asp Arg Ser Asn Ala Gly
                85                  90                  95

Gly Lys Lys Lys Tyr Val Leu Gly Thr Asn Gln Phe Ala Asp Leu Thr
            100                 105                 110

Ser Lys Glu Phe Ala Ala Met Tyr Thr Gly Leu Arg Lys Pro Ala Ala
        115                 120                 125

Val Pro Ser Gly Ala Lys Gln Ile Pro Ala Gly Ser Lys Tyr Gln Asn
    130                 135                 140

Phe Thr Arg Leu Asp Asp Asp Val Gln Val Asp Trp Arg Gln Gln Gly
145                 150                 155                 160

Ala Val Thr Pro Val Lys Asn Gln Gly Gln Cys Gly Cys Cys Trp Ala
                165                 170                 175

Phe Ser Ala Val Gly Ala Met Glu Gly Leu Ile Met Ile Thr Thr Gly
            180                 185                 190

Asn Leu Val Ser Leu Ser Glu Gln Gln Ile Leu Asp Cys Asp Glu Ser
        195                 200                 205

Asp Gly Asn Gln Gly Cys Asn Gly Gly Tyr Met Asp Asn Ala Phe Gln
    210                 215                 220

Tyr Val Ile Asn Asn Gly Gly Val Thr Thr Glu Asp Ala Tyr Pro Tyr
225                 230                 235                 240

Ser Ala Val Gln Gly Thr Cys Gln Asn Val Gln Pro Ala Ala Thr Ile
                245                 250                 255

Ser Gly Phe Gln Asp Leu Pro Ser Gly Asp Glu Asn Ala Leu Ala Asn
            260                 265                 270

Ala Val Ala Asn Gln Pro Val Ser Val Gly Val Asp Gly Gly Ser Ser
        275                 280                 285

Pro Phe Gln Phe Tyr Gln Gly Gly Ile Tyr Asp Gly Asp Gly Cys Gly
    290                 295                 300

Thr Asp Met Asn His Ala Val Thr Ala Ile Gly Tyr Gly Ala Asp Asp
305                 310                 315                 320

Gln Gly Thr Gln Tyr Trp Ile Leu Lys Asn Ser Trp Gly Thr Gly Trp
                325                 330                 335

Gly Glu Asn Gly Phe Met Gln Leu Gln Met Gly Val Gly Ala Cys Gly
            340                 345                 350

Ile Ser Thr Met Ala Ser Tyr Pro Thr Pro
        355                 360

<210>  5
<211>  2373
<212>  DNA
<213>  Artificial Sequence

<220>
<223>  cDNA of Zm00001d049145 of genotype H102

<400> 5
atgccgccgc cgcgccggct ccacctccat gccgccttcc tcctcctgcc catcttcctg 60
ctcgcgacgt ggcggcccgc ggcggcgtcc gcactctcca ccttcgcgct ggccaaggcg 120
gacgccacca ccatagtgtg cgcgctcctg ccgtccgcgg cgtcgcctct gctggtcgac 180
ctcaactgca cggcggccgg cggcgaccac gcgcgccagg agacgtaccc gtcctcgcac 240
cccttcgccg cgctggccgg aggggaggac ttcctctgcg ccgtggggcc ctccggcgag 300
cgcgcgggcg acgtggacat gcgctggtgg gacctgtccg gcgacggtag caacaggtcc 360
aagcgggtct acatgggccc gccgctccgg gcgctgtcct cgagcgagta ccgcgtctgc 420
ggggtgctcc acagcgggga gctccactgc tggcgctggc gcggcctccg catctccagg 480
gacctccggt tcgtcgccgc cgccgtcggg gacggattcg tgtgcgccat cctcaagggg 540
acgccggcct ccatccgctg cttcggcaac gacacagcgg ccccggccgt cgccggcgcg 600
ccgacgaacg ggagcttcga cgtcgtggcc gcttgcgccc accgcgcgtg cgcgctgtcc 660
acggcggggg cgctcacgtg ctggggccgc gacccgccgc gcgtcggccc gggcgatgcg 720
gcggccaccg ggtacgcggc gctggcattg gggaggacgg gcgtctgcgg cctgcgcacc 780
aatttcacca tccgctgctt cggcgacggc gtggcccacc cgccgcccga cctcgcgcac 840
gcccagttcc tcgacgtgcg cgcgcacggc aaggccttct gcggcgtgct catggccaac 900
tactccctcg tctgctgggg cggccctgag ttcaacacca ccaaccgcct cgtcttcggc 960
cgcgtcatgc cgggcccctg cctgcccatg tcctcgtgcc aatgcggcgt cttgccgggc 1020
tccgccaact tctgcgacga cagccgctgc gtctgcgtcg actgcgcgtt cgagctcaac 1080
gtcgccaggc ccaacgtgtc ggtcctcccc gggaagggag gttggagcag gaggaccatg 1140
tggattgcaa tcgccgcgtc ggccgccgtt ttcctcgtgt tctttgtggc attccagttc 1200
gcgctggtcc tatggtgtcg tcgccgccgc cgccgccgca ggaacgaact ggatgcctcc 1260
gctggtgtgc agcagtcgct gatggcgccg aggctcgggt ccagcaggag caagggcccg 1320
ggcagcgtgg tggagcactt cacgctggac atgctccagg ccgcgacgga cgggttctcc 1380
gacgacagcc ggatcggaac cgggagcttc gggtccgtgt accgcggcac cctacccgac 1440
ggccgcgagg tcgcgatcaa gcgcgccgag gactcggcca aggcgtcgag ctcgatggcg 1500
cgcccggcgc ggcgccggga ccgcgagacg gcgttcaact cggagctgac cgcgctggcg 1560
cgcgccaacc acaagaacat cgtgtgcctg ctgggctgct gcgcggactc cggcgagcgc 1620
gtgctggtgt tcgagttcat ggcgaacggc acgctgcacg accagctcca cagccgcagc 1680
cccatggctg cggccgtgtc gtcgtggcgc ggccgcctca ccatcgcgct gggcgcggcg 1740
cggggcatcg agtacatgca cgtctacgcc gtgccgccca tcatccaccg cgacgtcaag 1800
tccgccaaca tcctgctcga cgacgcgtgg acggccaaga tcgccgactt cgggctgtcc 1860

tccgtcctgg accccggcgt gggcgcctgc ggcgacggca cgccgcagga gcccctctac 1920

accggcggca cggtggggta catggacccg gagtactacc ggctgcagca cctgacggac 1980

aagagcgacg tgtacagctt cggcgtggtg ctcctggagc tcatgtccgg gtgccgcgtc 2040

gtgcagcgct acgccgagag cgtgacgccc aagaacgtgg tggagttcgc ggtgccgcac 2100

atcctcgccg acgacgtcgc gcgcgtgctc gacccgcgcc tgcccgcgcc cacgcccgac 2160

gaagccgagg cgctggccta cgtgggctac ctcgccgccg actgcgtggg gccagtgggg 2220

tgcgaccgcc cgtccatgac cgaggtcgtg gacgccctgg agcgcgccct cgcggcctgc 2280

ggggctgggc cgctctcccg cgccggcacc ggccgccgcc ccgtgctctc ccgctccggc 2340

accgaccagt tcgacctcac cgacaccgac tag 2373

<210> 6
<211> 790
<212> PRT
<213> Zea mays

<400> 6

Met Pro Pro Pro Arg Arg Leu His Leu His Ala Ala Phe Leu Leu Leu
1               5                   10                  15

Pro Ile Phe Leu Leu Ala Thr Trp Arg Pro Ala Ala Ala Ser Ala Leu
            20                  25                  30

Ser Thr Phe Ala Leu Ala Lys Ala Asp Ala Thr Thr Ile Val Cys Ala
        35                  40                  45

Leu Leu Pro Ser Ala Ala Ser Pro Leu Leu Val Asp Leu Asn Cys Thr
    50                  55                  60

Ala Ala Gly Gly Asp His Ala Arg Gln Glu Thr Tyr Pro Ser Ser His
65                  70                  75                  80

Pro Phe Ala Ala Leu Ala Gly Gly Glu Asp Phe Leu Cys Ala Val Gly
                85                  90                  95

Pro Ser Gly Glu Arg Ala Gly Asp Val Asp Met Arg Trp Trp Asp Leu
            100                 105                 110

Ser Gly Asp Gly Ser Asn Arg Ser Lys Arg Val Tyr Met Gly Pro Pro
        115                 120                 125

Leu Arg Ala Leu Ser Ser Ser Glu Tyr Arg Val Cys Gly Val Leu His
    130                 135                 140

Ser Gly Glu Leu His Cys Trp Arg Trp Arg Gly Leu Arg Ile Ser Arg
145                 150                 155                 160

Asp Leu Arg Phe Val Ala Ala Ala Val Gly Asp Gly Phe Val Cys Ala
                165                 170                 175

Ile Leu Lys Gly Thr Pro Ala Ser Ile Arg Cys Phe Gly Asn Asp Thr
            180                 185                 190

Ala Ala Pro Ala Val Ala Gly Ala Pro Thr Asn Gly Ser Phe Asp Val
        195                 200                 205

Val Ala Ala Cys Ala His Arg Ala Cys Ala Leu Ser Thr Ala Gly Ala
    210                 215                 220

Leu Thr Cys Trp Gly Arg Asp Pro Pro Arg Val Gly Pro Gly Asp Ala
225                 230                 235                 240

Ala Ala Thr Gly Tyr Ala Ala Leu Ala Leu Gly Glu Asp Gly Val Cys
                245                 250                 255

Gly Leu Arg Thr Asn Phe Thr Ile Arg Cys Phe Gly Asp Gly Val Ala
            260                 265                 270

His Pro Pro Pro Asp Leu Ala His Ala Gln Phe Leu Asp Val Arg Ala
        275                 280                 285

His Gly Lys Ala Phe Cys Gly Val Leu Met Ala Asn Tyr Ser Leu Val
    290                 295                 300

Cys Trp Gly Gly Pro Glu Phe Asn Thr Thr Asn Arg Leu Val Phe Gly
305                 310                 315                 320

Arg Val Met Pro Gly Pro Cys Leu Pro Met Ser Ser Cys Gln Cys Gly
                325                 330                 335

Val Leu Pro Gly Ser Ala Asn Phe Cys Asp Asp Ser Arg Cys Val Cys
            340                 345                 350

Val Asp Cys Ala Phe Glu Leu Asn Val Ala Arg Pro Asn Val Ser Val
        355                 360                 365

Leu Pro Gly Lys Gly Gly Trp Ser Arg Arg Thr Met Trp Ile Ala Ile
    370                 375                 380

Ala Ala Ser Ala Ala Val Phe Leu Val Phe Phe Val Ala Phe Gln Phe
385                 390                 395                 400

Ala Leu Val Leu Trp Cys Arg Arg Arg Arg Arg Arg Arg Arg Asn Glu
                    405                 410                 415

Leu Asp Ala Ser Ala Gly Val Gln Gln Ser Leu Met Ala Pro Arg Leu
                420                 425                 430

Gly Ser Ser Arg Ser Lys Gly Pro Gly Ser Val Val Glu His Phe Thr
            435                 440                 445

Leu Asp Met Leu Gln Ala Ala Thr Asp Gly Phe Ser Asp Asp Ser Arg
    450                 455                 460

Ile Gly Thr Gly Ser Phe Gly Ser Val Tyr Arg Gly Thr Leu Pro Asp
465                 470                 475                 480

Gly Arg Glu Val Ala Ile Lys Arg Ala Glu Asp Ser Ala Lys Ala Ser
                    485                 490                 495

Ser Ser Met Ala Arg Pro Ala Arg Arg Arg Asp Arg Glu Thr Ala Phe
                500                 505                 510

Asn Ser Glu Leu Thr Ala Leu Ala Arg Ala Asn His Lys Asn Ile Val
            515                 520                 525

Cys Leu Leu Gly Cys Cys Ala Asp Ser Gly Glu Arg Val Leu Val Phe
    530                 535                 540

Glu Phe Met Ala Asn Gly Thr Leu His Asp Gln Leu His Ser Arg Ser
545                 550                 555                 560

Pro Met Ala Ala Ala Val Ser Ser Trp Arg Gly Arg Leu Thr Ile Ala
                    565                 570                 575

Leu Gly Ala Ala Arg Gly Ile Glu Tyr Met His Val Tyr Ala Val Pro
                580                 585                 590

Pro Ile Ile His Arg Asp Val Lys Ser Ala Asn Ile Leu Leu Asp Asp
            595                 600                 605

Ala Trp Thr Ala Lys Ile Ala Asp Phe Gly Leu Ser Ser Val Leu Asp
    610                 615                 620

Pro Gly Val Gly Ala Cys Gly Asp Gly Thr Pro Gln Glu Pro Leu Tyr
625                 630                 635                 640

Thr Gly Gly Thr Val Gly Tyr Met Asp Pro Glu Tyr Tyr Arg Leu Gln
                    645                 650                 655

His Leu Thr Asp Lys Ser Asp Val Tyr Ser Phe Gly Val Val Leu Leu
            660                 665                 670

Glu Leu Met Ser Gly Cys Arg Val Val Gln Arg Tyr Ala Glu Ser Val
        675                 680                 685

Thr Pro Lys Asn Val Val Glu Phe Ala Val Pro His Ile Leu Ala Asp
    690                 695                 700

Asp Val Ala Arg Val Leu Asp Pro Arg Leu Pro Ala Pro Thr Pro Asp
705                 710                 715                 720

Glu Ala Glu Ala Leu Ala Tyr Val Gly Tyr Leu Ala Ala Asp Cys Val
                725                 730                 735

Gly Pro Val Gly Cys Asp Arg Pro Ser Met Thr Glu Val Val Asp Ala
            740                 745                 750

Leu Glu Arg Ala Leu Ala Ala Cys Gly Ala Gly Pro Leu Ser Arg Ala
        755                 760                 765

Gly Thr Gly Arg Arg Pro Val Leu Ser Arg Ser Gly Thr Asp Gln Phe
    770                 775                 780

Asp Leu Thr Asp Thr Asp
785                 790

<210>  7
<211>  1344
<212>  DNA
<213>  Artificial Sequence

<220>
<223>  cDNA of Zm00001d049148 of genotype H102

<400>  7
atgcaggcct cttccctccg gcgagccctc cttctgcggg gaccgcgagg cgcccagctc      60

ttccccattc ccgtaggcaa caagccccgt gccttctcct ccgcgtcccc cgcggcagcg     120

cgctctccca aagacgctca taatgcgccg ccgccgatta tgccgacgct gccgtggggt     180

gatgcgctcg cagccacgca gcgcaccttc tgcctgccgc tcgctgggcg cgtcctcgcc     240

gcctcggcga ccgagaacgc ggccgtggcc cccgtcgcag tctacgcctc gctggccctg     300

gccgccgccg gcgcgcgcgg cgacacgcgg cggcaggccc tccaggcgct gggcggcggc     360

ggcgggggca ggggcgccgc ggttcaagcg gccaacttgg cgtcgcgcgt gatcaagcgg     420

gtgttcaagg atcggtccac gtccgggggc ccgcggctgg ccttcgcagg aggaatatgg     480

gccgacacct caacgagcct atcgcctgtg ttcgtggagg ccgcccgtag tgtctacagc 540

tgcacggcgc ggacggctga cttcattaac aagcctgaag atgctgctaa gcaaattaac 600

atgtgggtca aacaatccac aaaagatact gttacctcgc tccttccgga tggactaatt 660

gacaagaata ccggacttgt tattggcagc gcactgtatt ttaggggaag atggcttgac 720

aggactgata cgaggagtga tgcggtacaa aagttctgct gtctggatcg aacttgtgtt 780

gacgttcctt ttgtggaata tgatagaact cgacctttcg cagtccacga tggatttaaa 840

gtgatcaagc ttccttacca gcaaggaaat aatgaacgga agttctccat gtacatcttc 900

ctgcctgatg ctcatgatgg cttgtttgaa ttagctaaga aagttttcgc ggaaccatca 960

ttcttagaac aacacttgcc aactgagaag cggcatgtgg atatcaagat tcccaagttc 1020

acagtgtcat tccaggtcaa tatgaagcag tttctgaaag aaatgggcct cgaattaccc 1080

ttcctccgtg acgcagattt ctctgatatg gtcaaggaag atggatccag aaatccgttg 1140

tatctatccg acatacttca taaagcagtt ttggaggtta atgatgaagg cgttgaagaa 1200

acttctgtga gaattggcat agggaagccc tcaccaggag agcactttgt tgccgaccac 1260

cctttcttct ttgtcattag ggaggaggtg tctggctcat taatgttcat ggggcacata 1320

ctggacccat catcacaatc ttaa 1344

<210> 8
<211> 447
<212> PRT
<213> Zea mays

<400> 8

Met Gln Ala Ser Ser Leu Arg Arg Ala Leu Leu Leu Arg Gly Pro Arg
1               5                   10                  15

Gly Ala Gln Leu Phe Pro Ile Pro Val Gly Asn Lys Pro Arg Ala Phe
            20                  25                  30

Ser Ser Ala Ser Pro Ala Ala Ala Arg Ser Pro Lys Asp Ala His Asn
        35                  40                  45

Ala Pro Pro Pro Ile Met Pro Thr Leu Pro Trp Gly Asp Ala Leu Ala
    50                  55                  60

Ala Thr Gln Arg Thr Phe Cys Leu Pro Leu Ala Gly Arg Val Leu Ala
65                  70                  75                  80

Ala Ser Ala Thr Glu Asn Ala Ala Val Ala Pro Val Ala Val Tyr Ala
                85                  90                  95

Ser Leu Ala Leu Ala Ala Ala Gly Ala Arg Gly Asp Thr Arg Arg Gln

            100                 105                 110

Ala Leu Gln Ala Leu Gly Gly Gly Gly Gly Gly Arg Gly Ala Ala Val
        115                 120                 125

Gln Ala Ala Asn Leu Ala Ser Arg Val Ile Lys Arg Val Phe Lys Asp
    130                 135                 140

Arg Ser Thr Ser Gly Gly Pro Arg Leu Ala Phe Ala Gly Gly Ile Trp
145                 150                 155                 160

Ala Asp Thr Ser Thr Ser Leu Ser Pro Val Phe Val Glu Ala Ala Arg
                165                 170                 175

Ser Val Tyr Ser Cys Thr Ala Arg Thr Ala Asp Phe Ile Asn Lys Pro
            180                 185                 190

Glu Asp Ala Ala Lys Gln Ile Asn Met Trp Val Lys Gln Ser Thr Lys
        195                 200                 205

Asp Thr Val Thr Ser Leu Leu Pro Asp Gly Leu Ile Asp Lys Asn Thr
    210                 215                 220

Gly Leu Val Ile Gly Ser Ala Leu Tyr Phe Arg Gly Arg Trp Leu Asp
225                 230                 235                 240

Arg Thr Asp Thr Arg Ser Asp Ala Val Gln Lys Phe Cys Cys Leu Asp
                245                 250                 255

Arg Thr Cys Val Asp Val Pro Phe Val Glu Tyr Asp Arg Thr Arg Pro
            260                 265                 270

Phe Ala Val His Asp Gly Phe Lys Val Ile Lys Leu Pro Tyr Gln Gln
        275                 280                 285

Gly Asn Asn Glu Arg Lys Phe Ser Met Tyr Ile Phe Leu Pro Asp Ala
    290                 295                 300

His Asp Gly Leu Phe Glu Leu Ala Lys Lys Val Phe Ala Glu Pro Ser
305                 310                 315                 320

Phe Leu Glu Gln His Leu Pro Thr Glu Lys Arg His Val Asp Ile Lys
                325                 330                 335

Ile Pro Lys Phe Thr Val Ser Phe Gln Val Asn Met Lys Gln Phe Leu
            340                 345                 350

Lys Glu Met Gly Leu Glu Leu Pro Phe Leu Arg Asp Ala Asp Phe Ser
        355                 360                 365

Asp Met Val Lys Glu Asp Gly Ser Arg Asn Pro Leu Tyr Leu Ser Asp
    370                 375                 380

Ile Leu His Lys Ala Val Leu Glu Val Asn Asp Glu Gly Val Glu Glu
385                 390                 395                 400

Thr Ser Val Arg Ile Gly Ile Gly Lys Pro Ser Pro Gly Glu His Phe
                405                 410                 415

Val Ala Asp His Pro Phe Phe Phe Val Ile Arg Glu Glu Val Ser Gly
            420                 425                 430

Ser Leu Met Phe Met Gly His Ile Leu Asp Pro Ser Ser Gln Ser
        435                 440                 445

<210>  9
<211>  2751
<212>  DNA
<213>  Artificial Sequence

<220>
<223>  cDNA of Zm00001d049159 of genotype H102

<400>  9
atggggaggg cgggatttct cctcctcccc ctcctgctcc tcctcgtctt cgccggcccc      60

ggccccggcc ccgcctccgc ggcgacggcc atgaccccgg acgcgggcgt catcttggac     120

ctcgccaagt cgctgaccaa cccgccgccg tcctggaccg gcaccgacgt ctgcggcggc     180

gtctccttca gcggaatcac ctgcaacggc gccgcccgcg tcaccggcat caacctcgcc     240

aagctgcatt tatccggcac cctttcctcg tccctcgcca atctcaccgc gctccaatcg     300

ctgcagctcc aaggcaacgc gctggagggc gacctgccct ccctggccca gatgggctcc     360

ctcgagaccc tcgttctcga cggcaacgcc ttctccaccc tgccgccgga cttcctcgag     420

ggtctcccct cgctgctcaa gctcagtatg gacgacctcc ctctcgagcc atggagcatc     480

cccgacgcta tcgcggggtg cgcgatgctt cagaccttct cggcctccaa tgcctctgtc     540

tctggcccct tcccggcggt tcttgccaac ctgacgtcgc tacagacgct gcggctgtcg     600

tacaataacc ttaccggggt cctgccggtg gggttagagg cgttgggctc gctggagacg     660

ctgcagctca acagccagag gtcgaacggc atgctgtcag ggccaattga cgttgtggcg     720

aagttgccga gcttgaagac actgtggctt cagtccaact cgttcaccgg cccgatcccg     780

gagtttgatc ccaacacgca gctggagact ttcaatgtca gagacaacag cctcaccgga     840

ccggtgccac cctctcttgt agggatcaca agccttcagg atgtggcgct gtccaataac     900

tttcttcaag ggccaaaacc aaaattcgca gctaagactg tggatattga ttctgggaac 960

gggttctgtc acgaagaccc tgggccatgc gatccactgg tgaccactct gcttggggtg 1020

gcttcagggt ttggataccc attgcagctc aagaagtggg caggcaacaa cccatgcgat 1080

ccatggcctg gtctttcctg catcaaaatg gatgtaactc aaatcaaatt gcctcgccgg 1140

aacttgtcag ggcttatttc accggctttt gcgaacttga ctagacttca gcggctggat 1200

ctgtctaaca atcgacttac tggggtaata ccagatgctt tgacgacact gaaaagtctc 1260

aattatcttg atgtctcaaa taatcgcctt actggacaag tgcctgagtt caagcagcat 1320

ataaagctga tgactgcagg caactcgttt ggggaatcag gtggtgatag tggagggggt 1380

ggctccaatg ttcggtcctc atcttcaaat cctacaggtt cacataactc aaagtcaaat 1440

gcgggaatga tcgttggaat tcttctagtt gtcattcttc ttgtcatttg tgttgggctt 1500

ttcctacatc atcgaaggaa caagaatgtg gacaagttca gtcctatctc aacaaagagc 1560

ccttctggtg aatctgaggt gatgaagatt caggtagttg ggacaaatgg ccatagcaac 1620

ataagtggtt ctgtaggctc aactgagctt tacagtcatt caagtgctga caacacaagc 1680

attgctgacc tcttcgagtc ccatgggatg caattaccga tgagtgtgct actaaaggcc 1740

acaaacaact ttgatgagga ctacatctta ggtacagggg gctttggggt ggtttttaag 1800

gggactctca atgacaagct ggttgctgtg aagaggtgcg acagtggcac tatgggaact 1860

aaagggctgc aagaattcat ggctgagatt gacgttctta ggaaggtgag acatcggcac 1920

ttggttgcat tgcttggata ctgcacccat ggcaatgaga ggctcttggt ctatgaatat 1980

atgtcgggag gaacactgcg ccagcacttg tgtgatcttg agcagagtgg ttatactcct 2040

cttacatgga cacagaggat gacaatagct ttggatgttg cccgggggat agaatatttg 2100

catggtttgg cgcaggagac tttcattcat agggatctga agccttccaa catattgctt 2160

gatcaggatt taagagccaa ggtttcggac tttggattgg tcaagcttgc gaaagataca 2220

gataagtcca tgatgacaag agtagcaggg acatttggat accttgcgcc tgaatatgcc 2280

actacaggaa aggtcactac aaaagtagat gtgtatgcat acggtgtgat actgatggag 2340

atgattacgg gaaggaaagt acttgatgac tcattacccg atggcgaaac gcatctcgta 2400

acgagcttcc gaaaaaacat gctcgacaaa gagaagttca ggaagttttt ggaccccacc 2460

ctggagctca gcgctgaatc ttggaacagc ctgctggagg tggccgatct tgctcgccat 2520

tgcacggcgc gggaaccgta ccagcggcca gacatgggtc actgcgtgaa caggctttcc 2580

agcctagtgg accagtggaa gccaaccaac attgttgatg atgacgatga ggagggtggg 2640

acaagcgaga tgggtcttca ccagcaccta gaaatatgga gatgcgatga tttcactata 2700

tcggattcgg attcattcag cgcacacaac acatcaagga agtaccattg a 2751

<210>  10
<211>  916
<212>  PRT
<213>  Zea mays

<400>  10
   Met Gly Arg Ala Gly Phe Leu Leu Leu Pro Leu Leu Leu Leu Leu Val
   1               5                   10                  15
   Phe Ala Gly Pro Gly Pro Gly Pro Ala Ser Ala Ala Thr Ala Met Thr
               20                  25                  30
   Pro Asp Ala Gly Val Ile Leu Asp Leu Ala Lys Ser Leu Thr Asn Pro
           35                  40                  45
   Pro Pro Ser Trp Thr Gly Thr Asp Val Cys Gly Gly Val Ser Phe Ser
       50                  55                  60
   Gly Ile Thr Cys Asn Gly Ala Ala Arg Val Thr Gly Ile Asn Leu Ala
   65                  70                  75                  80
   Lys Leu His Leu Ser Gly Thr Leu Ser Ser Ser Leu Ala Asn Leu Thr
                   85                  90                  95
   Ala Leu Gln Ser Leu Gln Leu Gln Gly Asn Ala Leu Glu Gly Asp Leu
               100                 105                 110
   Pro Ser Leu Ala Gln Met Gly Ser Leu Glu Thr Leu Val Leu Asp Gly
           115                 120                 125
   Asn Ala Phe Ser Thr Leu Pro Pro Asp Phe Leu Glu Gly Leu Pro Ser
       130                 135                 140
   Leu Leu Lys Leu Ser Met Asp Asp Leu Pro Leu Glu Pro Trp Ser Ile
   145                 150                 155                 160
   Pro Asp Ala Ile Ala Gly Cys Ala Met Leu Gln Thr Phe Ser Ala Ser
                   165                 170                 175
   Asn Ala Ser Val Ser Gly Pro Phe Pro Ala Val Leu Ala Asn Leu Thr
               180                 185                 190
   Ser Leu Gln Thr Leu Arg Leu Ser Tyr Asn Asn Leu Thr Gly Val Leu
           195                 200                 205
   Pro Val Gly Leu Glu Ala Leu Gly Ser Leu Glu Thr Leu Gln Leu Asn
       210                 215                 220
   Ser Gln Arg Ser Asn Gly Met Leu Ser Gly Pro Ile Asp Val Val Ala
   225                 230                 235                 240
   Lys Leu Pro Ser Leu Lys Thr Leu Trp Leu Gln Ser Asn Ser Phe Thr
                   245                 250                 255
   Gly Pro Ile Pro Glu Phe Asp Pro Asn Thr Gln Leu Glu Thr Phe Asn
               260                 265                 270
   Val Arg Asp Asn Ser Leu Thr Gly Pro Val Pro Pro Ser Leu Val Gly
           275                 280                 285
   Ile Thr Ser Leu Gln Asp Val Ala Leu Ser Asn Asn Phe Leu Gln Gly
       290                 295                 300
   Pro Lys Pro Lys Phe Ala Ala Lys Thr Val Asp Ile Asp Ser Gly Asn
   305                 310                 315                 320
   Gly Phe Cys His Glu Asp Pro Gly Pro Cys Asp Pro Leu Val Thr Thr
                   325                 330                 335
   Leu Leu Gly Val Ala Ser Gly Phe Gly Tyr Pro Leu Gln Leu Lys Lys
               340                 345                 350
   Trp Ala Gly Asn Asn Pro Cys Asp Pro Trp Pro Gly Leu Ser Cys Ile
           355                 360                 365
   Lys Met Asp Val Thr Gln Ile Lys Leu Pro Arg Arg Asn Leu Ser Gly
       370                 375                 380
   Leu Ile Ser Pro Ala Phe Ala Asn Leu Thr Arg Leu Gln Arg Leu Asp
   385                 390                 395                 400
   Leu Ser Asn Asn Arg Leu Thr Gly Val Ile Pro Asp Ala Leu Thr Thr
                   405                 410                 415
   Leu Lys Ser Leu Asn Tyr Leu Asp Val Ser Asn Asn Arg Leu Thr Gly
               420                 425                 430
   Gln Val Pro Glu Phe Lys Gln His Ile Lys Leu Met Thr Ala Gly Asn
           435                 440                 445
   Ser Phe Gly Glu Ser Gly Gly Asp Ser Gly Gly Gly Gly Ser Asn Val
       450                 455                 460
   Arg Ser Ser Ser Ser Asn Pro Thr Gly Ser His Asn Ser Lys Ser Asn
   465                 470                 475                 480
   Ala Gly Met Ile Val Gly Ile Leu Leu Val Val Ile Leu Leu Val Ile
                   485                 490                 495
   Cys Val Gly Leu Phe Leu His His Arg Arg Asn Lys Asn Val Asp Lys
               500                 505                 510
   Phe Ser Pro Ile Ser Thr Lys Ser Pro Ser Gly Glu Ser Glu Val Met
           515                 520                 525
   Lys Ile Gln Val Val Gly Thr Asn Gly His Ser Asn Ile Ser Gly Ser
       530                 535                 540
   Val Gly Ser Thr Glu Leu Tyr Ser His Ser Ser Ala Asp Asn Thr Ser
   545                 550                 555                 560
   Ile Ala Asp Leu Phe Glu Ser His Gly Met Gln Leu Pro Met Ser Val
                   565                 570                 575
   Leu Leu Lys Ala Thr Asn Asn Phe Asp Glu Asp Tyr Ile Leu Gly Thr
               580                 585                 590
   Gly Gly Phe Gly Val Val Phe Lys Gly Thr Leu Asn Asp Lys Leu Val
           595                 600                 605
   Ala Val Lys Arg Cys Asp Ser Gly Thr Met Gly Thr Lys Gly Leu Gln
       610                 615                 620
   Glu Phe Met Ala Glu Ile Asp Val Leu Arg Lys Val Arg His Arg His
   625                 630                 635                 640
   Leu Val Ala Leu Leu Gly Tyr Cys Thr His Gly Asn Glu Arg Leu Leu
                   645                 650                 655
   Val Tyr Glu Tyr Met Ser Gly Gly Thr Leu Arg Gln His Leu Cys Asp
               660                 665                 670
   Leu Glu Gln Ser Gly Tyr Thr Pro Leu Thr Trp Thr Gln Arg Met Thr
           675                 680                 685
   Ile Ala Leu Asp Val Ala Arg Gly Ile Glu Tyr Leu His Gly Leu Ala
       690                 695                 700
   Gln Glu Thr Phe Ile His Arg Asp Leu Lys Pro Ser Asn Ile Leu Leu
   705                 710                 715                 720
   Asp Gln Asp Leu Arg Ala Lys Val Ser Asp Phe Gly Leu Val Lys Leu
                   725                 730                 735
   Ala Lys Asp Thr Asp Lys Ser Met Met Thr Arg Val Ala Gly Thr Phe
               740                 745                 750
   Gly Tyr Leu Ala Pro Glu Tyr Ala Thr Thr Gly Lys Val Thr Thr Lys
           755                 760                 765
   Val Asp Val Tyr Ala Tyr Gly Val Ile Leu Met Glu Met Ile Thr Gly
       770                 775                 780
   Arg Lys Val Leu Asp Asp Ser Leu Pro Asp Gly Glu Thr His Leu Val
   785                 790                 795                 800
   Thr Ser Phe Arg Lys Asn Met Leu Asp Lys Glu Lys Phe Arg Lys Phe
                   805                 810                 815
   Leu Asp Pro Thr Leu Glu Leu Ser Ala Glu Ser Trp Asn Ser Leu Leu
               820                 825                 830
   Glu Val Ala Asp Leu Ala Arg His Cys Thr Ala Arg Glu Pro Tyr Gln
           835                 840                 845
   Arg Pro Asp Met Gly His Cys Val Asn Arg Leu Ser Ser Leu Val Asp
       850                 855                 860
   Gln Trp Lys Pro Thr Asn Ile Val Asp Asp Asp Asp Glu Glu Gly Gly
   865                 870                 875                 880
   Thr Ser Glu Met Gly Leu His Gln His Leu Glu Ile Trp Arg Cys Asp
                   885                 890                 895
   Asp Phe Thr Ile Ser Asp Ser Asp Ser Phe Ser Ala His Asn Thr Ser
               900                 905                 910
   Arg Lys Tyr His
           915

<210>  11
<211>  2694
<212>  DNA
<213>  Artificial Sequence

<220>
<223>  cDNA of Zm00001d049121 of genotype B73

<400>  11
atggcggagg ccatcgtggg gtcgatgctg tggaagctgc agcaggtggc ggtgagtgag        60

gcgcggacgc tggtggccgt gaacgaggac atccggagcc tccgggacaa gctcatgtgg       120

atgcaggcgt tcctgcacga cgtccagccg tcgcgccgcg tgcagcccaa cgagctcatc 180

aaggtgtggc tgcagcagac ccgcgacgcc gtcttcgacg ccgaagacgc cgtcgaccac 240

tttaccaccc aagtggttgt ccgtcgcgac ctctccagca ggatcaggct gatcaatgga 300

aggctggagg gaatcattgc caacaaggac aggtacaggt tgggagagtc tactacgctg 360

ggagcaattt ggaggccatc tagctccacg tcaccgctct cggaaatgat ggacgaggtg 420

gtgctgccac tggtgggacg agaggaactg gtgcgcaatc ttaagaattg gctttataaa 480

ggcagcggga atcataaaaa tgtgatcacc gtgacggggg aaagtggagt cggcaagaca 540

aagctggtga ggaacttgta cgacagcaag cagaccctgt ctcactttga catctacgag 600

tgggtgagct ttgggcccaa tctcagtgcc tctgatgtcc tcaagatcat catcagacgc 660

attacagatg gagaagaatg ttccaaggac aacatagaga ggaagctgcg ggagatactg 720

aaagaaaaga agtatctgtt ggtgatagat gctgagctca gtaactcgga gtggaatcgc 780

attttcgcta tgctccctga ccccaaggat gtggatgtcg atgctgccag cagaatagtg 840

aggatttctc agattccgcc acataagcca cccccacact atcaagaagc caaacttgag 900

gttccgaagt tttatgacca agaagtagtc atcaatctgt tcaaggaaac cttccaatca 960

tgtggcagaa atgaacttcc tgacgaagca ataagagaat acagacagag aatcttggac 1020

aacacaaaag ggttgccgct ggcaatagtt cttctgtcag gccttctgcg gaccaaggag 1080

taccctagtg aatggagacg ggtgttcgag cacctggaca ggatgcagtc aaagcagatc 1140

gacaagattc tatccctctg cttcgatgac cttcactatg acctgaaatc gtgcctcctc 1200

tactttgctg cattgccagt gaataccttt atcagggcaa gcaacatcgt gtgcatgtgg 1260

atggcggagg gcttcctggt acccaaggcc acaacggtgg aaaaagtagg agagcagtac 1320

ctgatggagc tgatagatag gcgcctcgtc aacttggcgc cagtgggcta caatgttcct 1380

gggtacgagc gtgtagctgt tcagagcaaa gtacacgctt tcctgcagct cgaagcacag 1440

gagcaatgct tcgtggagat ccacagtggt gacgacatcc cggctttgtc ggatgcacga 1500

cgcttgtcac tccagaacca caaagacaag tatgcagcac tgtcacaccc gctgccgaag 1560

ctgcgagcca tcctgtcaaa ctttgagacg gagcaagagg ctgcacaagt tgctggcgag 1620

aagccaccag atgaagctgg agaagggcaa catggaaatg gatgccggcc tggtttcaac 1680

aaaaagaaaa tgcaagccaa gtcttgtgtg caggacctgc tacggaactc aaggttcctc 1740

cgtgtcatcc atttcaacgg cctcgaggtg gacaaagagc ttcctgacga aatcggcaaa 1800

gttgtgaaac ctgcaaagat gccgccgtcg attggcaggc tgtgcaacct ccagactctt 1860

gatgtccggg gcacctcggt ccgaagcttg ccagatgggt tctggaggat tcagacgctc 1920

cgtcatgtgc tgggcgaccg tctcatcctg cctaaacgtg ttggtgactt gaggaatctg 1980

cagacactca acagcgtgaa gcccgactat ggtgaccccc atgcctggga cgacaagacc 2040

ttcaaccaca tgatccgcct cttgtacttg cacatccggc gcggtgagag tctgggcgtc 2100

caggaaatct tctccaaccg caagaacaag gctgccttgg taaaagcagt atccattctc 2160

aagtaccttg tcgtcctcat cataaaagct cctgtgatcc cctcagaagt gttcactagg 2220

tccaacctcc aacgtctcaa ggcaatggaa ctggtcggga agcttgacct tcctaaaaat 2280

ggcatccctg agatggatgt ccgcaatcgc ctcccaaacc tcaagaagct gaggcttatg 2340

aagacaatgg tgtcacagga attcattaac caacttggag gcctacagtt cctttccacc 2400

cttgaactaa ccagcaattc tttcagggac agggagcttg tcttcacgga aggattttgc 2460

agcctcaaag aactgacatt gcatgaggag acactcgaga ggttggagat acatgagtcg 2520

gcacttcccg agcttgggga tctggatatt gtcggccata gggacaagct ccatgttgtt 2580

atccatggtc attccgtgct cgtgcaacac attgaagcag aggatgaaga tatttttaag 2640

gttacggaag taataacgac gcaacaagtc caagtggaag accacaaagt ctga 2694

<210> 12
<211> 897
<212> PRT
<213> Zea mays

<400> 12

Met Ala Glu Ala Ile Val Gly Ser Met Leu Trp Lys Leu Gln Gln Val
1               5                   10                  15

Ala Val Ser Glu Ala Arg Thr Leu Val Ala Val Asn Glu Asp Ile Arg
            20                  25                  30

Ser Leu Arg Asp Lys Leu Met Trp Met Gln Ala Phe Leu His Asp Val
        35                  40                  45

Gln Pro Ser Arg Arg Val Gln Pro Asn Glu Leu Ile Lys Val Trp Leu
    50                  55                  60

Gln Gln Thr Arg Asp Ala Val Phe Asp Ala Glu Asp Ala Val Asp His
65                  70                  75                  80

Phe Thr Thr Gln Val Val Val Arg Arg Asp Leu Ser Ser Arg Ile Arg
                85                  90                  95

Leu Ile Asn Gly Arg Leu Glu Gly Ile Ile Ala Asn Lys Asp Arg Tyr
            100                 105                 110

Arg Leu Gly Glu Ser Thr Thr Leu Gly Ala Ile Trp Arg Pro Ser Ser
        115                 120                 125

Ser Thr Ser Pro Leu Ser Glu Met Met Asp Glu Val Val Leu Pro Leu
    130                 135                 140

Val Gly Arg Glu Glu Leu Val Arg Asn Leu Lys Asn Trp Leu Tyr Lys
145                 150                 155                 160

Gly Ser Gly Asn His Lys Asn Val Ile Thr Val Thr Gly Glu Ser Gly
                165                 170                 175

Val Gly Lys Thr Lys Leu Val Arg Asn Leu Tyr Asp Ser Lys Gln Thr
            180                 185                 190

Leu Ser His Phe Asp Ile Tyr Glu Trp Val Ser Phe Gly Pro Asn Leu
        195                 200                 205

Ser Ala Ser Asp Val Leu Lys Ile Ile Ile Arg Arg Ile Thr Asp Gly
    210                 215                 220

Glu Glu Cys Ser Lys Asp Asn Ile Glu Arg Lys Leu Arg Glu Ile Leu
225                 230                 235                 240

Lys Glu Lys Lys Tyr Leu Leu Val Ile Asp Ala Glu Leu Ser Asn Ser
                245                 250                 255

Glu Trp Asn Arg Ile Phe Ala Met Leu Pro Asp Pro Lys Asp Val Asp
            260                 265                 270

Val Asp Ala Ala Ser Arg Ile Val Arg Ile Ser Gln Ile Pro Pro His
        275                 280                 285

Lys Pro Pro Pro His Tyr Gln Glu Ala Lys Leu Glu Val Pro Lys Phe
    290                 295                 300

Tyr Asp Gln Glu Val Val Ile Asn Leu Phe Lys Glu Thr Phe Gln Ser
305                 310                 315                 320

Cys Gly Arg Asn Glu Leu Pro Asp Glu Ala Ile Arg Glu Tyr Arg Gln
                325                 330                 335

Arg Ile Leu Asp Asn Thr Lys Gly Leu Pro Leu Ala Ile Val Leu Leu
            340                 345                 350

Ser Gly Leu Leu Arg Thr Lys Glu Tyr Pro Ser Glu Trp Glu Thr Val
        355                 360                 365

Phe Glu His Leu Asp Arg Met Gln Ser Lys Gln Ile Asp Lys Ile Leu
    370                 375                 380

Ser Leu Cys Phe Asp Asp Leu His Tyr Asp Leu Lys Ser Cys Leu Leu
385                 390                 395                 400

Tyr Phe Ala Ala Leu Pro Val Asn Thr Phe Ile Arg Ala Ser Asn Ile
                405                 410                 415

Val Cys Met Trp Met Ala Glu Gly Phe Leu Val Pro Lys Ala Thr Thr
            420                 425                 430

Val Glu Lys Val Gly Glu Gln Tyr Leu Met Glu Leu Ile Asp Arg Arg
        435                 440                 445

Leu Val Asn Leu Ala Pro Val Gly Tyr Asn Val Pro Gly Tyr Glu Arg
    450                 455                 460

Val Ala Val Gln Ser Lys Val His Ala Phe Leu Gln Leu Glu Ala Gln
465                 470                 475                 480

Glu Gln Cys Phe Val Glu Ile His Ser Gly Asp Asp Ile Pro Ala Leu
                485                 490                 495

Ser Asp Ala Arg Arg Leu Ser Leu Gln Asn His Lys Asp Lys Tyr Ala
            500                 505                 510

Ala Leu Ser His Pro Leu Pro Lys Leu Arg Ala Ile Leu Ser Asn Phe
        515                 520                 525

Glu Thr Glu Gln Glu Ala Ala Gln Val Ala Gly Glu Lys Pro Pro Asp
    530                 535                 540

Glu Ala Gly Glu Gly Gln His Gly Asn Gly Cys Arg Pro Gly Phe Asn
545                 550                 555                 560

Lys Lys Lys Met Gln Ala Lys Ser Cys Val Gln Asp Leu Leu Arg Asn
                565                 570                 575

Ser Arg Phe Leu Arg Val Ile His Phe Asn Gly Leu Glu Val Asp Lys
            580                 585                 590

Glu Leu Pro Asp Glu Ile Gly Lys Val Val Lys Pro Ala Lys Met Pro
        595                 600                 605

Pro Ser Ile Gly Arg Leu Cys Asn Leu Gln Thr Leu Asp Val Arg Gly
    610                 615                 620

Thr Ser Val Arg Ser Leu Pro Asp Gly Phe Trp Arg Ile Gln Thr Leu
625                 630                 635                 640

Arg His Val Leu Gly Asp Arg Leu Ile Leu Pro Lys Arg Val Gly Asp
                645                 650                 655

Leu Arg Asn Leu Gln Thr Leu Asn Ser Val Lys Pro Asp Tyr Gly Asp
            660                 665                 670

Pro His Ala Trp Asp Asp Lys Thr Phe Asn His Met Ile Arg Leu Leu
        675                 680                 685

Tyr Leu His Ile Arg Arg Gly Glu Ser Leu Gly Val Gln Glu Ile Phe
    690                 695                 700

Ser Asn Arg Lys Asn Lys Ala Ala Leu Val Lys Ala Val Ser Ile Leu
705                 710                 715                 720

Lys Tyr Leu Val Val Leu Ile Ile Lys Ala Pro Val Ile Pro Ser Glu
                725                 730                 735

Val Phe Thr Arg Ser Asn Leu Gln Arg Leu Lys Ala Met Glu Leu Val
            740                 745                 750

Gly Lys Leu Asp Leu Pro Lys Asn Gly Ile Pro Glu Met Asp Val Arg
        755                 760                 765

Asn Arg Leu Pro Asn Leu Lys Lys Leu Arg Leu Met Lys Thr Met Val
    770                 775                 780

Ser Gln Glu Phe Ile Asn Gln Leu Gly Gly Leu Gln Phe Leu Ser Thr
785                 790                 795                 800

Leu Glu Leu Thr Ser Asn Ser Phe Arg Asp Arg Glu Leu Val Phe Thr
                805                 810                 815

Glu Gly Phe Cys Ser Leu Lys Glu Leu Thr Leu His Glu Glu Thr Leu
            820                 825                 830

Glu Arg Leu Glu Ile His Glu Ser Ala Leu Pro Glu Leu Gly Asp Leu
        835                 840                 845

Asp Ile Val Gly His Arg Asp Lys Leu His Val Val Ile His Gly His
    850                 855                 860

Ser Val Leu Val Gln His Ile Glu Ala Glu Asp Glu Asp Ile Phe Lys
865                 870                 875                 880

Val Thr Glu Val Ile Thr Thr Gln Gln Val Gln Val Glu Asp His Lys

885                 890                 895

Val

<210>  13
<211>  1092
<212>  DNA
<213>  Artificial Sequence

<220>
<223>  cDNA of Zm00001d049113 of genotype B73

<400>  13
atggatcagt caaacattag caacaagcac atgacgatga caaccctaat gctcctcctc      60

tgtgtcatag ccattgcaga ttgcatttgc cacgccgcag tggcagcccg ggtggagcca     120

tccaccaccg tcggcagaac tacaggagga gacgaggcga tgatgatggc gaggtacaag     180

aagtggatgg cgcagtatcg ccggaagtac aaggacgacg ccgagaaagc acaccgtttc     240

caggtattca aggcgaacgc ggagtttatt gacaggtcta acgctggagg aaagaagaag     300

tacgtcctag ggaccaacca gttcgccgac ctgaccagca aagagttcgc ggccatgtac     360

accggtttga ggaaaccggc ggcggtgcct tccggggcca agcagatccc tgcagctggt     420

tccaagtacc agaattttac gcgcctagat gatgatgtcc aggttgattg gaggcagcag     480

ggtgctgtca ctcctgtcaa gaaccaaggc caatgtggct gttgctgggc gttctctgca     540

gtaggtgcca tggaaggttt gatcatgata acgacaggaa acctggtctc cctgtcggag     600

cagcagattc tagactgcga cgagtcagac gggaaccagg gctgcaacgg tggctacatg     660

gacaacgcct tccagtacgt catcaacaat ggcggcgtca ccactgagga cgcctaccct     720

tactctgcag tccaagggac gtgccaaaac gtccagccag ccgccaccat cagcggcttc     780

caggacctgc ccagcggcga cgagaacgcg ctcgccaacg cagtcgccaa ccagccggtg     840

tctgttggcg tcgacggcgg atcgagtcct ttccagtttt accagggtgg catatacgac     900

ggggatggct gtggcacgga catgaaccat gcagtgacgg cgatcggcta cggcgccgat     960

gaccagggaa cccagtactg gatcctcaag aactcctggg gcacaggatg gggtgagaac    1020

ggtttcatgc agctacagat gggcgtcggc gcctgtggta tctccacgat ggcctcctac    1080

ccaactccat ga                                                        1092

<210>  14
<211>  363
<212>  PRT
<213>  Zea mays

<400>  14

Met Asp Gln Ser Asn Ile Ser Asn Lys His Met Thr Met Thr Thr Leu

1               5                   10                  15

Met Leu Leu Leu Cys Val Ile Ala Ile Ala Asp Cys Ile Cys His Ala
            20                  25                  30

Ala Val Ala Ala Arg Val Glu Pro Ser Thr Thr Val Gly Arg Thr Thr
        35                  40                  45

Gly Gly Asp Glu Ala Met Met Met Ala Arg Tyr Lys Lys Trp Met Ala
    50                  55                  60

Gln Tyr Arg Arg Lys Tyr Lys Asp Asp Ala Glu Lys Ala His Arg Phe
65                  70                  75                  80

Gln Val Phe Lys Ala Asn Ala Glu Phe Ile Asp Arg Ser Asn Ala Gly
                85                  90                  95

Gly Lys Lys Lys Tyr Val Leu Gly Thr Asn Gln Phe Ala Asp Leu Thr
            100                 105                 110

Ser Lys Glu Phe Ala Ala Met Tyr Thr Gly Leu Arg Lys Pro Ala Ala
        115                 120                 125

Val Pro Ser Gly Ala Lys Gln Ile Pro Ala Ala Gly Ser Lys Tyr Gln
    130                 135                 140

Asn Phe Thr Arg Leu Asp Asp Asp Val Gln Val Asp Trp Arg Gln Gln
145                 150                 155                 160

Gly Ala Val Thr Pro Val Lys Asn Gln Gly Gln Cys Gly Cys Cys Trp
                165                 170                 175

Ala Phe Ser Ala Val Gly Ala Met Glu Gly Leu Ile Met Ile Thr Thr
            180                 185                 190

Gly Asn Leu Val Ser Leu Ser Glu Gln Gln Ile Leu Asp Cys Asp Glu
        195                 200                 205

Ser Asp Gly Asn Gln Gly Cys Asn Gly Gly Tyr Met Asp Asn Ala Phe
    210                 215                 220

Gln Tyr Val Ile Asn Asn Gly Gly Val Thr Thr Glu Asp Ala Tyr Pro
225                 230                 235                 240

Tyr Ser Ala Val Gln Gly Thr Cys Gln Asn Val Gln Pro Ala Ala Thr
                245                 250                 255

Ile Ser Gly Phe Gln Asp Leu Pro Ser Gly Asp Glu Asn Ala Leu Ala
            260                 265                 270

Asn Ala Val Ala Asn Gln Pro Val Ser Val Gly Val Asp Gly Gly Ser
        275                 280                 285

Ser Pro Phe Gln Phe Tyr Gln Gly Gly Ile Tyr Asp Gly Asp Gly Cys
    290                 295                 300

Gly Thr Asp Met Asn His Ala Val Thr Ala Ile Gly Tyr Gly Ala Asp
305                 310                 315                 320

Asp Gln Gly Thr Gln Tyr Trp Ile Leu Lys Asn Ser Trp Gly Thr Gly
                325                 330                 335

Trp Gly Glu Asn Gly Phe Met Gln Leu Gln Met Gly Val Gly Ala Cys
            340                 345                 350

Gly Ile Ser Thr Met Ala Ser Tyr Pro Thr Pro
        355                 360

<210>  15
<211>  2370
<212>  DNA
<213>  Artificial Sequence

<220>
<223>  cDNA of Zm00001d049145 of genotype B73

<400>  15
atgccgccgc cgcgccggct ccacctccat gccgccttcc tcctcctgcc catcttcctg      60

ctcgcgacgt ggcggcccgc ggcggcgtcc gcactctcca ccttcgcgct ggccaaggcg     120

gacgccacca ccatagtgtg cgcgctcctg ccgtccgcgg cgtcgcctct gctggtcgac     180

ctcaactgca cggccgccgg cggcgaccac gcgcgccagg agacgtaccc gtcctcgcac     240

cccttcaacg cgctggccgg aggggaggac ttcctctgcg ccgtggggcc ctccggcgag     300

cgcgcgggcg acgtggacat gcgctggtgg gacctgtccg gcgacggtag caacaggtcc     360

aagcgggtct acatgggccc gccgctccgg gcgctgtcct cgagcgagta ccgcgtctgc     420

ggggtgctcc acagcgggga gctccactgc tggcgctggc gcggcctccg catctccagg     480

gacctccggt tcgtcgccgc cgccgtcggg gacggattcg tgtgcgccat cctcaagggg     540

acgccggcct ccatccgctg cttcggcaac gacacagcgg ccccggccgt cgccggcgcg     600

ccgacgaacg ggagcttcga cgtcgtggcc gcttgcgccc accgcgcgtg cgcgctgtcc     660

acggcggggg cgctcacgtg ctggggccgc gacccgccgc gcgtcggccc gggcgatgcg     720

gcggccaccg ggtacgcggc gctggcattg ggggaggacg gcgtctgcgg cctgcgcacc     780

aatttcacca tccgctgctt cggcgacggc gtggcccacc cgccgcccga cctcgcgcac 840

gcccagttcc tcgacgtgcg cgcgcacggc aaggccttct gcggcgtgct catggccaac 900

tactccctcg tctgctgggg cggccctgag ttcaacacca ccaaccgcct cgtcttcggc 960

cgcgtcatgc cgggcccctg cctgcccatg tcctcgtgcc aatgcggcgt cttgccgggc 1020

tccgccaact tctgcgacga cagccgctgc gtctgcgtcg actgcgcgtt cgagctcaac 1080

gtcgccaggc ccaacgcgtc ggtcgtcccc gggaagggag gttggagcag gaggaccatg 1140

tggattgcaa tcgccgcggc ggccgccgtt ttcctcgtgt tcttcgtggc attccagttc 1200

gcgctggtcc tatggtgtcg ccgccgccgc cgccgcagga aagaactgga tgcctccgct 1260

ggtgtgcagc agtcgctgat gccgccgagg ctcgggtcca gcaggagcaa gggcccgggc 1320

agcgtggtgg agcacttcac gctagacatg ctccaggccg cgacggacgg gttctccgat 1380

gacagccgga tcggaaccgg gagcttcggg tccgtgtacc gtggcaccct acccgacggc 1440

cgcgaggtcg cgatcaagcg cgccgaggac tcggccaagg cgtcgagctc gatggcgcgc 1500

ccggcgcggc gccgggaccg cgagacggcg ttcaactcgg agctgaccgc gctggcgcgc 1560

gccaaccaca agaacatcgt gtgcctgctg ggctgctgcg cggactccgg cgagcgcgtg 1620

ctggtgttcg agttcatggc gaacggcacg ctgcacgacc agctccacag ccgcagcccc 1680

atggctgcgg ccgtgtcgtc gtggcgcggc cgcctcacca tcgcgctggg cgcggcgcgg 1740

ggcatcgagt acatgcacgt ctacgccgtg ccgcccatca tccaccgcga cgtcaagtcc 1800

gccaacatcc tgctcgacga cgcgtggacg gccaagatcg ccgacttcgg gctgtcctcc 1860

gtcctggacc ccggcgtggg cgcctgcggc gacggcacgc cgcaggagcc cctctacacc 1920

ggcggcacgg tggggtacat ggacccggag tactaccggc tgcagcacct gacggacaag 1980

agcgacgtgt acagcttcgg cgtggtgctc ctggagctca tgtccgggtg ccgcgtcgtg 2040

cagcgctacg ccgagagcgt gacgcccaag aacgtggtgg agttcgcggt gccgcacatc 2100

ctcgccgacg acgtcgcgcg cgtgctcgac ccgcgcctgc ccgcgcccac gcccgacgaa 2160

gccgaggcgc tggcctacgt gggctacctc gccgccgact gcgtggggcc agtggggtgc 2220

gaccgcccgt ccatgaccga ggtcgtggac gccctggagc gcgccctcgc ggcctgcggg 2280

gctgggccgc tctcccgcgc cggcaccggc cgccgccccg tgctctcccg ctccggcacc 2340

gaccagttcg acctcaccga caccgactag 2370

<210> 16
<211> 789
<212> PRT
<213> Zea mays

<400> 16

Met Pro Pro Pro Arg Arg Leu His Leu His Ala Ala Phe Leu Leu Leu

1               5                   10                  15

Pro Ile Phe Leu Leu Ala Thr Trp Arg Pro Ala Ala Ala Ser Ala Leu
            20                  25                  30

Ser Thr Phe Ala Leu Ala Lys Ala Asp Ala Thr Thr Ile Val Cys Ala
        35                  40                  45

Leu Leu Pro Ser Ala Ala Ser Pro Leu Leu Val Asp Leu Asn Cys Thr
    50                  55                  60

Ala Ala Gly Gly Asp His Ala Arg Gln Glu Thr Tyr Pro Ser Ser His
65                  70                  75                  80

Pro Phe Asn Ala Leu Ala Gly Gly Glu Asp Phe Leu Cys Ala Val Gly
                85                  90                  95

Pro Ser Gly Glu Arg Ala Gly Asp Val Asp Met Arg Trp Trp Asp Leu
            100                 105                 110

Ser Gly Asp Gly Ser Asn Arg Ser Lys Arg Val Tyr Met Gly Pro Pro
        115                 120                 125

Leu Arg Ala Leu Ser Ser Ser Glu Tyr Arg Val Cys Gly Val Leu His
    130                 135                 140

Ser Gly Glu Leu His Cys Trp Arg Trp Arg Gly Leu Arg Ile Ser Arg
145                 150                 155                 160

Asp Leu Arg Phe Val Ala Ala Ala Val Gly Asp Gly Phe Val Cys Ala
                165                 170                 175

Ile Leu Lys Gly Thr Pro Ala Ser Ile Arg Cys Phe Gly Asn Asp Thr
            180                 185                 190

Ala Ala Pro Ala Val Ala Gly Ala Pro Thr Asn Gly Ser Phe Asp Val
        195                 200                 205

Val Ala Ala Cys Ala His Arg Ala Cys Ala Leu Ser Thr Ala Gly Ala
    210                 215                 220

Leu Thr Cys Trp Gly Arg Asp Pro Pro Arg Val Gly Pro Gly Asp Ala
225                 230                 235                 240

Ala Ala Thr Gly Tyr Ala Ala Leu Ala Leu Gly Glu Asp Gly Val Cys
                245                 250                 255

Gly Leu Arg Thr Asn Phe Thr Ile Arg Cys Phe Gly Asp Gly Val Ala
            260                 265                 270

His Pro Pro Pro Asp Leu Ala His Ala Gln Phe Leu Asp Val Arg Ala
        275                 280                 285

His Gly Lys Ala Phe Cys Gly Val Leu Met Ala Asn Tyr Ser Leu Val
    290                 295                 300

Cys Trp Gly Gly Pro Glu Phe Asn Thr Thr Asn Arg Leu Val Phe Gly
305                 310                 315                 320

Arg Val Met Pro Gly Pro Cys Leu Pro Met Ser Ser Cys Gln Cys Gly
                325                 330                 335

Val Leu Pro Gly Ser Ala Asn Phe Cys Asp Asp Ser Arg Cys Val Cys
            340                 345                 350

Val Asp Cys Ala Phe Glu Leu Asn Val Ala Arg Pro Asn Ala Ser Val
        355                 360                 365

Val Pro Gly Lys Gly Gly Trp Ser Arg Arg Thr Met Trp Ile Ala Ile
    370                 375                 380

Ala Ala Ala Ala Ala Val Phe Leu Val Phe Phe Val Ala Phe Gln Phe
385                 390                 395                 400

Ala Leu Val Leu Trp Cys Arg Arg Arg Arg Arg Arg Arg Lys Glu Leu
                405                 410                 415

Asp Ala Ser Ala Gly Val Gln Gln Ser Leu Met Pro Pro Arg Leu Gly
            420                 425                 430

Ser Ser Arg Ser Lys Gly Pro Gly Ser Val Val Glu His Phe Thr Leu
        435                 440                 445

Asp Met Leu Gln Ala Ala Thr Asp Gly Phe Ser Asp Asp Ser Arg Ile
    450                 455                 460

Gly Thr Gly Ser Phe Gly Ser Val Tyr Arg Gly Thr Leu Pro Asp Gly
465                 470                 475                 480

Arg Glu Val Ala Ile Lys Arg Ala Glu Asp Ser Ala Lys Ala Ser Ser
                485                 490                 495

Ser Met Ala Arg Pro Ala Arg Arg Arg Asp Arg Glu Thr Ala Phe Asn
            500                 505                 510

Ser Glu Leu Thr Ala Leu Ala Arg Ala Asn His Lys Asn Ile Val Cys
        515                 520                 525

Leu Leu Gly Cys Cys Ala Asp Ser Gly Glu Arg Val Leu Val Phe Glu
    530                 535                 540

Phe Met Ala Asn Gly Thr Leu His Asp Gln Leu His Ser Arg Ser Pro
545                 550                 555                 560

Met Ala Ala Ala Val Ser Ser Trp Arg Gly Arg Leu Thr Ile Ala Leu
                565                 570                 575

Gly Ala Ala Arg Gly Ile Glu Tyr Met His Val Tyr Ala Val Pro Pro
            580                 585                 590

Ile Ile His Arg Asp Val Lys Ser Ala Asn Ile Leu Leu Asp Asp Ala
        595                 600                 605

Trp Thr Ala Lys Ile Ala Asp Phe Gly Leu Ser Ser Val Leu Asp Pro
    610                 615                 620

Gly Val Gly Ala Cys Gly Asp Gly Thr Pro Gln Glu Pro Leu Tyr Thr
625                 630                 635                 640

Gly Gly Thr Val Gly Tyr Met Asp Pro Glu Tyr Tyr Arg Leu Gln His
                645                 650                 655

Leu Thr Asp Lys Ser Asp Val Tyr Ser Phe Gly Val Val Leu Leu Glu
            660                 665                 670

Leu Met Ser Gly Cys Arg Val Val Gln Arg Tyr Ala Glu Ser Val Thr
        675                 680                 685

Pro Lys Asn Val Val Glu Phe Ala Val Pro His Ile Leu Ala Asp Asp
    690                 695                 700

Val Ala Arg Val Leu Asp Pro Arg Leu Pro Ala Pro Thr Pro Asp Glu
705                 710                 715                 720

Ala Glu Ala Leu Ala Tyr Val Gly Tyr Leu Ala Ala Asp Cys Val Gly
                725                 730                 735

Pro Val Gly Cys Asp Arg Pro Ser Met Thr Glu Val Val Asp Ala Leu
            740                 745                 750

Glu Arg Ala Leu Ala Ala Cys Gly Ala Gly Pro Leu Ser Arg Ala Gly
        755                 760                 765

Thr Gly Arg Arg Pro Val Leu Ser Arg Ser Gly Thr Asp Gln Phe Asp
    770                 775                 780

Leu Thr Asp Thr Asp
785

<210>  17
<211>  1344
<212>  DNA
<213>  Artificial Sequence

<220>
<223>  cDNA of Zm00001d049148 of genotype B73

<400>  17
atgcaggcct cttccctccg gcgagccctc cttctgcggg gaccgcgagg cgcccagctc      60

ttccccattc ccgtaggcaa caagccccgt gccttctcct ccgcgtcccc cgcggcagcg     120

cgctctccca aagacgctca taatgcgccg ccgccgatta tgccgacgct gccgtggggt     180

gatgcgctcg cagccacgca gcgcaccttc tgcctgccgc tcgctgggcg cgtcctcgcc     240

gcctcggcga ccgagaacgc ggccgtggcc cccgtcgcag tctacgcctc gctggccctg     300

gccgccgccg gcgcgcgcgg cgacacgcgg cggcaggtcc tccaggcgct gggcggcggc     360

ggcgggggca ggggcgccgc ggttcaagcg gccaacttgg cgtcgcgcgt ggtcaagcgg     420

gtgttcaagg accggtccac gtccggcggc ccgcggctgg ccttcgcagg aggaatatgg     480

gccgacacct caacgagcct atcgccgggg ttcgtggagg ccgcccgtag tgtctacagc     540

tgcacggcgc ggacggctga cttcattaac aagcctgaag atgccgccaa gctaattaac     600

atgtgggtca aacaatccac aaaagatact gttacctcgc tccttccgga tggactaatt     660

gacaagaata caggacttgt tattggcagc gcactgtatt ttaggggaag atggcttgac     720

aggactgata cgaggagtga cgcggtacaa aaattctgct gtctggatcg aacttgtgtt     780

gacgttcctt ttgtggaata tgatagaact cgacctttcg cagtccacga tggatttaaa     840

gtgattaagc ttccttacca gcaaggaaat aatgaacgga agttctccat gtacatcttc     900

ctgcctgatg ctcatgatgg cttgtttgaa ttagctaaga aagttttcgc ggaaccatca     960

ttcttagaac aacacttgcc aactgagaag cggcacgtgg atatcaagat tcccaagttc    1020

acagtgtcat tccaggtcaa tatgaagcag tttctgaaag aaatgggcct cgaattaccc    1080

ttcctccgtg atgcagattt cactgatatg gtcaaggaag atggatccag aaatccgttg    1140

tatctatccg acatacttca taaagcagtt ttggaggtta atgatgaagg cgttgaagaa    1200

acttctgtga gaattggcat agggaagccc tcaccaggag agcactttgt tgccgaccat    1260

cctttcttct ttgtcattag ggaggaggtg tctggctcag taatgttcat ggggcacata    1320

ctggacccat catcacaatc ttaa                                              1344

<210>  18
<211>  447
<212>  PRT
<213>  Zea mays

<400>  18

Met Gln Ala Ser Ser Leu Arg Arg Ala Leu Leu Leu Arg Gly Pro Arg
1               5                   10                  15

Gly Ala Gln Leu Phe Pro Ile Pro Val Gly Asn Lys Pro Arg Ala Phe
            20                  25                  30

Ser Ser Ala Ser Pro Ala Ala Ala Arg Ser Pro Lys Asp Ala His Asn
        35                  40                  45

Ala Pro Pro Pro Ile Met Pro Thr Leu Pro Trp Gly Asp Ala Leu Ala
    50                  55                  60

Ala Thr Gln Arg Thr Phe Cys Leu Pro Leu Ala Gly Arg Val Leu Ala
65                  70                  75                  80

Ala Ser Ala Thr Glu Asn Ala Ala Val Ala Pro Val Ala Val Tyr Ala
                85                  90                  95

Ser Leu Ala Leu Ala Ala Ala Gly Ala Arg Gly Asp Thr Arg Arg Gln
            100                 105                 110

Val Leu Gln Ala Leu Gly Gly Gly Gly Gly Gly Arg Gly Ala Ala Val
        115                 120                 125

Gln Ala Ala Asn Leu Ala Ser Arg Val Val Lys Arg Val Phe Lys Asp
    130                 135                 140

Arg Ser Thr Ser Gly Gly Pro Arg Leu Ala Phe Ala Gly Gly Ile Trp
145                 150                 155                 160

Ala Asp Thr Ser Thr Ser Leu Ser Pro Gly Phe Val Glu Ala Ala Arg
                165                 170                 175

Ser Val Tyr Ser Cys Thr Ala Arg Thr Ala Asp Phe Ile Asn Lys Pro
            180                 185                 190

Glu Asp Ala Ala Lys Leu Ile Asn Met Trp Val Lys Gln Ser Thr Lys
        195                 200                 205

Asp Thr Val Thr Ser Leu Leu Pro Asp Gly Leu Ile Asp Lys Asn Thr

210                 215                 220

Gly Leu Val Ile Gly Ser Ala Leu Tyr Phe Arg Gly Arg Trp Leu Asp
225                 230                 235                 240

Arg Thr Asp Thr Arg Ser Asp Ala Val Gln Lys Phe Cys Cys Leu Asp
                245                 250                 255

Arg Thr Cys Val Asp Val Pro Phe Val Glu Tyr Asp Arg Thr Arg Pro
            260                 265                 270

Phe Ala Val His Asp Gly Phe Lys Val Ile Lys Leu Pro Tyr Gln Gln
        275                 280                 285

Gly Asn Asn Glu Arg Lys Phe Ser Met Tyr Ile Phe Leu Pro Asp Ala
    290                 295                 300

His Asp Gly Leu Phe Glu Leu Ala Lys Lys Val Phe Ala Glu Pro Ser
305                 310                 315                 320

Phe Leu Glu Gln His Leu Pro Thr Glu Lys Arg His Val Asp Ile Lys
                325                 330                 335

Ile Pro Lys Phe Thr Val Ser Phe Gln Val Asn Met Lys Gln Phe Leu
            340                 345                 350

Lys Glu Met Gly Leu Glu Leu Pro Phe Leu Arg Asp Ala Asp Phe Thr
        355                 360                 365

Asp Met Val Lys Glu Asp Gly Ser Arg Asn Pro Leu Tyr Leu Ser Asp
    370                 375                 380

Ile Leu His Lys Ala Val Leu Glu Val Asn Asp Glu Gly Val Glu Glu
385                 390                 395                 400

Thr Ser Val Arg Ile Gly Ile Gly Lys Pro Ser Pro Gly Glu His Phe
                405                 410                 415

Val Ala Asp His Pro Phe Phe Phe Val Ile Arg Glu Glu Val Ser Gly
            420                 425                 430

Ser Val Met Phe Met Gly His Ile Leu Asp Pro Ser Ser Gln Ser
        435                 440                 445

<210>  19
<211>  2745
<212>  DNA
<213>  Artificial Sequence

<220>
<223>  cDNA of Zm00001d049159 of genotype B73

<400>  19
atggggaggg cgggatttct cttcctcccc ctcctgctcc tcctcgcctt cgccggcccc      60

ggccccgcct ccgcggcgac ggccatgacc ccggacgcgg gcgtcatctt ggacctcgcc     120

aagtcgctga ccaacccgcc gccgtcctgg accggcaccg acgtctgcgg cggcgtctcc     180

ttcagcggcg tcacctgcaa cggcgccgcc cgcgtcaccg gcatcaacct cgccaagctg     240

catttatccg gcaccctttc ctcgtccctc gccaatctca ccgcgctcca atcgctgcag     300

ctccaaggca acgcgctgga gggcgacctg ccctccctgg cccagatggg ctccctcgag     360

accctcgttc tcgacggcaa cgccttctcc accctgccgc cggacttcct cgagggtctc     420

ccctcgctgc tcaagctcag tatggacgac ctccctctcg agccatggag catccccgac     480

gctatcgcgg ggtgcgcgat gcttcagacc ttctcggcct ccaatgcctc tgtctctggc     540

cccttcccgg cggttcttgc caacctgacg tcgctacaga cgctgcggct gtcgtacaat     600

aaccttaccg gggtcctgcc ggtggggtta gaggcgttgg gctcgctgga gacgctgcag     660

ctcaacagcc agaggtcgaa cggcatgctg tcagggccaa ttgacgttgt ggcgaagttg     720

ccgagcttga agacactgtg gcttcagtcc aactcgttca ccggcccgat cccggagttt     780

gatcccaaca cgcagctgga gactttcaat gtcagagaca acagcctcac cggaccggtg     840

ccaccctctc ttgtagggat cacaagcctt caggatgtgg cgctgtccaa taactttctt     900

caagggccaa aaccaaaatt cgcagctaag actgtggata ttgattctgg gaacgggttc     960

tgtcacgaag accctgggcc atgcgatcca ctggtgacca ctctgcttgg ggtggcttca    1020

gggtttggat acccattgca gctcaagaag tgggcaggca acaacccatg cgatccatgg    1080

cctggtcttt cctgcatcaa aatggatgta actcaaatca aattgcctcg ccggaacttg    1140

tcagggctta tttcaccggc ttttgcgaac ttgactagac ttcagcggct ggatctgtct    1200

aacaatcgac ttactggggt aataccagat gctttgacga cactgaaaag tctcaattat    1260

cttgatgtct caaataatcg ccttactgga caagtgcctg agttcaagca gcatataaag    1320

ctgatgactg caggcaactc gtttggggaa tcaggtggtg atagtggagg gggtggctcc    1380

aatgttcggt cctcatcttc aaatcctaca ggttcacata actcaaagtc aaatgcggga    1440

atgatcgttg gaattcttct agttgtcatt cttcttgtca tttgtgttgg gcttttccta    1500

catcatcgaa ggaacaagaa tgtggacaag ttcagtccta tctcaacaaa gagcccttct    1560

ggtgaatctg aggtgatgaa gattcaggta gttgggacaa atggccatag caacataagt    1620

ggttctgtag gctcaactga gctttacagt cattcaagtg ctgacaacac aagcattgct    1680

gacctcttcg agtcccatgg gatgcaatta ccgatgagtg tgctactaaa ggccacaaac    1740

aactttgatg aggactacat cttaggtaca gggggctttg gggtggtttt taaggggact 1800

ctcaatgaca agctggttgc tgtgaagagg tgcgacagtg gcactatggg aactaaaggg 1860

ctgcaagaat tcatggctga gattgacgtt cttaggaagg tgagacatcg gcacttggtt 1920

gcattgcttg gatactgcac ccatggcaat gagaggctct tggtctatga atatatgtca 1980

ggaggaacac tgcgccagca cttgtgtgat cttcagcaga gtggttatac tcctcttaca 2040

tggacacaga ggatgacaat agctttggat gttgcccggg ggatagaata tttgcatggt 2100

ttggcgcagg agactttcat tcatagggat ctgaagcctt ccaacatatt gcttgatcaa 2160

gatttaagag ccaaggtttc ggactttgga ttggtcaagc ttgcgaaaga tacagataag 2220

tccatgatga caagagtagc agggacattt ggataccttg cgcctgaata tgccactaca 2280

ggaaaggtca ctacaaaagt agatgtgtat gcatacggtg tgatactgat ggagatgatt 2340

acgggaagga aagtacttga tgactcatta cccgatggcg aaacgcatct cgtaacgagc 2400

ttccgaaaaa acatgctcga caaagagaag ttcaggaagt ttttggaccc caccctggag 2460

ctcagcgctg aatcttggaa cagcctgctg gaggtggccg atcttgctcg ccattgcacg 2520

gcgcgggaac cgtaccagcg gccagacatg ggtcactgcg tgaacaggct ttccagccta 2580

gtggaccagt ggaagccaac caacattgtt gatgatgacg atgaggaggg tgggacaagc 2640

gagatgggtc ttcaccagca cctagaaata tggagatgcg atgatttcac tatatcggat 2700

tcggattcat tcagcgcaca caacacatca aggaagtacc attga 2745

<210> 20
<211> 914
<212> PRT
<213> Zea mays

<400> 20

Met Gly Arg Ala Gly Phe Leu Phe Leu Pro Leu Leu Leu Leu Leu Ala
1               5                   10                  15

Phe Ala Gly Pro Gly Pro Ala Ser Ala Ala Thr Ala Met Thr Pro Asp
            20                  25                  30

Ala Gly Val Ile Leu Asp Leu Ala Lys Ser Leu Thr Asn Pro Pro Pro
        35                  40                  45

Ser Trp Thr Gly Thr Asp Val Cys Gly Gly Val Ser Phe Ser Gly Val
    50                  55                  60

Thr Cys Asn Gly Ala Ala Arg Val Thr Gly Ile Asn Leu Ala Lys Leu
65                  70                  75                  80

His Leu Ser Gly Thr Leu Ser Ser Ser Leu Ala Asn Leu Thr Ala Leu

                85                  90                  95

Gln Ser Leu Gln Leu Gln Gly Asn Ala Leu Glu Gly Asp Leu Pro Ser
            100                 105                 110

Leu Ala Gln Met Gly Ser Leu Glu Thr Leu Val Leu Asp Gly Asn Ala
        115                 120                 125

Phe Ser Thr Leu Pro Pro Asp Phe Leu Glu Gly Leu Pro Ser Leu Leu
    130                 135                 140

Lys Leu Ser Met Asp Asp Leu Pro Leu Glu Pro Trp Ser Ile Pro Asp
145                 150                 155                 160

Ala Ile Ala Gly Cys Ala Met Leu Gln Thr Phe Ser Ala Ser Asn Ala
                165                 170                 175

Ser Val Ser Gly Pro Phe Pro Ala Val Leu Ala Asn Leu Thr Ser Leu
            180                 185                 190

Gln Thr Leu Arg Leu Ser Tyr Asn Asn Leu Thr Gly Val Leu Pro Val
        195                 200                 205

Gly Leu Glu Ala Leu Gly Ser Leu Glu Thr Leu Gln Leu Asn Ser Gln
    210                 215                 220

Arg Ser Asn Gly Met Leu Ser Gly Pro Ile Asp Val Val Ala Lys Leu
225                 230                 235                 240

Pro Ser Leu Lys Thr Leu Trp Leu Gln Ser Asn Ser Phe Thr Gly Pro
                245                 250                 255

Ile Pro Glu Phe Asp Pro Asn Thr Gln Leu Glu Thr Phe Asn Val Arg
            260                 265                 270

Asp Asn Ser Leu Thr Gly Pro Val Pro Pro Ser Leu Val Gly Ile Thr
        275                 280                 285

Ser Leu Gln Asp Val Ala Leu Ser Asn Asn Phe Leu Gln Gly Pro Lys
    290                 295                 300

Pro Lys Phe Ala Ala Lys Thr Val Asp Ile Asp Ser Gly Asn Gly Phe
305                 310                 315                 320

Cys His Glu Asp Pro Gly Pro Cys Asp Pro Leu Val Thr Thr Leu Leu
                325                 330                 335

Gly Val Ala Ser Gly Phe Gly Tyr Pro Leu Gln Leu Lys Lys Trp Ala
        340                 345                 350

Gly Asn Asn Pro Cys Asp Pro Trp Pro Gly Leu Ser Cys Ile Lys Met
    355                 360                 365

Asp Val Thr Gln Ile Lys Leu Pro Arg Arg Asn Leu Ser Gly Leu Ile
    370                 375                 380

Ser Pro Ala Phe Ala Asn Leu Thr Arg Leu Gln Arg Leu Asp Leu Ser
385                 390                 395                 400

Asn Asn Arg Leu Thr Gly Val Ile Pro Asp Ala Leu Thr Thr Leu Lys
                405                 410                 415

Ser Leu Asn Tyr Leu Asp Val Ser Asn Asn Arg Leu Thr Gly Gln Val
            420                 425                 430

Pro Glu Phe Lys Gln His Ile Lys Leu Met Thr Ala Gly Asn Ser Phe
        435                 440                 445

Gly Glu Ser Gly Gly Asp Ser Gly Gly Gly Gly Ser Asn Val Arg Ser
    450                 455                 460

Ser Ser Ser Asn Pro Thr Gly Ser His Asn Ser Lys Ser Asn Ala Gly
465                 470                 475                 480

Met Ile Val Gly Ile Leu Leu Val Val Ile Leu Leu Val Ile Cys Val
                485                 490                 495

Gly Leu Phe Leu His His Arg Arg Asn Lys Asn Val Asp Lys Phe Ser
            500                 505                 510

Pro Ile Ser Thr Lys Ser Pro Ser Gly Glu Ser Glu Val Met Lys Ile
        515                 520                 525

Gln Val Val Gly Thr Asn Gly His Ser Asn Ile Ser Gly Ser Val Gly
    530                 535                 540

Ser Thr Glu Leu Tyr Ser His Ser Ser Ala Asp Asn Thr Ser Ile Ala
545                 550                 555                 560

Asp Leu Phe Glu Ser His Gly Met Gln Leu Pro Met Ser Val Leu Leu
                565                 570                 575

Lys Ala Thr Asn Asn Phe Asp Glu Asp Tyr Ile Leu Gly Thr Gly Gly
            580                 585                 590

Phe Gly Val Val Phe Lys Gly Thr Leu Asn Asp Lys Leu Val Ala Val
        595                 600                 605

Lys Arg Cys Asp Ser Gly Thr Met Gly Thr Lys Gly Leu Gln Glu Phe
    610                 615                 620

Met Ala Glu Ile Asp Val Leu Arg Lys Val Arg His Arg His Leu Val
625                 630                 635                 640

Ala Leu Leu Gly Tyr Cys Thr His Gly Asn Glu Arg Leu Leu Val Tyr
                645                 650                 655

Glu Tyr Met Ser Gly Gly Thr Leu Arg Gln His Leu Cys Asp Leu Gln
            660                 665                 670

Gln Ser Gly Tyr Thr Pro Leu Thr Trp Thr Gln Arg Met Thr Ile Ala
        675                 680                 685

Leu Asp Val Ala Arg Gly Ile Glu Tyr Leu His Gly Leu Ala Gln Glu
    690                 695                 700

Thr Phe Ile His Arg Asp Leu Lys Pro Ser Asn Ile Leu Leu Asp Gln
705                 710                 715                 720

Asp Leu Arg Ala Lys Val Ser Asp Phe Gly Leu Val Lys Leu Ala Lys
                725                 730                 735

Asp Thr Asp Lys Ser Met Met Thr Arg Val Ala Gly Thr Phe Gly Tyr
            740                 745                 750

Leu Ala Pro Glu Tyr Ala Thr Thr Gly Lys Val Thr Thr Lys Val Asp
        755                 760                 765

Val Tyr Ala Tyr Gly Val Ile Leu Met Glu Met Ile Thr Gly Arg Lys
    770                 775                 780

Val Leu Asp Asp Ser Leu Pro Asp Gly Glu Thr His Leu Val Thr Ser
785                 790                 795                 800

Phe Arg Lys Asn Met Leu Asp Lys Glu Lys Phe Arg Lys Phe Leu Asp
                805                 810                 815

Pro Thr Leu Glu Leu Ser Ala Glu Ser Trp Asn Ser Leu Leu Glu Val
            820                 825                 830

Ala Asp Leu Ala Arg His Cys Thr Ala Arg Glu Pro Tyr Gln Arg Pro
        835                 840                 845

Asp Met Gly His Cys Val Asn Arg Leu Ser Ser Leu Val Asp Gln Trp
    850                 855                 860

Lys Pro Thr Asn Ile Val Asp Asp Asp Asp Glu Glu Gly Gly Thr Ser
865                 870                 875                 880

Glu Met Gly Leu His Gln His Leu Glu Ile Trp Arg Cys Asp Asp Phe
                885                 890                 895

Thr Ile Ser Asp Ser Asp Ser Phe Ser Ala His Asn Thr Ser Arg Lys
            900                 905                 910

Tyr His

<210>  21
<211>  21
<212>  DNA
<213>  Artificial Sequence

<220>
<223>  diagnostic marker for Zm00001d049121

<400>  21
aagaccttca rccacatgat c                                                21

<210>  22
<211>  21
<212>  DNA
<213>  Artificial Sequence

<220>
<223>  diagnostic marker for Zm00001d049121

<400>  22
ggcacttccc ragcttgggg a                                                21

<210>  23
<211>  21
<212>  DNA
<213>  Artificial Sequence

<220>
<223>  diagnostic marker for Zm00001d049113

<400>  23
gtattaaaam wtcggtactg g                                                21

<210>  24
<211>  47
<212>  DNA
<213>  Artificial Sequence

<220>
<223>  marker allele A - primer allele X

<400>  24
gaaggtgacc aagttcatgc ttccgctagc tagatgattc tcattta                47


<210>  25
<211>  46
<212>  DNA
<213>  Artificial Sequence

<220>
<223>  marker allele A - primer allele Y

<400>  25
gaaggtcgga gtcaacggat tccgctagct agatgattct catttc                 46


<210>  26
<211>  25
<212>  DNA
<213>  Artificial Sequence

<220>
<223>  marker allele A - primer common

<400>  26
cacacgactg ttgaaccgtt ccaat                                        25


<210>  27
<211>  40
<212>  DNA
<213>  Artificial Sequence

<220>
<223>  marker allele B - primer allele X

<400>  27
gaaggtgacc aagttcatgc tcacacccat gatgttaccg                        40


<210>  28
<211>  43
<212>  DNA
<213>  Artificial Sequence

<220>
<223>  marker allele B - primer allele Y

<400>  28
gaaggtcgga gtcaacggat tgctcacacc catgatgtta cca                    43


<210>  29
<211>  24
<212>  DNA
<213>  Artificial Sequence

<220>
<223>  marker allele B - primer common

<400>  29
tcggataaga accctgcagg agat                                              24


<210>  30
<211>  44
<212>  DNA
<213>  Artificial Sequence

<220>
<223>  marker allele C - primer allele X

<400>  30
gaaggtgacc aagttcatgc tggcgctcta cctaaatcca catc                        44


<210>  31
<211>  44
<212>  DNA
<213>  Artificial Sequence

<220>
<223>  marker allele C - primer allele Y

<400>  31
gaaggtcgga gtcaacggat tggcgctcta cctaaatcca cata                        44


<210>  32
<211>  29
<212>  DNA
<213>  Artificial Sequence

<220>
<223>  marker allele C - primer common

<400>  32
caggattgac acaagaatct gataccaat                                         29


<210>  33
<211>  31
<212>  DNA
<213>  Artificial Sequence

<220>
<223>  marker allele M7

<400>  33
gtacgtccga gtgacctcct gctccttgga g                                      31

## Description

### FIELD OF THE INVENTION

The invention relates to maize plants having increased pathogen resistance or tolerance, in particular increased resistance or tolerance to *Exserohilum turcicum.*

### BACKGROUND OF THE INVENTION

In maize (Zea mays L.), there are a large number of fungal pathogens which cause leaf diseases. The fungus which can cause by far the most damage under tropical and also under temperate climatic conditions, such as those in large parts of Europe and North America as well as in Africa and India, is known as *Helminthosporium turcicum* or synonymously as *Exserohilum turcicum* (Pass.) Leonard and Suggs (teleomorph: *Setosphaeria turcica* (Luttrell) Leonard & Suggs). *H. turcicum* is the cause of the leaf spot disease known as "Northern Corn Leaf Blight" (NCLB), which can occur in epidemic proportions during wet years, attacking vulnerable maize varieties and causing a great deal of damage and loss of yield of 30% and more over wide areas (Perkins & Pedersen, 1987, Disease development and yield losses associated with northern leaf blight on corn. Plant Disease, 71(10), 940-943.; Raymundo et al., 1981, Effect of gene HtN on the development of northern corn leaf blight epidemics. Plant disease.). Since the 1970s, natural resistance in genetic material has been sought. Currently, both quantitative and qualitative resistance are known. While the oligo- or polygenically inherited quantitative resistance appears incomplete and non-specific with regard to race and is influenced by additional and partially dominant genes, qualitative resistance is typically race-specific and can be inherited through individual, mostly dominant genes such as Ht1, Ht2, Ht3, Htm1 or Htn1 (Lipps et al., 1997; Welz & Geiger, 2000). Backcrosses in many frequently used inbred maize lines such as W22, A619, B37 or B73 have successfully brought about introgression of the HT genes, where they exhibit a partial dominance and expression as a function of the respective genetic background (Welz, 1998, Genetics and epidemiology of the pathosystem Zea mays/Setosphaeria turcica." Habilitationsschrift Institut for Pflanzenzüchtung, Saatgutforschung and Populationsgenetik, Universität Hohenheim.).

Despite this complex genetic architecture of NCLB resistance in maize, until now principally the use of the Ht1 gene in maize together with a partial quantitative resistance has been sufficient to control helminthrosporiosis (Welz, 1998). The basis for this is that globally, race 0 of *H. turcicum* dominates as regards to occurrence (approximately 55%) (Lipps et al., 1997, Interaction of Ht and partial resistance to Exserohilum turcicum in maize. Plant Disease 81: 277-282.; Ferguson & Carson, 2007, Temporal Variation in Setosphaeria turcica Between 1974 and 1994 and Origin of Races 1, 23, and 23N in the United States." Phytopathology 97: 1501-1511.), while other races such as 2N and 23N only rarely occur and then even in a geographically restricted area (Moghaddam & Pataky, 1994, Reactions for isolates from mating of races 1 and 23N of Exserohilum turcicum. Plant Disease 78: 767-771.; Jordan et al., 1983, Occurrence of race 2 of Exserohilum turcicum on corn in the central and eastern United States. Plant Disease 67: 1163-1165.; Lipps & Hite, 1982, Exserohilum turcicum virulent on corn with the Ht resistance gene in Ohio. Plant Disease 66: 397-398.; Thakur et al., 1989, Effects of temperature and light on virulence of Exserohilum turcicum on corn." Phytopathology 1989, 79: 631-635.; Welz, 1998). This race 0 is avirulent with regard to a maize plant with Ht1, so that when provided with a suitable quantitative resistance, the maize plant exhibits a sufficient general resistance to NCLB.

However, many studies have reported an increasing dissemination of the less common races (Jordan et al., 1983; Welz, 1998; Pratt & Gordon, 2006, Breeding for resistance to maize foliar pathogens." Plant Breed Rev. 27: 119-173.). The reasons for this are linked to the population dynamic of a pathogen which allows changes in pathogen virulence by new mutations on avirulence genes and new combinations of available virulence genes. Finally, this can lead to the occurrence of new, suitable, sometimes more aggressive pathogenic races. In Brazil, for example, the *H. turcicum* population already appears to be substantially more diverse having regard to the race composition than, for example, in North America. Gianasi et al. (1996, Raçes fisiológocas de Exserohilum turcicum identificadas em regiões produtoras de milho no Brasil, Safra 93/94." Summa Phytopathol. 22: 214-217.) reported *H. turcicum* races which have already broken through the resistance conferred by the Ht1 gene. In addition, there is the instability of the resistance genes to certain environmental factors such as temperature and light intensity in some climate zones (Thakur et al., 1989). As a result the use of novel HT resistance genes which, until now received little attention, is getting more important globally for the production commercial maize plants, so as to ensure a broader and more long-lasting resistance to *H. turcicum* in maize. Initial approaches in this regard were attempted as early as 1998 by Pataky et al. (Disease severity and yield of sweet corn hybrids with resistance to Northern Leaf Blight. Plant Disease 82(1): 57-63.). The NCLB resistance in sh2 elite maize was improved by using a combination of Ht1 and Htn1.

In the meantime, many sources of new NCLB resistance have been identified (Galiano-Carneiro, A. L., & Miedaner, T. (2017). Genetics of Resistance and Pathogenicity in the Maize/Setosphaeria turcica Pathosystem and Implications for Breeding. Frontiers in plant science, 8, 1490.), however the molecular characterisation is often lacking. Marker development is very difficult and the optimized use of these resistances in breeding is limited. Nevertheless, there is a continuing need for the well-characterized resistance genes which can then be used for the development of novel NCLB-resistant plant varieties.

### SUMMARY OF THE INVENTION

The present invention relates to maize plants having increased pathogen resistance or tolerance, in particular increased resistance or tolerance to pathogens causing Northern Corn Leaf Blight, i.e. *Exserohilum turcicum* (also known as *Helminthosporium turcicum*). Such maize plants can be characterized as having a particular QTL allele or particular molecular markers, as defined herein elsewhere. Such maize plants can be characterized as having a particular resistance gene, as defined herein elsewhere. The invention further relates to methods for generating such maize plants, as well as methods for identifying such maize plants. The invention further relates to the use of such maize plants in controlling pathogen infestation. The invention also relates to (isolated) polynucleic acids, such as polynucleic acids comprising or encoding the QTL, molecular markers, or resistance genes or polynucleic acids specifically hybridizing therewith, as well as the use of such polynucleic acids for identifying or generating the maize plants of the invention. The invention also relates to maize plants or plant parts thereof, obtained by or obtainable by the method as described herein, as well as maize plants or plant parts thereof comprising the polynucleic acids as described herein.

The present invention is in particular captured by any one or any combination of one or more of the below numbered statements 1 to 72, with any other statement and/or embodiments.

A method for identifying a maize plant or plant part, preferably a maize plant or plant part having increased pathogen resistance and/or tolerance, preferably wherein said pathogen is *Exserohilum turcicum,* comprising screening for the presence of a QTL allele located on chromosome 4 in a maize plant or plant part, wherein said QTL allele is located on a chromosomal interval flanked by marker alleles A and B, wherein marker alleles A and B are SNPs which are respectively adenine (A) at the position 16107190 bp on reference genome B73 AGPv04 and cytosine (C) at the position 18470371 bp on reference genome B73 AGPv04.

Marker allele A can be determined or detected by use of primer sequences as set forth in SEQ ID NOs: 24 to 26, and marker allele B by primer sequences as set forth in SEQ ID NOs: 27 to 29, preferably in a KASP marker system (Table 1A). Marker alleles may be genetically linked to the presence of the QTL. The QTL is associated with said increased pathogen resistance and/or tolerance.

The method according to statement [01], wherein said QTL allele is flanked by marker alleles A and/or B.

The method according to statement [01], wherein said QTL allele is located on a chromosomal interval comprising marker alleles A and C, wherein marker alleles A and C are SNPs which are respectively adenine (A) at the position 16107190 bp on reference genome B73 AGPv04 and thymine (T) at the position 22847078 bp on reference genome B73 AGPv04.

Marker allele A can be determined or detected by use of primer sequences as set forth in SEQ ID NOs: 24 to 26, and marker allele C by primer sequences as set forth in SEQ ID NOs: 30 to 32, preferably in a KASP marker system (Table 1A). Marker alleles may be genetically linked to the presence of the QTL.

The method according to statement [03], wherein said QTL allele is flanked by marker alleles A and/or C.

The method according to any of statements [01] to [04], wherein said QTL allele comprises one or more of marker alleles M1 to M56, wherein
- M1 is a SNP which is thymine (T) at the position 1071 of the coding sequence of Zm00001d049121 referenced to the B73 reference genome AGPv4;
- M2 is a SNP which is guanine (G) at the position 1302 of the coding sequence of Zm00001d049121 referenced to the B73 reference genome AGPv4;
- M3 is a SNP which is cytosine (C) at the position 1497 of the coding sequence of Zm00001d049121 referenced to the B73 reference genome AGPv4;
- M4 is a SNP which is guanine (G) at the position 1631 of the coding sequence of Zm00001d049121 referenced to the B73 reference genome AGPv4;
- M5 is a SNP which is cytosine (C) at the position 1691 of the coding sequence of Zm00001d049121 referenced to the B73 reference genome AGPv4;
- M6 is an indel which is deletion of the nucleotide at the position 1807 of the coding sequence of Zm00001d049121 referenced to the B73 reference genome AGPv4;
- M7 is an indel which is an insertion of one or more nucleotides between nucleotides at positions 1815 and 1816 of the coding sequence of Zm00001d049121 referenced to the B73 reference genome AGPv4;
- M8 is a SNP which is guanine (G) at the position 2045 of the coding sequence of Zm00001d049121 referenced to the B73 reference genome AGPv4;
- M9 is an indel which is deletion of nucleotides at positions 2424-2429 of Zm00001d049121 referenced to the B73 reference genome AGPv4;
- M10 is a SNP which is cytosine (C) at the position 2458 of the coding sequence of Zm00001d049121 referenced to the B73 reference genome AGPv4;
- M11 is a SNP which is adenine (A) at the position 2530 of the coding sequence of Zm00001d049121 referenced to the B73 reference genome AGPv4;
- M12 is a SNP which is adenine (A) at the position 2683 of the coding sequence of Zm00001d049121 referenced to the B73 reference genome AGPv4;
- M13 is a SNP which is cytosine (C) at the position 12 of the coding sequence of Zm00001d049113 referenced to the B73 reference genome AGPv4;
- M14 is a SNP which is cytosine (C) at the position 291 of the coding sequence of Zm00001d049113 referenced to the B73 reference genome AGPv4;
- M15 is a SNP which is thymine (T) at the position 309 of the coding sequence of Zm00001d049113 referenced to the B73 reference genome AGPv4;
- M16 is a SNP which is thymine (T) at the position 324 of the coding sequence of Zm00001d049113 referenced to the B73 reference genome AGPv4;
- M17 is an indel which is deletion of nucleotides at positions 415-417 of the coding sequence of Zm00001d049113 referenced to the B73 reference genome AGPv4;
- M18 is a SNP which is guanine (G) at the position 195 of the coding sequence of Zm00001d049145 referenced to the B73 reference genome AGPv4;
- M19 is a SNP which is guanine (G) at the position 247 of the coding sequence of Zm00001d049145 referenced to the B73 reference genome AGPv4;
- M20 is a SNP which is cytosine (C) at the position 248 of the coding sequence of Zm00001d049145 referenced to the B73 reference genome AGPv4;
- M21 is a SNP which is thymine (T) at the position 1097 of the coding sequence of Zm00001d049145 referenced to the B73 reference genome AGPv4;
- M22 is a SNP which is cytosine (C) at the position 1105 of the coding sequence of Zm00001d049145 referenced to the B73 reference genome AGPv4;
- M23 is a SNP which is thymine (T) at the position 1159 of the coding sequence of Zm00001d049145 referenced to the B73 reference genome AGPv4;
- M24 is a SNP which is thymine (T) at the position 1185 of the coding sequence of Zm00001d049145 referenced to the B73 reference genome AGPv4;
- M25 is an indel which is an insertion of one or more nucleotides between nucleotides at positions 1216 and 1217 of the coding sequence of Zm00001d049145 referenced to the B73 reference genome AGPv4;
- M26 is a SNP which is cytosine (C) at the position 1242 of the coding sequence of Zm00001d049145 referenced to the B73 reference genome AGPv4;
- M27 is a SNP which is guanine (G) at the position 1282 of the coding sequence of Zm00001d049145 referenced to the B73 reference genome AGPv4;
- M28 is a SNP which is guanine (G) at the position 1344 of the coding sequence of Zm00001d049145 referenced to the B73 reference genome AGPv4;
- M29 is a SNP which is cytosine (C) at the position 1380 of the coding sequence of Zm00001d049145 referenced to the B73 reference genome AGPv4;
- M30 is a SNP which is cytosine (C) at the position 1422 of the coding sequence of Zm00001d049145 referenced to the B73 reference genome AGPv4;
- M31 is a SNP which is cytosine (C) at the position 338 of the coding sequence of Zm00001d049148 referenced to the B73 reference genome AGPv4;
- M32 is a SNP which is adenine (A) at the position 412 of the coding sequence of Zm00001d049148 referenced to the B73 reference genome AGPv4;
- M33 is a SNP which is thymine (T) at the position 432 of the coding sequence of Zm00001d049148 referenced to the B73 reference genome AGPv4;
- M34 is a SNP which is guanine (G) at the position 447 of the coding sequence of Zm00001d049148 referenced to the B73 reference genome AGPv4;
- M35 is a SNP which is thymine (T) at the position 507 of the coding sequence of Zm00001d049148 referenced to the B73 reference genome AGPv4;
- M36 is a SNP which is thymine (T) at the position 509 of the coding sequence of Zm00001d049148 referenced to the B73 reference genome AGPv4;
- M37 is a SNP which is thymine (T) at the position 585 of the coding sequence of Zm00001d049148 referenced to the B73 reference genome AGPv4;
- M38 is a SNP which is thymine (T) at the position 588 of the coding sequence of Zm00001d049148 referenced to the B73 reference genome AGPv4;
- M39 is a SNP which is adenine (A) at the position 593 of the coding sequence of Zm00001d049148 referenced to the B73 reference genome AGPv4;
- M40 is a SNP which is cytosine (C) at the position 672 of the coding sequence of Zm00001d049148 referenced to the B73 reference genome AGPv4;
- M41 is a SNP which is thymine (T) at the position 741 of the coding sequence of Zm00001d049148 referenced to the B73 reference genome AGPv4;
- M42 is a SNP which is guanine (G) at the position 753 of the coding sequence of Zm00001d049148 referenced to the B73 reference genome AGPv4;
- M43 is a SNP which is cytosine (C) at the position 846 of the coding sequence of Zm00001d049148 referenced to the B73 reference genome AGPv4;
- M44 is a SNP which is thymine (T) at the position 996 of the coding sequence of Zm00001d049148 referenced to the B73 reference genome AGPv4;
- M45 is a SNP which is cytosine (C) at the position 1092 of the coding sequence of Zm00001d049148 referenced to the B73 reference genome AGPv4;
- M46 is a SNP which is thymine (T) at the position 1102 of the coding sequence of Zm00001d049148 referenced to the B73 reference genome AGPv4;
- M47 is a SNP which is cytosine (C) at the position 1260 of the coding sequence of Zm00001d049148 referenced to the B73 reference genome AGPv4;
- M48 is a SNP which is thymine (T) at the position 1300 of the coding sequence of Zm00001d049148 referenced to the B73 reference genome AGPv4;
- M49 is a SNP which is cytosine (C) at the position 22 of the coding sequence of Zm00001d049159 referenced to the B73 reference genome AGPv4;
- M50 is a SNP which is thymine (T) at the position 47 of the coding sequence of Zm00001d049159 referenced to the B73 reference genome AGPv4;
- M51 is an indel which is an insertion of one or more nucleotides between nucleotides at positions 52 and 53 of the coding sequence of Zm00001d049159 referenced to the B73 reference genome AGPv4;
- M52 is a SNP which is adenine (A) at the position 189 of the coding sequence of Zm00001d049159 referenced to the B73 reference genome AGPv4;
- M53 is a SNP which is adenine (A) at the position 190 of the coding sequence of Zm00001d049159 referenced to the B73 reference genome AGPv4;
- M54 is a SNP which is guanine (G) at the position 1980 of the coding sequence of Zm00001d049159 referenced to the B73 reference genome AGPv4;
- M55 is a SNP which is guanine (G) at the position 2014 of the coding sequence of Zm00001d049159 referenced to the B73 reference genome AGPv4;
- M56 is a SNP which is guanine (G) at the position 2160 of the coding sequence of Zm00001d049159 referenced to the B73 reference genome AGPv4.

The method according to statement [05], wherein M7 is an insertion of 31 nucleotides.

The method according to statement [06], wherein M7 is an insertion of a polynucleic acid sequence as set forth in SEQ ID NO: 33.

The method according to statement [05], wherein M25 is an insertion of 3 nucleotides.

The method according to statement [06], wherein M25 is an insertion of a polynucleic acid sequence of GTC.

The method according to statement [05], wherein M51 is an insertion of 6 nucleotides.

The method according to statement [06], wherein M51 is an insertion of a polynucleic acid sequence of CCGGCC.

The method according to statement [05], wherein the coding sequence of
- Zm00001d049121 referenced to the B73 reference genome AGPv4 comprises a sequence as set forth in SEQ ID NO: 11;
- Zm00001d049113 referenced to the B73 reference genome AGPv4 comprises a sequence as set forth in SEQ ID NO: 13;
- Zm00001d049145 referenced to the B73 reference genome AGPv4 comprises a sequence as set forth in SEQ ID NO: 15;
- Zm00001d049148 referenced to the B73 reference genome AGPv4 comprises a sequence as set forth in SEQ ID NO: 17; and/or
- Zm00001d049159 referenced to the B73 reference genome AGPv4 comprises a sequence as set forth in SEQ ID NO: 19.

The method according to statement [05], wherein
- Zm00001d049121 referenced to the B73 reference genome AGPv4 encodes a sequence as set forth in SEQ ID NO: 12;
- Zm00001d049113 referenced to the B73 reference genome AGPv4 encodes a sequence as set forth in SEQ ID NO: 14;
- Zm00001d049145 referenced to the B73 reference genome AGPv4 encodes a sequence as set forth in SEQ ID NO: 16;
- Zm00001d049148 referenced to the B73 reference genome AGPv4 encodes a sequence as set forth in SEQ ID NO: 18; and/or
- Zm00001d049159 referenced to the B73 reference genome AGPv4 encodes a sequence as set forth in SEQ ID NO: 20.

The method according to any of statements [01] to [13], wherein screening for the presence of said QTL allele comprises identifying any one or more of marker alleles A, B, or C and/or identifying any one or more of marker alleles selected from M1 to M56.

A method for identifying a maize plant or plant part, preferably a maize plant or plant part having increased pathogen resistance and/or tolerance, preferably wherein said pathogen is *Exserohilum turcicum,* comprising screening for the presence of one or more marker allele selected from M1 to M56.

The method according to statement [15], comprising screening for the presence of
- one or more marker allele selected from M1 to M12;
- one or more marker allele selected from M13 to M17;
- one or more marker allele selected from M18 to M30;
- one or more marker allele selected from M31 to M48; and/or
- one or more marker allele selected from M49 to M56.

A method for identifying a maize plant or plant part, preferably a maize plant or plant part having increased pathogen resistance and/or tolerance, preferably wherein said pathogen is *Exserohilum turcicum,* comprising screening for the presence of
- Zm00001d049121 comprising a coding sequence as set forth in SEQ ID NO: 1 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence;
- Zm00001d049113 comprising a coding sequence as set forth in SEQ ID NO: 3 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence;
- Zm00001d049145 comprising a coding sequence as set forth in SEQ ID NO: 5 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence;
- Zm00001d049148 comprising a coding sequence as set forth in SEQ ID NO: 7 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence; and/or
- Zm00001d049159 comprising a coding sequence as set forth in SEQ ID NO: 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

A method for identifying a maize plant or plant part, preferably a maize plant or plant part having increased pathogen resistance and/or tolerance, preferably wherein said pathogen is *Exserohilum turcicum,* comprising screening for the presence of
- Zm00001d049121 encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence;
- Zm00001d049113 encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence;
- Zm00001d049145 encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence;
- Zm00001d049148 encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence; and/or
- Zm00001d049159 encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

The method according to any of statements [01] to [18], further comprising selecting a plant or plant part comprising said QTL allele, one or more of marker allele selected from M1 to M56, one or more polynucleic acid having a coding sequence selected from SEQ ID NOs: 1, 3, 5, 7, or 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 1, 3, 5, 7, or 9, preferably over the entire length of the sequence, or one or more polynucleic acid encoding a sequence of any of SEQ ID NOs: 2, 4, 6, 8, or 10 or encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 2, 4, 6, 8, or 10, preferably over the entire length of the sequence, preferably over the entire length of the sequence.

The method according to any of statements [01] to [19], wherein a plant or plant part is identified as having increased resistance and/or tolerance to a pathogen if said QTL, one or more of said marker alleles, and/or one or more of said polynucleic acids as defined in statement [19] are present.

A method for generating a maize plant, preferably a maize plant having increased pathogen resistance and/or tolerance, or a part thereof, preferably wherein said pathogen is *Exserohilum turcicum,* comprising introducing into the genome of a plant or a plant part a QTL allele as defined in any of statements [01] to [14].

A method for generating a maize plant, preferably a maize plant having increased pathogen resistance and/or tolerance, or a part thereof, preferably wherein said pathogen is *Exserohilum turcicum,* comprising introducing (and expressing) into the genome of a plant or a plant part
- A polynucleic acid of an exogenous Zm00001d049121 comprising a coding sequence as set forth in SEQ ID NO: 1 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence;
- a polynucleic acid of an exogenous Zm00001d049113 comprising a coding sequence as set forth in SEQ ID NO: 3 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence;
- a polynucleic acid of an exogenous Zm00001d049145 comprising a coding sequence as set forth in SEQ ID NO: 5 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence;
- a polynucleic acid of an exogenous Zm00001d049148 comprising a coding sequence as set forth in SEQ ID NO: 7 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence; and/or
- a polynucleic acid of an exogenous Zm00001d049159 comprising a coding sequence as set forth in SEQ ID NO: 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

A method for generating a maize plant, preferably a maize plant having increased pathogen resistance and/or tolerance, or a part thereof, preferably wherein said pathogen is *Exserohilum turcicum,* comprising introducing (and expressing) into the genome of a plant or plant part
- a polynucleic acid of an exogenous Zm00001d049121 encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence;
- a polynucleic acid of an exogenous Zm00001d049113 encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence;
- a polynucleic acid of an exogenous Zm00001d049145 encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence;
- a polynucleic acid of an exogenous Zm00001d049148 encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence; and/or
- a polynucleic acid of an exogenous Zm00001d049159 encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

A method for generating a maize plant, preferably a maize plant having increased pathogen resistance and/or tolerance, or a part thereof, preferably wherein said pathogen is *Exserohilum turcicum,* comprising
- mutating endogenous Zm00001d049121 to a sequence as set forth in SEQ ID NO: 1 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence;
- mutating endogenous Zm00001d049113 to a sequence as set forth in SEQ ID NO: 3 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence;
- mutating endogenous Zm00001d049145 to a sequence as set forth in SEQ ID NO: 5 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence;
- mutating endogenous Zm00001d049148 to a sequence as set forth in SEQ ID NO: 7 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence; and/or
- mutating endogenous Zm00001d049159 to a sequence as set forth in SEQ ID NO: 9 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

A method for generating a maize plant, preferably a maize plant having increased pathogen resistance and/or tolerance, or a part thereof, preferably wherein said pathogen is *Exserohilum turcicum,* comprising
- mutating endogenous Zm00001d049121 to a sequence encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence;
- mutating endogenous Zm00001d049113 to a sequence encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence;
- mutating endogenous Zm00001d049145 to a sequence encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence;
- mutating endogenous Zm00001d049148 to a sequence encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence; and/or
- mutating endogenous Zm00001d049159 to a sequence encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

A method for generating a maize plant, preferably a maize plant having increased pathogen resistance and/or tolerance, or a part thereof, preferably wherein said pathogen is *Exserohilum turcicum,* comprising introducing (and expressing) or mutating into the genome of a plant or a plant part
- a polynucleic acid encoding Zm00001d049121 comprising one or more marker allele selected from M1 to M12;
- a polynucleic acid encoding Zm00001d049121 comprising one or more marker allele selected from M13 to M17;
- a polynucleic acid encoding Zm00001d049121 comprising one or more marker allele selected from M18 to M30;
- a polynucleic acid encoding Zm00001d049121 comprising one or more marker allele selected from M31 to M48; and/or
- a polynucleic acid encoding Zm00001d049121 comprising one or more marker allele selected from M49 to M56.

The method according to any of statements [21] to [26], wherein introducing into the genome comprises transgenesis or mutagenesis. Preferably, mutagenesis is based on TILLING, CRISPR-mediated gene editing and/or base editing

The method according to any of statements [21] to [26], wherein introducing into the genome comprises introgression.

The method according to any of statements [21] to [28], wherein said plant part is a cell, tissue, organ or seed.

The method according to statement [29], wherein said plant part is a protoplast, a callus, an immature or mature embryo or an inflorescence.

The method according to any of statements [21] to [30], comprising transforming a plant or plant part, preferably a plant cell or a plant tissue, more preferably a protoplast, a callus, an immature or mature embryo or an inflorescence, with a polynucleic acid comprising a QTL allele as defined in any of statements [01] to [14], and optionally regenerating a plant or plant part from said plant cell or said plant tissue, preferably said protoplast, said callus, said immature or mature embryo or said inflorescence.

The method according to any of statements [21] to [31], comprising transforming a plant or plant part, preferably a plant cell or a plant tissue, more preferably a protoplast, a callus, an immature or mature embryo or an inflorescence, with one or more polynucleic acid comprising one or more coding sequence selected from SEQ ID NOs: 1, 3, 5, 7, and 9, or with one or more polynucleic acid comprising a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 1, 3, 5, 7, and 9, preferably over the entire length of the sequence, and optionally regenerating a plant from said plant cell or said tissue, preferably said protoplast, said callus, said immature or mature embryo or said inflorescence.

The method according to any of statements [21] to [31], comprising transforming a plant or plant part, preferably a plant cell or a plant tissue, more preferably a protoplast, a callus, an immature or mature embryo or an inflorescence, with one or more polynucleic acid encoding one or more sequence selected from SEQ ID NOs: 2, 4, 6, 8, and 10 or with a polynucleic acid encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 2, 4, 6, 8, and 10, preferably over the entire length of the sequence, and optionally regenerating a plant from said plant cell or said tissue, preferably said protoplast, said callus, said immature or mature embryo or said inflorescence.

The method according to any of statements [21] to [33], wherein said QTL allele which is introduced has a sequence different to the corresponding endogenous QTL allele of the plant.

The method according to any of statements [22] to [34], wherein said polynucleic acid which is introduced has a sequence different to the corresponding endogenous polynucleic acid of the plant.

A method for generating a maize plant or plant part, preferably a maize plant or plant part having increased pathogen resistance and/or tolerance, preferably wherein said pathogen is *Exserohilum turcicum,* comprising (a) providing a first maize plant identified according to any of statements [01] to [20] or generated according to any of statements [21] to [34], (b) crossing said first maize plant with a second maize plant, (c) selecting progeny plants comprising said QTL allele, any one or more of marker alleles A, B, or C, any one or more of marker alleles selected from M1 to M56, a polynucleic acid sequence as set forth in any one or more sequence selected from SEQ ID NOs: 1, 3, 5, 7, and 9 or comprising a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 1, 3, 5, 7, and 9, preferably over the entire length of the sequence, and/or a polynucleotide sequence encoding a protein as set forth in any one or more sequence selected from SEQ ID NOs: 2, 4, 6, 8, and 10 or encoding a protein comprising a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 2, 4, 6, 8, and 10, preferably over the entire length of the sequence; and optionally (d) harvesting or obtaining said maize plant or plant part from said progeny.

The method according to any of statements [01] to [36], wherein said plant or plant part has increased resistance and/or tolerance to a pathogen.

The method according to statement [20] or [37], wherein said pathogen is a fungus, preferably causing Northern Corn Leaf Blight.

The method according to any of statements [20], [37], or [38], wherein said pathogen is an *Exserohilum sp.*

The method according to any of statements [20] or [37] to [39], wherein said pathogen is *Exserohilum turcicum.*

The method according to any of statements [01] to [40] wherein
- endogenous Zm00001d049121 comprises a coding sequence as set forth in SEQ ID NO: 11 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 11, preferably over the entire length of the sequence;
- endogenous Zm00001d049113 comprises a coding sequence as set forth in SEQ ID NO: 13 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 13, preferably over the entire length of the sequence;
- endogenous Zm00001d049145 comprises a coding sequence as set forth in SEQ ID NO: 15 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 15, preferably over the entire length of the sequence;
- endogenous Zm00001d049148 comprises a coding sequence as set forth in SEQ ID NO: 17 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 17, preferably over the entire length of the sequence; and/or
- endogenous Zm00001d049159 comprises a coding sequence as set forth in SEQ ID NO: 19 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 19, preferably over the entire length of the sequence.

The method according to any of statements [01] to [40] wherein
- endogenous Zm00001d049121 encodes a protein sequence as set forth in SEQ ID NO: 12 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 12, preferably over the entire length of the sequence;
- endogenous Zm00001d049113 encodes a protein sequence as set forth in SEQ ID NO: 14 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 14, preferably over the entire length of the sequence;
- endogenous Zm00001d049145 encodes a protein sequence as set forth in SEQ ID NO: 16 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 16, preferably over the entire length of the sequence;
- endogenous Zm00001d049148 encodes a protein sequence as set forth in SEQ ID NO: 18 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 18, preferably over the entire length of the sequence; and/or
- endogenous Zm00001d049159 encodes a protein sequence as set forth in SEQ ID NO: 20 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 20, preferably over the entire length of the sequence.

The method according to any of statements [01] to [40] wherein
- endogenous Zm00001d049121 comprises
   (i) a nucleotide sequence having the cDNA of SEQ ID NO: 11;
   (ii) a nucleotide sequence encoding for an amino acid sequence having the amino acid sequence of SEQ ID NO: 12;
   (iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 11, preferably over the entire length of the sequence;
- (iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 12, preferably over the entire length of the sequence;
   (v) a nucleotide sequence hybridizing with the reverse complement of a nucleotide sequence as defined in (i) or (ii) under stringent hybridization conditions; or
   (vi) a nucleotide sequence encoding a protein derived from the amino acid sequence encoded by the nucleotide sequence of (i) to (v) by way of substitution, deletion and/or addition of one or more amino acid(s);
- endogenous Zm00001d049113 comprises
   (i) a nucleotide sequence having the cDNA of SEQ ID NO 13;
   (ii) a nucleotide sequence encoding for an amino acid sequence having the amino acid sequence of SEQ ID NO: 14;
   (iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 13, preferably over the entire length of the sequence;
   (iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 14, preferably over the entire length of the sequence;
   (v) a nucleotide sequence hybridizing with the reverse complement of a nucleotide sequence as defined in (i) or (ii) under stringent hybridization conditions; or
   (vi) a nucleotide sequence encoding a protein derived from the amino acid sequence encoded by the nucleotide sequence of (i) to (v) by way of substitution, deletion and/or addition of one or more amino acid(s);
- endogenous Zm00001d049145 comprises
   (i) a nucleotide sequence having the cDNA of SEQ ID NO: 15;
   (ii) a nucleotide sequence encoding for an amino acid sequence having the amino acid sequence of SEQ ID NO: 16;
   (iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 15, preferably over the entire length of the sequence;
   (iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 16, preferably over the entire length of the sequence;
   (v) a nucleotide sequence hybridizing with the reverse complement of a nucleotide sequence as defined in (i) or (ii) under stringent hybridization conditions; or
   (vi) a nucleotide sequence encoding a protein derived from the amino acid sequence encoded by the nucleotide sequence of (i) to (v) by way of substitution, deletion and/or addition of one or more amino acid(s);
- endogenous Zm00001d049148 comprises
   (i) a nucleotide sequence having the cDNA of SEQ ID NO: 17;
   (ii) a nucleotide sequence encoding for an amino acid sequence having the amino acid sequence of SEQ ID NO: 18;
   (iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 17, preferably over the entire length of the sequence;
   (iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 18, preferably over the entire length of the sequence;
   (v) a nucleotide sequence hybridizing with the reverse complement of a nucleotide sequence as defined in (i) or (ii) under stringent hybridization conditions; or
   (vi) a nucleotide sequence encoding a protein derived from the amino acid sequence encoded by the nucleotide sequence of (i) to (v) by way of substitution, deletion and/or addition of one or more amino acid(s); and/or
- endogenous Zm00001d049159 comprises
   (i) a nucleotide sequence having the cDNA of SEQ ID NO: 19;
   (ii) a nucleotide sequence encoding for an amino acid sequence having the amino acid sequence of SEQ ID NO: 20;
   (iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 19, preferably over the entire length of the sequence;
   (iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 20, preferably over the entire length of the sequence;
   (v) a nucleotide sequence hybridizing with the reverse complement of a nucleotide sequence as defined in (i) or (ii) under stringent hybridization conditions; or
   (vi) a nucleotide sequence encoding a protein derived from the amino acid sequence encoded by the nucleotide sequence of (i) to (v) by way of substitution, deletion and/or addition of one or more amino acid(s).

The method according to statement [43], wherein
- endogenous Zm00001d049121 does not comprise one or more of marker alleles M1 to M12;
- endogenous Zm00001d049113 does not comprise one or more of marker alleles M13 to M17;
- endogenous Zm00001d049145 does not comprise one or more of marker alleles M18 to M30;
- endogenous Zm00001d049148 does not comprise one or more of marker alleles M31 to M48; and/or
- endogenous Zm00001d049159 does not comprise one or more of marker alleles M49 to M56.

The method according to statement [43] or [44], wherein
- endogenous Zm00001d049121 does not comprise a sequence as set forth in SEQ ID NO: 1;
- endogenous Zm00001d049113 does not comprise a sequence as set forth in SEQ ID NO: 3;
- endogenous Zm00001d049145 does not comprise a sequence as set forth in SEQ ID NO: 5;
- endogenous Zm00001d049148 does not comprise a sequence as set forth in SEQ ID NO: 7; and/or
- endogenous Zm00001d049159 does not comprise a sequence as set forth in SEQ ID NO: 9.

A maize plant, preferably a maize plant having increased pathogen resistance and/or tolerance, preferably wherein said pathogen is *Exserohilum turcicum* obtainable by the method according to any of statements [21] to [45], or a plant part thereof, or the progeny thereof.

A maize plant, preferably a maize plant having increased pathogen resistance and/or tolerance, preferably wherein said pathogen is *Exserohilum turcicum* comprising a QTL allele as defined in any of statements [01] to [14], one or more marker allele selected from M1 to M56, one or more polynucleic acid having a coding sequence selected from SEQ ID NOs: 1, 3, 5, 7, and 9, one or more polynucleic acid having a coding sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 1, 3, 5, 7, and 9, preferably over the entire length of the sequence, one or more polynucleic acid encoding a sequence selected from SEQ ID NOs: 2, 4, 6, 8, and 10 and/or one or more polynucleic acid encoding a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence.

The maize plant according to statement [47], wherein said QTL allele as defined in any of statements [01] to [14], one or more marker allele selected from M1 to M56, one or more polynucleic acid having a coding sequence selected from SEQ ID NOs: 1, 3, 5, 7, and 9, one or more polynucleic acid having a coding sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 1, 3, 5, 7, and 9, preferably over the entire length of the sequence, one or more polynucleic acid encoding a sequence selected from SEQ ID NOs: 2, 4, 6, 8, and 10 and/or one or more polynucleic acid encoding a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence, are heterozygous.

The maize plant according to statement [47], wherein said QTL allele as defined in any of statements [01] to [14], one or more marker allele selected from M1 to M56, one or more polynucleic acid having a coding sequence selected from SEQ ID NOs: 1, 3, 5, 7, and 9, one or more polynucleic acid having a coding sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 1, 3, 5, 7, and 9, preferably over the entire length of the sequence, one or more polynucleic acid encoding a sequence selected from SEQ ID NOs: 2, 4, 6, 8, and 10 and/or one or more polynucleic acid encoding a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence, are homozygous.

The maize plant or plant part according to any of statements [46] to [49] which is not maize line H102.

The maize plant or plant part according to any of statements [46] to [50], which is transgenic, gene-edited, base edited or mutagenized.

An (isolated) polynucleic acid comprising a coding sequence selected from SEQ ID NOs: 1, 3, 5, 7, and 9 or which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 1, 3, 5, 7, and 9; or which encodes a polypeptide selected from SEQ ID NOs: 2, 4, 6, 8, and 10 or a polypeptide which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 2, 4, 6, 8, and 10.

An (isolated) polynucleic acid specifically hybridizing with the polynucleic acid of statement [52], the complement thereof, or the reverse complement thereof.

The (isolated) polynucleic acid according to statement [53], wherein said polynucleic acid is a primer or a probe, preferably for the use as molecular marker.

A maize plant or plant part, preferably a maize plant or plant part having increased pathogen resistance and/or tolerance, preferably wherein said pathogen is *Exserohilum turcicum* comprising the polynucleic acid according to any of statements [52] or [53].

The maize plant according to statement [55], recombinantly expressing one or more protein encoded by one or more polynucleic acid having a coding sequence selected from SEQ ID NOs: 1, 3, 5, 7, and 9, one or more polynucleic acid having a coding sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 1, 3, 5, 7, and 9, preferably over the entire length of the sequence, one or more polynucleic acid encoding a sequence selected from SEQ ID NOs: 2, 4, 6, 8, and 10 and/or one or more polynucleic acid encoding a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence; or recombinantly expressing one or more protein having a sequence selected form SEQ ID NO: 2, 4, 6, 8, and 10 or a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence.

Method for controlling pathogen infestation in a maize plant (population) comprising
a) Providing (a) maize plant(s) according to any of statements [46] to [51] or [55] to [56] or growing from seeds (a) maize plant(s) according to any of statements [46] to [51] or [55] to [56],
b) Cultivating the plant(s) of a) under conditions of pathogen infestation.

The method according to statement [57], wherein pathogen infestation is reduced.

The method according to statement [57] or [58], wherein pathogen symptoms are reduced and/or the yield, in particular the biomass yield, dry matter yield, kernel yield or seed yield, is increased.

The method according to any of statements [57] to [59], wherein said conditions of pathogen infestation comprise the presence of a pathogen.

The method according to any of statements [57] to [60], wherein said pathogen is a fungus.

The method according to any of statements [57] to [61], wherein said pathogen is an *Exserohilum sp.*

The method according to any of statements [57] to [62], wherein said pathogen is *Exserohilum turcicum.*

Use of a polynucleic acid according to any of statements [52] to [54] for generating a maize plant or plant part.

Use of a polynucleic acid according to statement [64] for generating a maize plant or plant part having increased pathogen resistance and/or tolerance or for increasing pathogen resistance and/or tolerance in a maize plant, preferably according to any of the above methods for generating a maize plant or a plant part.

Use of a polynucleic acid according to any of statements [52] to [54] for identifying a maize plant or plant part.

Use according to statement [66] for identifying a maize plant or a plant part having increased pathogen resistance and/or tolerance, preferably according to any of the above methods for identifying a maize plant or a plant part.

Use of a maize plant according to any of statements [46] to [51] or [55] to [56] or part thereof for controlling pathogen infestation in a maize plant (population).

Use of a maize plant according to any of statements [46] to [51] or [55] to [56] or part thereof for increasing the yield (potential) of a plant, preferably under conditions of pathogen infestation.

Use according to statement [69], wherein the yield is biomass, dry matter yield, kernel yield or seed yield.

Use according to statement [70], wherein said biomass is whole plant biomass or biomass of a plant part.

Use according to statement [71], wherein said plant part is a tissue, organ, fruit, or seed.

### BRIEF DESCRIPTION OF THE FIGURES

**Figure 1****:** Sequence alignment of cDNAs of Zm00001d049121 derived from genotype B73 (SEQ ID NO: 11) and donor H102 (SEQ ID NO: 1).

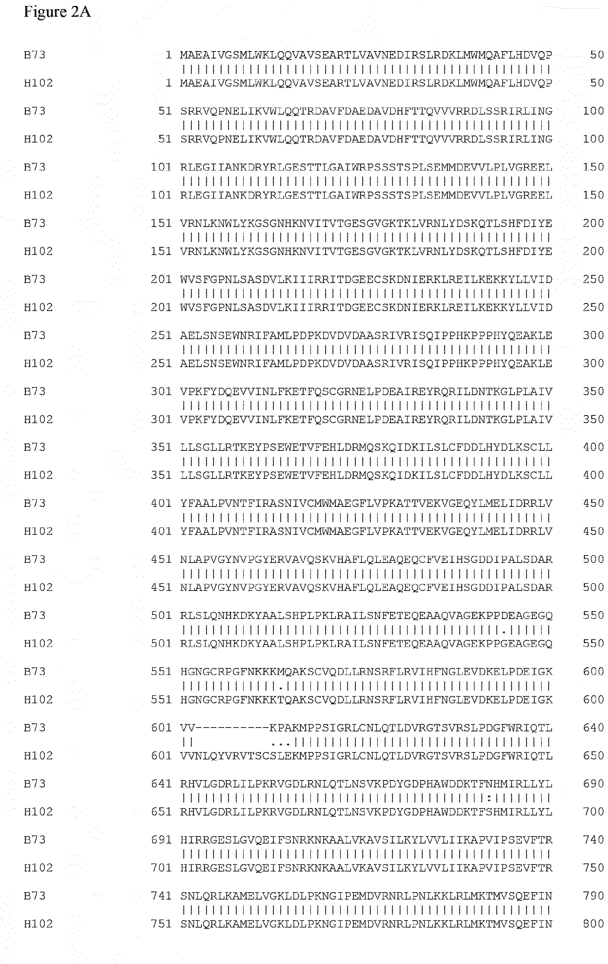

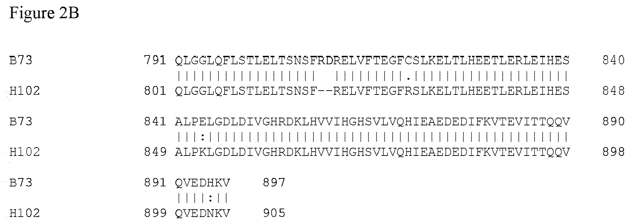

**Figure 2****:** Sequence alignment of amino acid sequences of Zm00001d049121 derived from genotype B73 (SEQ ID NO: 12) and donor H102 (SEQ ID NO: 2).
**Figure 3****:** Sequence alignment of cDNAs of Zm00001d049113 derived from genotype B73 (SEQ ID NO: 13) and donor H102 (SEQ ID NO: 3).

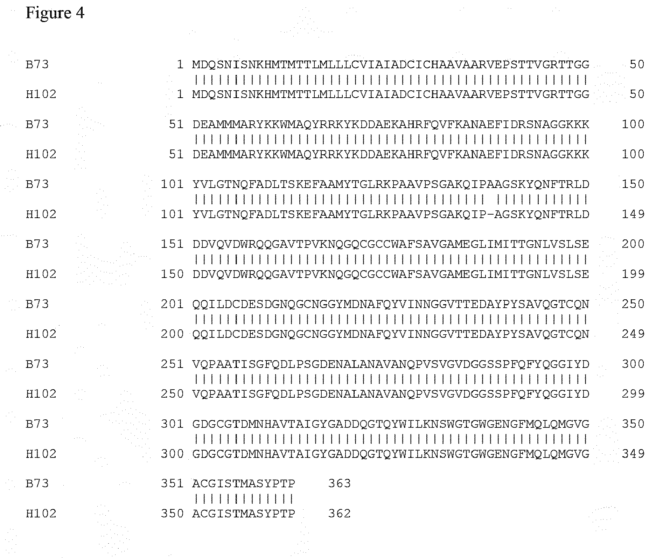

**Figure 4****:** Sequence alignment of amino acid sequences of Zm00001d049113 derived from genotype B73 (SEQ ID NO: 14) and donor H102 (SEQ ID NO: 4).
**Figure 5****:** Sequence alignment of cDNAs of Zm00001d049145 derived from genotype B73 (SEQ ID NO: 15) and donor H102 (SEQ ID NO: 5).

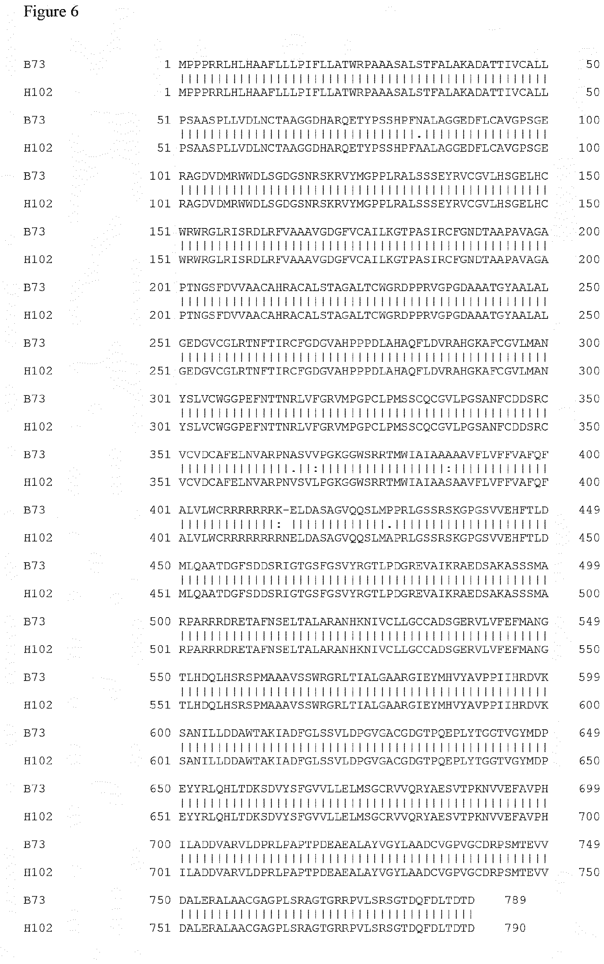

**Figure 6****:** Sequence alignment of amino acid sequences of Zm00001d049145 derived from genotype B73 (SEQ ID NO: 16) and donor H102 (SEQ ID NO: 6).
**Figure 7****:** Sequence alignment of cDNAs of Zm00001d049148 derived from genotype B73 (SEQ ID NO: 17) and donor H102 (SEQ ID NO: 7).

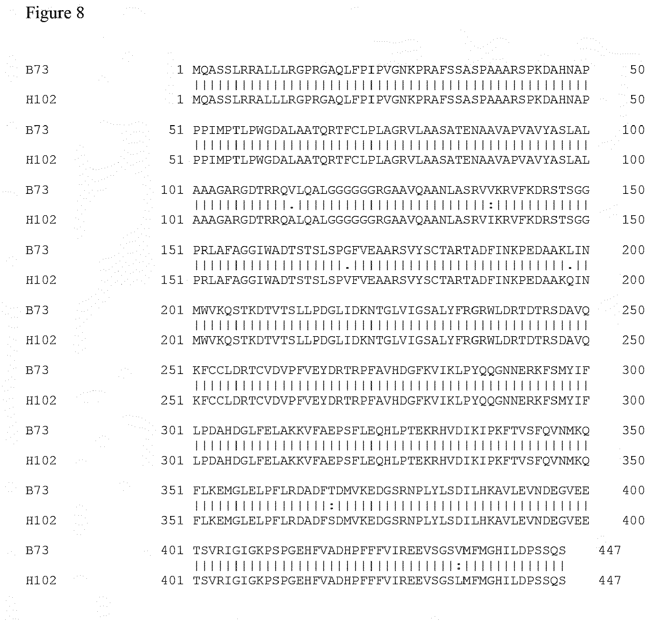

**Figure 8****:** Sequence alignment of amino acid sequences of Zm00001d049148 derived from genotype B73 ((SEQ ID NO: 18) and donor H102 (SEQ ID NO: 8).
**Figure 9****:** Sequence alignment of cDNAs of Zm00001d049159 derived from genotype B73 (SEQ ID NO: 19) and donor H102 (SEQ ID NO: 9).

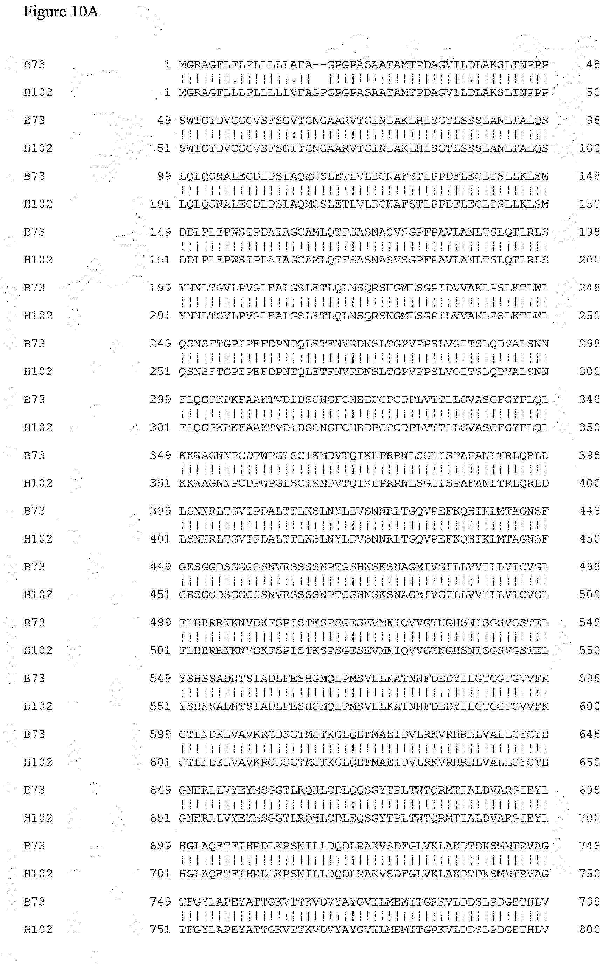

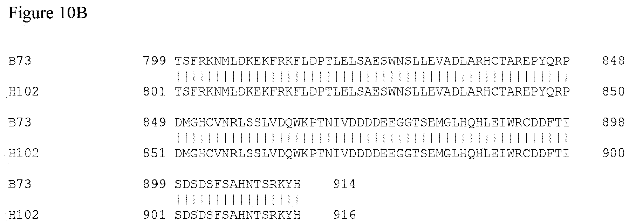

**Figure 10****:** Sequence alignment of amino acid sequences of Zm00001d049159 derived from genotype B73 (SEQ ID NO: 20) and donor H102 (SEQ ID NO: 10).

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilized and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous.

As used herein, "maize" refers to a plant of the species Zea *mays,* preferably Zea *mays ssp mays.*

The term "plant" includes whole plants, including descendants or progeny thereof. As used herein unless clearly indicated otherwise, the term "plant" intends to mean a plant at any developmental stage. The term "plant part" includes any part or derivative of the plant, including particular plant tissues or structures, plant cells, plant protoplast, plant cell or tissue culture from which plants can be regenerated, plant calli, plant clumps and plant cells that are intact in plants or parts of plants, such as seeds, kernels, cobs, flowers, cotyledons, leaves, stems, buds, roots, root tips, stover, and the like. Plant parts may include processed plant parts or derivatives, including flower, oils, extracts etc. "Parts of a plant" are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, pollen, trichomes and the like; and progeny of the same. Parts of plants may be attached to or separate from a whole intact plant. Such parts of a plant include, but are not limited to, organs, tissues, and cells of a plant, and preferably seeds. A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, seeds, cuttings, cell or tissue cultures, or any other part or product of a plant. This also includes callus or callus tissue as well as extracts (such as extracts from taproots) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

In certain embodiments, the plant part or derivative is not (functional) propagation material, such as germplasm, a seed, or plant embryo or other material from which a plant can be regenerated. In certain embodiments, the plant part or derivative does not comprise (functional) male and female reproductive organs. In certain embodiments, the plant part or derivative is or comprises propagation material, but propagation material which does not or cannot be used (anymore) to produce or generate new plants, such as propagation material which have been chemically, mechanically or otherwise rendered non-functional, for instance by heat treatment, acid treatment, compaction, crushing, chopping, etc.

As used herein, the term plant population may be used interchangeably with population of plants. A plant population preferably comprises a multitude of individual plants, such as preferably at least 10, such as 20, 30, 40, 50, 60, 70, 80, or 90, more preferably at least 100, such as 200, 300, 400, 500, 600, 700, 800, or 900, even more preferably at least 1000, such as at least 10000 or at least 100000.

The term "locus" (loci plural) means a specific place or places or a site on a chromosome where for example a QTL, a gene or genetic marker is found. As used herein, the term "quantitative trait locus" or "QTL" has its ordinary meaning known in the art. By means of further guidance, and without limitation, a QTL may refer to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window. A QTL may encode for one or more alleles that affect the expressivity of a continuously distributed (quantitative) phenotype. In certain embodiments, the QTL as described herein may be homozygous. In certain embodiments, the QTL as described herein may be heterozygous.

As used herein, the term "allele" or "alleles" refers to one or more alternative forms, i.e. different nucleotides or nucleotide sequences, of a locus.

As used herein, the term "mutant alleles" or "mutation" of alleles include alleles having one or more mutations, such as insertions, deletions, stop codons, base changes (e.g. , transitions or transversions), or alterations in splice junctions, which may or may not give rise to altered gene products. Modifications in alleles may arise in coding or noncoding regions (e.g. promoter regions, exons, introns or splice junctions).

As used herein, the terms "introgression", "introgressed" and "introgressing" refer to both a natural and artificial process whereby chromosomal fragments or genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, at a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times. "Introgression fragment" or "introgression segment" or "introgression region" refers to a chromosome fragment (or chromosome part or region) which has been introduced into another plant of the same or related species either artificially or naturally such as by crossing or traditional breeding techniques, such as backcrossing, i.e. the introgressed fragment is the result of breeding methods referred to by the verb "to introgress" (such as backcrossing). It is understood that the term "introgression fragment" never includes a whole chromosome, but only a part of a chromosome. The introgression fragment can be large, e.g. even three quarter or half of a chromosome, but is preferably smaller, such as about 15 Mb or less, such as about 10 Mb or less, about 9 Mb or less, about 8 Mb or less, about 7 Mb or less, about 6 Mb or less, about 5 Mb or less, about 4 Mb or less, about 3 Mb or less, about 2.5 Mb or 2 Mb or less, about 1 Mb (equals 1,000,000 base pairs) or less, or about 0.5 Mb (equals 500,000 base pairs) or less, such as about 200,000 bp (equals 200 kilo base pairs) or less, about 100,000 bp (100 kb) or less, about 50,000 bp (50 kb) or less, about 25,000 bp (25 kb) or less.

A genetic element, an introgression fragment, a QTL or a gene or allele conferring a trait (such as increased pathogen resistance or tolerance) is said to be "obtainable from" or can be "obtained from" or "derivable from" or can be "derived from" or "as present in" or "as found in" a plant or plant part as described herein elsewhere if it can be transferred from the plant in which it is present into another plant in which it is not present (such as a line or variety) using traditional breeding techniques without resulting in a phenotypic change of the recipient plant apart from the addition of the trait conferred by the genetic element, locus, introgression fragment, gene or allele. The terms are used interchangeably and the genetic element, locus, introgression fragment, gene or allele can thus be transferred into any other genetic background lacking the trait. Not only pants comprising the genetic element, locus, introgression fragment, gene or allele can be used, but also progeny/descendants from such plants which have been selected to retain the genetic element, locus, introgression fragment, gene or allele, can be used and are encompassed herein. Whether a plant (or genomic DNA, cell or tissue of a plant) comprises the same genetic element, locus, introgression fragment, gene or allele as obtainable from such plant can be determined by the skilled person using one or more techniques known in the art, such as phenotypic assays, whole genome sequencing, molecular marker analysis, trait mapping, chromosome painting, allelism tests and the like, or combinations of techniques. It will be understood that transgenic plants may also be encompassed.

As used herein the terms "genetic engineering", "transformation" and "genetic modification" are all used herein as synonyms for the transfer of isolated and cloned genes into the DNA, usually the chromosomal DNA or genome, of another organism.

"Transgenic" or "genetically modified organisms" (GMOs) as used herein are organisms whose genetic material has been altered using techniques generally known as "recombinant DNA technology". Recombinant DNA technology encompasses the ability to combine DNA molecules from different sources into one molecule ex vivo (e.g. in a test tube). The term "transgenic" here means genetically modified by the introduction of a non-endogenous nucleic acid sequence. Typically a species-specific nucleic acid sequence is introduced in a form, arrangement or quantity into the cell in a location where the nucleic acid sequence does not occur naturally in the cell. This terminology generally does not cover organisms whose genetic composition has been altered by conventional cross-breeding or by "mutagenesis" breeding, as these methods predate the discovery of recombinant DNA techniques. "Non-transgenic" as used herein refers to plants and food products derived from plants that are not "transgenic" or "genetically modified organisms" as defined above.

"Transgene" or "chimeric gene" refers to a genetic locus comprising a DNA sequence, such as a recombinant gene, which has been introduced into the genome of a plant by transformation, such as Agrobacterium mediated transformation. A plant comprising a transgene stably integrated into its genome is referred to as "transgenic plant".

"Gene editing" or "genome editing" refers to genetic engineering in which in which DNA or RNA is inserted, deleted, modified or replaced in the genome of a living organism. Gene editing may comprise targeted or non-targeted (random) mutagenesis. Targeted mutagenesis may be accomplished for instance with designer nucleases, such as for instance with meganucleases, zinc finger nucleases (ZFNs), transcription activator-like effector-based nucleases (TALEN), and the clustered regularly interspaced short palindromic repeats (CRISPR/Cas) system. These nucleases create site-specific double-strand breaks (DSBs) at desired locations in the genome. The induced double-strand breaks are repaired through nonhomologous end-joining (NHEJ) or homologous recombination (HR), resulting in targeted mutations or nucleic acid modifications. The use of designer nucleases is particularly suitable for generating gene knockouts or knockdowns. In certain embodiments, designer nucleases are developed which specifically introduce one or more of the molecular marker (marker allele) according to the invention as described herein. Delivery and expression systems of designer nuclease systems are well known in the art.

In certain embodiments, the nuclease or targeted/site-specific/homing nuclease is, comprises, consists essentially of, or consists of a (modified) CRISPR/Cas system or complex, a (modified) Cas protein, a (modified) zinc finger, a (modified) zinc finger nuclease (ZFN), a (modified) transcription factor-like effector (TALE), a (modified) transcription factor-like effector nuclease (TALEN), or a (modified) meganuclease. In certain embodiments, said (modified) nuclease or targeted/site-specific/homing nuclease is, comprises, consists essentially of, or consists of a (modified) RNA-guided nuclease. It will be understood that in certain embodiments, the nucleases may be codon optimized for expression in plants. As used herein, the term "targeting" of a selected nucleic acid sequence means that a nuclease or nuclease complex is acting in a nucleotide sequence specific manner. For instance, in the context of the CRISPR/Cas system, the guide RNA is capable of hybridizing with a selected nucleic acid sequence. As uses herein, "hybridization" or "hybridizing" refers to a reaction in which one or more polynucleotides react to form a complex that is stabilized via hydrogen bonding between the bases of the nucleotide residues, i.e. a process in which a single-stranded nucleic acid molecule attaches itself to a complementary nucleic acid strand, i.e. agrees with this base pairing. Standard procedures for hybridization are described, for example, in Sambrook et al. (Molecular Cloning. A Laboratory Manual, Cold Spring Harbor Laboratory Press, 3rd edition 2001). The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PGR, or the cleavage of a polynucleotide by an enzyme. A sequence capable of hybridizing with a given sequence is referred to as the "complement" of the given sequence. Preferably this will be understood to mean an at least 50%, more preferably at least 55%, 60%, 65%, 70%, 75%, 80% or 85%, more preferably 90%, 91%, 92%, 93%, 94%, 95%, 96%, 97%, 98% or 99% of the bases of the nucleic acid strand form base pairs with the complementary nucleic acid strand. The possibility of such binding depends on the stringency of the hybridization conditions.

Gene editing may involve transient, inducible, or constitutive expression of the gene editing components or systems. Gene editing may involve genomic integration or episomal presence of the gene editing components or systems. Gene editing components or systems may be provided on vectors, such as plasmids, which may be delivered by appropriate delivery vehicles, as is known in the art. Preferred vectors are expression vectors.

Gene editing may comprise the provision of recombination templates, to effect homology directed repair (HDR). For instance a genetic element may be replaced by gene editing in which a recombination template is provided. The DNA may be cut upstream and downstream of a sequence which needs to be replaced. As such, the sequence to be replaced is excised from the DNA. Through HDR, the excised sequence is then replaced by the template. In certain embodiments, the QTL allele of the invention as described herein may be provided on/as a template. By designing the system such that double strand breaks are introduced upstream and downstream of the corresponding region in the genome of a plant not comprising the QTL allele, this region is excised and can be replaced with the template comprising the QTL allele of the invention. In this way, introduction of the QTL allele of the invention in a plant need not involve multiple backcrossing, in particular in a plant of specific genetic background. Similarly, the polynucleic acid of the invention may be provided on/as a template. More advantageously however, the polynucleic acid of the invention may be generated without the use of a recombination template, but solely through the endonuclease action leading to a double strand DNA break which is repaired by NHEJ, resulting in the generation of indels. Further, gene editing my comprise also the exchange of single nucleotides by means of base eitors. A base editor as used herein refers to a protein or a fragment thereof having the capacity to mediate a targeted base modification, i.e., the conversion of a base of interest resulting in a point mutation of interest. Preferably, the at least one base editor in the context of the present invention is temporarily or permanently fused to at least one DSBI enzyme, or optionally to a component of at least one DSBI. The fusion can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al ((2017). Programmable base editing of A•T to G•C in genomic DNA without DNA cleavage. Nature, 551(7681), 464.) described adenine base editors (ABEs) that mediate the conversion of A•T to G•C in genomic DNA.

In certain embodiments, the nucleic acid modification is effected by random mutagenesis. Cells or organisms may be exposed to mutagens such as UV radiation or mutagenic chemicals (such as for instance such as ethyl methanesulfonate (EMS)), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nucleases. The products are then separated by size, such as by HPLC. See also McCallum et al. "Targeted screening for induced mutations"; Nat Biotechnol. 2000 Apr;18(4):455-7 and McCallum et al. "Targeting induced local lesions IN genomes (TILLING) for plant functional genomics"; Plant Physiol. 2000 Jun;123(2):439-42.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. As used herein, the term "heterozygote" refers to an individual cell or plant having different alleles at one or more or all loci. When the term is used with reference to a specific locus or gene, it means at least that locus or gene has different alleles. As used herein, the term "heterozygous" means a genetic condition existing when different alleles reside at corresponding loci on homologous chromosomes. In certain embodiments, the QTL and/or one or more marker(s) as described herein is/are homozygous. In certain embodiments, the QTL and/or one or more marker(s) as described herein are heterozygous. In certain embodiments, the QTL allele and/or one or more marker(s) allele(s) as described herein is/are homozygous. In certain embodiments, the QTL allele and/or one or more marker(s) allele(s) as described herein are heterozygous.

A "marker" is a (means of finding a position on a) genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" or "marker allele" may also be used to denote a nucleic acid or amino acid sequence that is sufficiently unique to characterize a specific locus on the genome.

Any detectable polymorphic trait can be used as a marker so long as it is inherited differentially and exhibits linkage disequilibrium with a phenotypic trait of interest.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g. via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

A "marker allele", alternatively an "allele of a marker locus", can refer to one of a plurality of polymorphic nucleotide sequences found at a marker locus in a population. With regard to a SNP marker, allele refers to the specific nucleotide base present at that SNP locus in that individual plant.

"Fine-mapping" refers to methods by which the position of a QTL can be determined more accurately (narrowed down) and by which the size of the introgression fragment comprising the QTL is reduced. For example Near Isogenic Lines for the QTL (QTL-NILs) can be made, which contain different, overlapping fragments of the introgression fragment within an otherwise uniform genetic background of the recurrent parent. Such lines can then be used to map on which fragment the QTL is located and to identify a line having a shorter introgression fragment comprising the QTL.

"Marker assisted selection" (of MAS) is a process by which individual plants are selected based on marker genotypes. "Marker assisted counter-selection" is a process by which marker genotypes are used to identify plants that will not be selected, allowing them to be removed from a breeding program or planting. Marker assisted selection uses the presence of molecular markers, which are genetically linked to a particular locus or to a particular chromosome region (e.g. introgression fragment, transgene, polymorphism, mutation, etc), to select plants for the presence of the specific locus or region (introgression fragment, transgene, polymorphism, mutation, etc). For example, a marker allele genetically linked to a QTL as defined herein, can be used to detect and/or select plants comprising the QTL on chromosome 4. The closer the genetic linkage of the marker allele to the locus (e.g. about 7 cM, 6 cM, 5 cM, 4 cM, 3 cM, 2 cM, 1 cM, 0.5 cM or less), the less likely it is that the marker is dissociated from the locus through meiotic recombination. Likewise, the closer two markers are linked to each other (e.g. within 7 or 5 cM, 4 cM , 3 cM, 2 cM, 1 cM or less) the less likely it is that the two markers will be separated from one another (and the more likely they will co-segregate as a unit). A marker "within 7 cM or within 5 cM, 3 cM, 2 cM, or 1 cM" of another marker refers to a marker which genetically maps to within the 7 cM or 5 cM, 3 cM, 2 cM, or 1 cM region flanking the marker (i.e. either side of the marker). Similarly, a marker within 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10kb, 5kb, 2kb, 1 kb or less of another marker refers to a marker which is physically located within the 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10 kb, 5 kb, 2 kb, 1 kb or less, of the genomic DNA region flanking the marker (i.e. either side of the marker). "LOD-score" (logarithm (base 10) of odds) refers to a statistical test often used for linkage analysis in animal and plant populations. The LOD score compares the likelihood of obtaining the test data if the two loci (molecular marker loci and/or a phenotypic trait locus) are indeed linked, to the likelihood of observing the same data purely by chance. Positive LOD scores favour the presence of linkage and a LOD score greater than 3.0 is considered evidence for linkage. A LOD score of +3 indicates 1000 to 1 odds that the linkage being observed did not occur by chance.

A "marker haplotype" refers to a combination of alleles at a marker locus.

A "marker locus" is a specific chromosome location in the genome of a species where a specific marker can be found. A marker locus can be used to track the presence of a second linked locus, e.g., one that affects the expression of a phenotypic trait. For example, a marker locus can be used to monitor segregation of alleles at a genetically or physically linked locus.

A "marker probe" is a nucleic acid sequence or molecule that can be used to identify the presence of a marker locus, e.g., a nucleic acid probe that is complementary to a marker locus sequence, through nucleic acid hybridization. Marker probes comprising 30 or more contiguous nucleotides of the marker locus ("all or a portion" of the marker locus sequence) may be used for nucleic acid hybridization. Alternatively, in some aspects, a marker probe refers to a probe of any type that is able to distinguish (i.e., genotype) the particular allele that is present at a marker locus.

The term "molecular marker" may be used to refer to a genetic marker or an encoded product thereof (e.g., a protein) used as a point of reference when identifying a linked locus. A marker can be derived from genomic nucleotide sequences or from expressed nucleotide sequences (e.g., from a spliced RNA, a cDNA, etc.), or from an encoded polypeptide. The term also refers to nucleic acid sequences complementary to or flanking the marker sequences, such as nucleic acids used as probes or primer pairs capable of amplifying the marker sequence. A "molecular marker probe" is a nucleic acid sequence or molecule that can be used to identify the presence of a marker locus, e.g., a nucleic acid probe that is complementary to a marker locus sequence. Alternatively, in some aspects, a marker probe refers to a probe of any type that is able to distinguish (i.e., genotype) the particular allele that is present at a marker locus. Nucleic acids are "complementary" when they specifically hybridize in solution, e.g., according to Watson-Crick base pairing rules. Some of the markers described herein are also referred to as hybridization markers when located on an indel region, such as the non- collinear region described herein. This is because the insertion region is, by definition, a polymorphism vis a vis a plant without the insertion. Thus, the marker need only indicate whether the indel region is present or absent. Any suitable marker detection technology may be used to identify such a hybridization marker, e.g. SNP technology is used in the examples provided herein.

"Genetic markers" are nucleic acids that are polymorphic in a population and where the alleles of which can be detected and distinguished by one or more analytic methods, e.g., RFLP, AFLP, isozyme, SNP, SSR, and the like. The terms "molecular marker" and "genetic marker" are used interchangeably herein. The term also refers to nucleic acid sequences complementary to the genomic sequences, such as nucleic acids used as probes. Markers corresponding to genetic polymorphisms between members of a population can be detected by methods well- established in the art. These include, e.g., PCR-based sequence specific amplification methods, detection of restriction fragment length polymorphisms (RFLP), detection of isozyme markers, detection of polynucleotide polymorphisms by allele specific hybridization (ASH), detection of amplified variable sequences of the plant genome, detection of self-sustained sequence replication, detection of simple sequence repeats (SSRs), detection of single nucleotide polymorphisms (SNPs), or detection of amplified fragment length polymorphisms (AFLPs). Well established methods are also know for the detection of expressed sequence tags (ESTs) and SSR markers derived from EST sequences and randomly amplified polymorphic DNA (RAPD).

A "polymorphism" is a variation in the DNA between two or more individuals within a population. A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

"Physical distance" between loci (e.g. between molecular markers and/or between phenotypic markers) on the same chromosome is the actually physical distance expressed in bases or base pairs (bp), kilo bases or kilo base pairs (kb) or megabases or mega base pairs (Mb).

"Genetic distance" between loci (e.g. between molecular markers and/or between phenotypic markers) on the same chromosome is measured by frequency of crossing-over, or recombination frequency (RF) and is indicated in centimorgans (cM). One cM corresponds to a recombination frequency of 1%. If no recombinants can be found, the RF is zero and the loci are either extremely close together physically or they are identical. The further apart two loci are, the higher the RF.

A "physical map" of the genome is a map showing the linear order of identifiable landmarks (including genes, markers, etc.) on chromosome DNA. However, in contrast to genetic maps, the distances between landmarks are absolute (for example, measured in base pairs or isolated and overlapping contiguous genetic fragments) and not based on genetic recombination (that can vary in different populations).

An allele "negatively" correlates with a trait when it is linked to it and when presence of the allele is an indicator that a desired trait or trait form will not occur in a plant comprising the allele. An allele "positively" correlates with a trait when it is linked to it and when presence of the allele is an indicator that the desired trait or trait form will occur in a plant comprising the allele.

A centimorgan ("cM") is a unit of measure of recombination frequency. One cM is equal to a 1 % chance that a marker at one genetic locus will be separated from a marker at a second locus due to crossing over in a single generation.

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%.

The term "closely linked", in the present application, means that recombination between two linked loci occurs with a frequency of equal to or less than about 10% (i.e., are separated on a genetic map by not more than 10 cM). Put another way, the closely linked loci co-segregate at least 90% of the time. Marker loci are especially useful with respect to the subject matter of the current disclosure when they demonstrate a significant probability of co-segregation (linkage) with a desired trait (e.g., resistance to gray leaf spot). Closely linked loci such as a marker locus and a second locus can display an inter-locus recombination frequency of 10% or less, preferably about 9% or less, still more preferably about 8% or less, yet more preferably about 7% or less, still more preferably about 6% or less, yet more preferably about 5% or less, still more preferably about 4% or less, yet more preferably about 3% or less, and still more preferably about 2% or less. In highly preferred embodiments, the relevant loci display a recombination a frequency of about 1 % or less, e.g., about 0.75% or less, more preferably about 0.5% or less, or yet more preferably about 0.25% or less. Two loci that are localized to the same chromosome, and at such a distance that recombination between the two loci occurs at a frequency of less than 10% (e.g., about 9 %, 8%, 7%, 6%, 5%, 4%, 3%, 2%, 1 %, 0.75%, 0.5%, 0.25%, or less) are also said to be "proximal to" each other. In some cases, two different markers can have the same genetic map coordinates. In that case, the two markers are in such close proximity to each other that recombination occurs between them with such low frequency that it is undetectable.

"Linkage" refers to the tendency for alleles to segregate together more often than expected by chance if their transmission was independent. Typically, linkage refers to alleles on the same chromosome. Genetic recombination occurs with an assumed random frequency over the entire genome. Genetic maps are constructed by measuring the frequency of recombination between pairs of traits or markers. The closer the traits or markers are to each other on the chromosome, the lower the frequency of recombination, and the greater the degree of linkage. Traits or markers are considered herein to be linked if they generally co- segregate. A 1/100 probability of recombination per generation is defined as a genetic map distance of 1.0 centiMorgan (1.0 cM). The term "linkage disequilibrium" refers to a non-random segregation of genetic loci or traits (or both). In either case, linkage disequilibrium implies that the relevant loci are within sufficient physical proximity along a length of a chromosome so that they segregate together with greater than random (i.e., non-random) frequency. Markers that show linkage disequilibrium are considered linked. Linked loci co-segregate more than 50% of the time, e.g., from about 51 % to about 100% of the time. In other words, two markers that co-segregate have a recombination frequency of less than 50% (and by definition, are separated by less than 50 cM on the same linkage group.) As used herein, linkage can be between two markers, or alternatively between a marker and a locus affecting a phenotype. A marker locus can be "associated with" (linked to) a trait. The degree of linkage of a marker locus and a locus affecting a phenotypic trait is measured, e.g., as a statistical probability of co-segregation of that molecular marker with the phenotype (e.g., an F statistic or LOD score).

The genetic elements or genes located on a single chromosome segment are physically linked. In some embodiments, the two loci are located in close proximity such that recombination between homologous chromosome pairs does not occur between the two loci during meiosis with high frequency, e.g., such that linked loci co-segregate at least about 90% of the time, e.g., 91%, 92%, 93%, 94%, 95%, 96%, 97%, 98%, 99%, 99.5%, 99.75%, or more of the time. The genetic elements located within a chromosomal segment are also "genetically linked", typically within a genetic recombination distance of less than or equal to 50cM, e.g., about 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0.75, 0.5, 0.25 cM or less. That is, two genetic elements within a single chromosomal segment undergo recombination during meiosis with each other at a frequency of less than or equal to about 50%, e.g., about 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.75%, 0.5%, 0.25% or less. "Closely linked" markers display a cross over frequency with a given marker of about 10% or less, e.g., 9%, 8%, 7%, 6%, 5%, 4%, 3%, 2%, 1%, 0.75%, 0.5%, 0.25% or less (the given marker locus is within about 10 cM of a closely linked marker locus, e.g., 9, 8, 7, 6, 5, 4, 3, 2, 1, 0.75, 0.5, 0.25 cM or less of a closely linked marker locus). Put another way, closely linked marker loci co- segregate at least about 90% the time, e.g., 91%, 92%, 93%, 94%, 95%, 96%, 97%, 98%, 99%, 99.5%, 99.75%, or more of the time.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (Bl2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the Bl2seq program can be found in the readme file accompanying BLASTZ. BI2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seql .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the Bl2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

An "isolated nucleic acid sequence" or "isolated DNA" refers to a nucleic acid sequence which is no longer in the natural environment from which it was isolated, e.g. the nucleic acid sequence in a bacterial host cell or in the plant nuclear or plastid genome. When referring to a "sequence" herein, it is understood that the molecule having such a sequence is referred to, e.g. the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

When reference is made to a nucleic acid sequence (e.g. DNA or genomic DNA) having "substantial sequence identity to" a reference sequence or having a sequence identity of at least 80%>, e.g. at least 85%, 90%, 95%, 98%> or 99%> nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically, stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative<1>, in which an amino acid residue contained in the wild-type protein is replaced with another naturally-occurring amino acid of similar character, for example Gly<→Ala, Val<→Ile<→Leu, Asp<→Glu, Lys<→Arg, Asn<→ Gln or Phe<→Trp<→Tyr. Substitutions encompassed by the present invention may also be "non-conservative", in which an amino acid residue which is present in the wild-type protein is substituted with an amino acid with different properties, such as a naturally-occurring amino acid from a different group (e.g. substituting a charged or hydrophobic amino acid with alanine. "Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

As used herein, the term "endogenous" refers to a gene or allele which is present in its natural genomic location. The term "endogenous" can be used interchangeably with "native" or "wild-type". This does not however exclude the presence of one or more nucleic acid differences with the wild-type allele. In particular embodiments, the difference with a wild-type allele can be limited to less than 9 preferably less than 6, more particularly less than 3 nucleotide differences, such as 0 nucleotides difference. More particularly, the difference with the wildtype sequence can be in only one nucleotide. Preferably, the endogenous allele encodes a modified protein having less than 9, preferably less than 6, more particularly less than 3 and even more preferably only one or no amino acid difference with the wild-type protein.

A used herein, the term "exogenous polynucleotide" refers to a polynucleotide, such as a gene (or cDNA) or allele which is or has been recombinantly introduced in a cell (or plant). The exogenous polynucleotide may be episomal or genomically integrated. Integration may be random or site-directed. Integration may include replacement of a corresponding endogenous polynucleotide. It will be understood that an exogenous polynucleotide is not naturally present in the cell or plant.

As used herein, the B73 reference genome AGPv4 refers to the assembly B73 RefGen_v4 (also known as AGPv4, B73 RefGen_v4) as provided on the Maize Genetics and Genomics Database (https://www.maizegdb.org/genome/genome_assembly/Zm-B73-REFERENCE-GRAMENE-4.0).

In an aspect, the invention relates to a method for identifying a maize plant or plant part, preferably a maize plant or plant part having increased pathogen resistance and/or tolerance, preferably wherein said pathogen is *Exserohilum turcicum,* comprising screening for the presence of a QTL allele located on chromosome 4 in a maize plant or plant part, wherein said QTL allele is located on a chromosomal interval flanked by marker alleles A and B, wherein marker alleles A and B are SNPs which are respectively adenine (A) at the position 16107190 bp on reference genome B73 AGPv04 and cytosine (C) at the position 18470371 bp on reference genome B73 AGPv04.

Marker allele A can be determined or detected by use of primer sequences as set forth in SEQ ID NOs: 24 to 26, and marker allele B by primer sequences as set forth in SEQ ID NOs: 27 to 29, preferably in a KASP marker system (Table 1A). Marker alleles may be genetically linked to the presence of the QTL. The QTL is associated with said increased pathogen resistance and/or tolerance.

Methods for screening for the presence of a QTL allele or (molecular) marker allele as described herein are known in the art. Without limitation, screening may encompass or comprise sequencing, hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc.

As used herein the terms "increased pathogen tolerance" and "increased pathogen resistance" relate to any relief from, reduced presentation of, improvement of, or any combination thereof of any symptom (such as damage or loss in biomass, in dry matter yield/content or in seed/kernel yield) of an infection by a pathogen. Increased pathogen resistance or tolerance as referred to herein may also relate to the ability to which a plant maintains for instance its biomass production (such as harvestable biomass production, such as seed yield) upon or during pathogen infection. A pathogen resistant or tolerant plant, plant cell or plant part may refer herein to a plant, plant cell or plant part, respectively, having increased resistance/tolerance to a pathogen compared to a parent plant from which they are derived (e.g. not comprising the QTL allele or one or more of the molecular marker (allele) or polynucleic acid according to the invention as described herein). Resistance may relate herein to the plant's ability to limit pathogen multiplication. Tolerance may relate herein to a plant's ability to reduce the effect of infection on its fitness regardless of the level of pathogen multiplication. Methods of determining pathogen resistance/tolerance are known to the person of skill in the art, such as visual scoring of pathogen infection or pathogen-induced damage, determination of biomass (yield), etc. As used herein, the terms "increased pathogen tolerance" and "increased pathogen resistance" may be used interchangeably with "reduced sensitivity" or "reduced susceptibility" towards pathogens. Accordingly, a plant, plant part, or plant population according to the invention which is more resistant or more tolerant towards a pathogen is considered less sensitive toward such pathogen. Less sensitive or less susceptible when used herein may be seen as "more tolerant" or "more resistant". Similarly, "more tolerant" or "more resistant" may, vice versa, be seen as "less sensitive" or "less susceptible". More sensitive or more susceptible when used herein may, vice versa, be seen as "less tolerant" or "less resistant". Similarly, "less tolerant" or "less resistant" may, vice versa, be seen as "more sensitive" or "more susceptible".

In an embodiment, the QTL allele is flanked by marker alleles A and/or B, preferably both.

In an embodiment, the QTL allele is located on a chromosomal interval comprising marker alleles A and C, wherein marker alleles A and C are SNPs which are respectively adenine (A) at the position 16107190 bp on reference genome B73 AGPv04 and thymine (T) at the position 22847078 bp on reference genome B73 AGPv04.

Marker allele A can be determined or detected by use of primer sequences as set forth in SEQ ID NOs: 24 to 26, and marker allele C by primer sequences as set forth in SEQ ID NOs: 30 to 32, preferably in a KASP marker system (Table 1A). Marker alleles may be genetically linked to the presence of the QTL.

In an embodiment, the QTL allele is flanked by molecular markers A and/or C, preferably both.

In an aspect, the invention relates to a method for identifying a maize plant or plant part, such as a maize plant or plant part having increased pathogen resistance and/or tolerance, comprising screening for the presence of a QTL allele located on chromosome 4 in a maize plant or plant part, wherein said QTL allele is located on a chromosomal interval comprising marker alleles A and C, wherein marker alleles A and C are SNPs which are respectively adenine (A) at the position 16107190 bp on reference genome B73 AGPv04 and thymine (T) at the position 22847078 bp on reference genome B73 AGPv04.

Marker allele A can be determined or detected by use of primer sequences as set forth in SEQ ID NOs: 24 to 26, and marker allele C by primer sequences as set forth in SEQ ID NOs: 30 to 32, preferably in a KASP marker system (Table 1A). Marker alleles may be genetically linked to the presence of the QTL.

In an embodiment, the QTL allele is flanked by molecular markers A and/or C, preferably both.

In an embodiment, the QTL allele is located on a chromosomal interval flanked by marker alleles A and B, wherein marker alleles A and B are SNPs which are respectively adenine (A) at the position 16107190 bp on reference genome B73 AGPv04 and cytosine (C) at the position 18470371 bp on reference genome B73 AGPv04.

Marker allele A can be determined or detected by use of primer sequences as set forth in SEQ ID NOs: 24 to 26, and marker allele B by primer sequences as set forth in SEQ ID NOs: 27 to 29, preferably in a KASP marker system (Table 1A). Marker alleles may be genetically linked to the presence of the QTL. The QTL is associated with said increased pathogen resistance and/or tolerance.

In an embodiment, the QTL allele is flanked by molecular markers A and/or B, preferably both.

As referred to herein, a polynucleic acid, such as for instance a QTL (allele) as described herein, is said to be flanked by certain molecular markers or molecular marker alleles if the polynucleic acid is comprised within a polynucleic acid wherein respectively a first marker (allele) is located upstream (i.e. 5') of said polynucleic acid and a second marker (allele) is located downstream (i.e. 3') of said polynucleic acid. Such first and second marker (allele) may border the polynucleic acid. The nucleic acid may equally comprise such first and second marker (allele), such as respectively at or near the 5' and 3' end, for instance respectively within 50 kb of the 5' and 3' end, preferably within 10 kb of the 5' and 3' end, such as within 5 kb of the 5' and 3' end, within 1 kb of the 5' and 3' end, or less.

In an embodiment, the QTL allele comprises one or more molecular markers (marker alleles) M1 to M56 selected from the molecular markers (marker alleles) listed in Table 1B, preferably referenced to the B73 reference genome AGPv4 It is to be understood that the indicated nucleotide positions are the nucleotide positions of the indicated B73 cDNAs and that the marker positions in the maize plants according to the invention correspond to the indicated marker positions, but are or comprise not necessarily identical positions. The skilled person will understand that corresponding nucleotide positions can be determined by suitable alignment, as is known in the art.

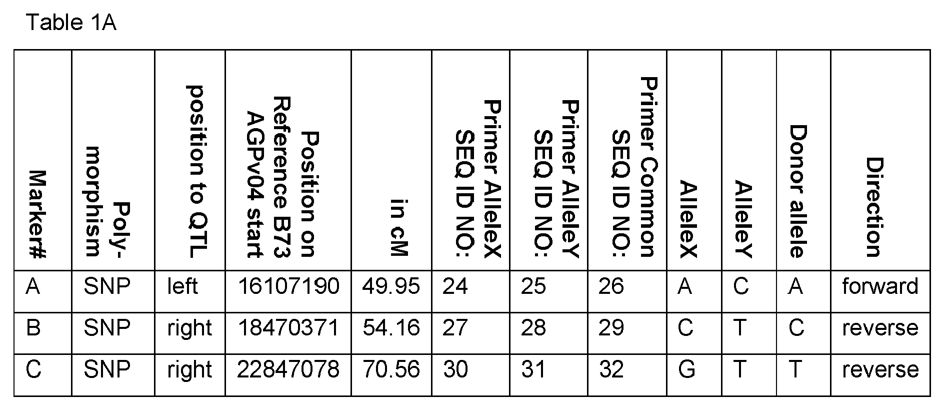

**Table 1B**

| **Marker #** | **Polymorphism** | **Molecular marker allele** | **Nucleotide position referenced to B73 allele of indicated gene cDNA** | |
|---|---|---|---|---|
| M1 | SNP | T | Zm00001d049121 | 1071 |
| M2 | SNP | G | Zm00001d049121 | 1302 |
| M3 | SNP | C | Zm00001d049121 | 1497 |
| M4 | SNP | G | Zm00001d049121 | 1631 |
| M5 | SNP | C | Zm00001d049121 | 1691 |
| M6 | InDel | - | Zm00001d049121 | 1807 |
| M7 | InDel | Insertion, preferably | Zm00001d049121 | between 1815 and 1816 |
| | | GTACGTCCG AGTGACCTC CTGCTCCTT GGAG | | |
| M8 | SNP | G | Zm00001d049121 | 2045 |
| M9 | InDel | - | Zm00001d049121 | 2424 - 2429 |
| M10 | SNP | C | Zm00001d049121 | 2458 |
| M11 | SNP | A | Zm00001d049121 | 2530 |
| M12 | SNP | A | Zm00001d049121 | 2683 |
| M13 | SNP | C | Zm00001d049113 | 12 |
| M14 | SNP | C | Zm00001d049113 | 291 |
| M15 | SNP | T | Zm00001d049113 | 309 |
| M16 | SNP | T | Zm00001d049113 | 324 |
| M17 | InDel | - | Zm00001d049113 | 415 - 417 |
| M18 | SNP | G | Zm00001d049145 | 195 |
| M19 | SNP | G | Zm00001d049145 | 247 |
| M20 | SNP | C | Zm00001d049145 | 248 |
| M21 | SNP | T | Zm00001d049145 | 1097 |
| M22 | SNP | C | Zm00001d049145 | 1105 |
| M23 | SNP | T | Zm00001d049145 | 1159 |
| M24 | SNP | T | Zm00001d049145 | 1185 |
| M25 | InDel | Insertion, preferably GTC | Zm00001d049145 | between 1216 and 1217 |
| M26 | SNP | C | Zm00001d049145 | 1242 |
| M27 | SNP | G | Zm00001d049145 | 1282 |
| M28 | SNP | G | Zm00001d049145 | 1344 |
| M29 | SNP | C | Zm00001d049145 | 1380 |
| M30 | SNP | C | Zm00001d049145 | 1422 |
| M31 | SNP | C | Zm00001d049148 | 338 |
| M32 | SNP | A | Zm00001d049148 | 412 |
| M33 | SNP | T | Zm00001d049148 | 432 |
| M34 | SNP | G | Zm00001d049148 | 447 |
| M35 | SNP | T | Zm00001d049148 | 507 |
| M36 | SNP | T | Zm00001d049148 | 509 |
| M37 | SNP | T | Zm00001d049148 | 585 |
| M38 | SNP | T | Zm00001d049148 | 588 |
| M39 | SNP | A | Zm00001d049148 | 593 |
| M40 | SNP | C | Zm00001d049148 | 672 |
| M41 | SNP | T | Zm00001d049148 | 741 |
| M42 | SNP | G | Zm00001d049148 | 753 |
| M43 | SNP | C | Zm00001d049148 | 846 |
| M44 | SNP | T | Zm00001d049148 | 996 |
| M45 | SNP | C | Zm00001d049148 | 1092 |
| M46 | SNP | T | Zm00001d049148 | 1102 |
| M47 | SNP | C | Zm00001d049148 | 1260 |
| M48 | SNP | T | Zm00001d049148 | 1300 |
| M49 | SNP | C | Zm00001d049159 | 22 |
| M50 | SNP | T | Zm00001d049159 | 47 |
| M51 | InDel | Insertion, preferably CCGGCC | Zm00001d049159 | between 52 and 53 |
| M52 | SNP | A | Zm00001d049159 | 189 |
| M53 | SNP | A | Zm00001d049159 | 190 |
| M54 | SNP | G | Zm00001d049159 | 1980 |
| M55 | SNP | G | Zm00001d049159 | 2014 |
| M56 | SNP | G | Zm00001d049159 | 2160 |

In an embodiment, the QTL allele comprises all of molecular markers (marker alleles) M1 to M56.

In an aspect, the invention relates to a method for identifying a maize plant or plant part, such as a maize plant or plant part having increased pathogen resistance and/or tolerance, comprising screening for the presence of one or more molecular marker (alleles) listed in Table 1B, preferably referenced to the B73 reference genome AGPv4.

In an embodiment M7 is an insertion of 31 ± 3, 6, 9, 12, 15, 18, 21, 24, 27, or 30 nucleotides. In an embodiment M7 is an insertion of a polynucleic acid sequence as set forth in SEQ ID NO: 33.

In an embodiment, screening for the presence of M6 and M7 is performed.

In an embodiment, M25 is an insertion of 3 nucleotides. In an embodiment, M25 is an insertion of a polynucleic acid sequence of GTC.

In an embodiment, M51 is an insertion of 6 ± 3 nucleotides. In an embodiment, M51 is an insertion of a polynucleic acid sequence of CCGGCC.

In certain embodiments in the methods, plants or plant parts, polynucleic acids, or uses as described herein, Zm00001d049121 comprises a coding sequence as set forth in SEQ ID NO: 11 referenced to the B73 reference genome AGPv4.

In certain embodiments in the methods, plants or plant parts, polynucleic acids, or uses as described herein, Zm00001d049113 comprises a coding sequence as set forth in SEQ ID NO: 13 referenced to the B73 reference genome AGPv4.

In certain embodiments in the methods, plants or plant parts, polynucleic acids, or uses as described herein, Zm00001d049145 comprises a coding sequence as set forth in SEQ ID NO: 15 referenced to the B73 reference genome AGPv4.

In certain embodiments in the methods, plants or plant parts, polynucleic acids, or uses as described herein, Zm00001d049148 comprises a coding sequence as set forth in SEQ ID NO: 17 referenced to the B73 reference genome AGPv4.

In certain embodiments in the methods, plants or plant parts, polynucleic acids, or uses as described herein, Zm00001d049159 comprises a coding sequence as set forth in SEQ ID NO: 19 referenced to the B73 reference genome AGPv4.

In certain embodiments in the methods, plants or plant parts, polynucleic acids, or uses as described herein, Zm00001d049121 encodes a protein sequence as set forth in SEQ ID NO: 12 referenced to the B73 reference genome AGPv4.

In certain embodiments in the methods, plants or plant parts, polynucleic acids, or uses as described herein, Zm00001d049113 encodes a protein sequence as set forth in SEQ ID NO: 14 referenced to the B73 reference genome AGPv4.

In certain embodiments in the methods, plants or plant parts, polynucleic acids, or uses as described herein, Zm00001d049145 encodes a protein sequence as set forth in SEQ ID NO: 16 referenced to the B73 reference genome AGPv4.

In certain embodiments in the methods, plants or plant parts, polynucleic acids, or uses as described herein, Zm00001d049148 encodes a protein sequence as set forth in SEQ ID NO: 18 referenced to the B73 reference genome AGPv4.

In certain embodiments in the methods, plants or plant parts, polynucleic acids, or uses as described herein, Zm00001d049159 encodes a protein sequence as set forth in SEQ ID NO: 20 referenced to the B73 reference genome AGPv4.

Zm00001d049121 is annotated as Nucleotide-binding site-leucine-rich repeat protein (NBS-LRR protein). Zm00001d049113 annotated as Papain like cysteine protease (C1 type). Zm00001d049145 is annotated as CRINKLY4-like protein kinase. Zm00001d049148 is annotated as Serine (or cysteine) protease inhibitor. Zm00001d049159 is annotated as Leucine-rich repeat receptor-like protein kinase.

In an embodiment, the invention relates to a method for identifying a maize plant or plant part, such as a maize plant or plant part having increased pathogen resistance and/or tolerance, comprising screening for the presence of molecular marker (marker allele) M1. In an embodiment, the invention relates to a method for identifying a maize plant or plant part, such as a maize plant or plant part having increased pathogen resistance and/or tolerance, comprising screening for the presence of molecular marker (marker allele) M2. In an embodiment, the invention relates to a method for identifying a maize plant or plant part, such as a maize plant or plant part having increased pathogen resistance and/or tolerance, comprising screening for the presence of molecular marker (marker allele) M3. In an embodiment, the invention relates to a method for identifying a maize plant or plant part, such as a maize plant or plant part having increased pathogen resistance and/or tolerance, comprising screening for the presence of molecular marker (marker allele) M4. In an embodiment, the invention relates to a method for identifying a maize plant or plant part, such as a maize plant or plant part having increased pathogen resistance and/or tolerance, comprising screening for the presence of molecular marker (marker allele) M5. In an embodiment, the invention relates to a method for identifying a maize plant or plant part, such as a maize plant or plant part having increased pathogen resistance and/or tolerance, comprising screening for the presence of molecular marker (marker allele) M6. In an embodiment, the invention relates to a method for identifying a maize plant or plant part, such as a maize plant or plant part having increased pathogen resistance and/or tolerance, comprising screening for the presence of molecular marker (marker allele) M7. In an embodiment, the invention relates to a method for identifying a maize plant or plant part, such as a maize plant or plant part having increased pathogen resistance and/or tolerance, comprising screening for the presence of molecular marker (marker allele) M8. In an embodiment, the invention relates to a method for identifying a maize plant or plant part, such as a maize plant or plant part having increased pathogen resistance and/or tolerance, comprising screening for the presence of molecular marker (marker allele) M9. In an embodiment, the invention relates to a method for identifying a maize plant or plant part, such as a maize plant or plant part having increased pathogen resistance and/or tolerance, comprising screening for the presence of molecular marker (marker allele) M10. In an embodiment, the invention relates to a method for identifying a maize plant or plant part, such as a maize plant or plant part having increased pathogen resistance and/or tolerance, comprising screening for the presence of molecular marker (marker allele) M11. In an embodiment, the invention relates to a method for identifying a maize plant or plant part, such as a maize plant or plant part having increased pathogen resistance and/or tolerance, comprising screening for the presence of molecular marker (marker allele) M12. In an embodiment, the invention relates to a method for identifying a maize plant or plant part, such as a maize plant or plant part having increased pathogen resistance and/or tolerance, comprising screening for the presence of molecular marker (marker allele) M13. In an embodiment, the invention relates to a method for identifying a maize plant or plant part, such as a maize plant or plant part having increased pathogen resistance and/or tolerance, comprising screening for the presence of molecular marker (marker allele) M14. In an embodiment, the invention relates to a method for identifying a maize plant or plant part, such as a maize plant or plant part having increased pathogen resistance and/or tolerance, comprising screening for the presence of molecular marker (marker allele) M15. In an embodiment, the invention relates to a method for identifying a maize plant or plant part, such as a maize plant or plant part having increased pathogen resistance and/or tolerance, comprising screening for the presence of molecular marker (marker allele) M16. In an embodiment, the invention relates to a method for identifying a maize plant or plant part, such as a maize plant or plant part having increased pathogen resistance and/or tolerance, comprising screening for the presence of molecular marker (marker allele) M17. In an embodiment, the invention relates to a method for identifying a maize plant or plant part, such as a maize plant or plant part having increased pathogen resistance and/or tolerance, comprising screening for the presence of molecular marker (marker allele) M18. In an embodiment, the invention relates to a method for identifying a maize plant or plant part, such as a maize plant or plant part having increased pathogen resistance and/or tolerance, comprising screening for the presence of molecular marker (marker allele) M19. In an embodiment, the invention relates to a method for identifying a maize plant or plant part, such as a maize plant or plant part having increased pathogen resistance and/or tolerance, comprising screening for the presence of molecular marker (marker allele) M20. In an embodiment, the invention relates to a method for identifying a maize plant or plant part, such as a maize plant or plant part having increased pathogen resistance and/or tolerance, comprising screening for the presence of molecular marker (marker allele) M21. In an embodiment, the invention relates to a method for identifying a maize plant or plant part, such as a maize plant or plant part having increased pathogen resistance and/or tolerance, comprising screening for the presence of molecular marker (marker allele) M22. In an embodiment, the invention relates to a method for identifying a maize plant or plant part, such as a maize plant or plant part having increased pathogen resistance and/or tolerance, comprising screening for the presence of molecular marker (marker allele) M23. In an embodiment, the invention relates to a method for identifying a maize plant or plant part, such as a maize plant or plant part having increased pathogen resistance and/or tolerance, comprising screening for the presence of molecular marker (marker allele) M24. In an embodiment, the invention relates to a method for identifying a maize plant or plant part, such as a maize plant or plant part having increased pathogen resistance and/or tolerance, comprising screening for the presence of molecular marker (marker allele) M25. In an embodiment, the invention relates to a method for identifying a maize plant or plant part, such as a maize plant or plant part having increased pathogen resistance and/or tolerance, comprising screening for the presence of molecular marker (marker allele) M25. In an embodiment, the invention relates to a method for identifying a maize plant or plant part, such as a maize plant or plant part having increased pathogen resistance and/or tolerance, comprising screening for the presence of molecular marker (marker allele) M27. In an embodiment, the invention relates to a method for identifying a maize plant or plant part, such as a maize plant or plant part having increased pathogen resistance and/or tolerance, comprising screening for the presence of molecular marker (marker allele) M28. In an embodiment, the invention relates to a method for identifying a maize plant or plant part, such as a maize plant or plant part having increased pathogen resistance and/or tolerance, comprising screening for the presence of molecular marker (marker allele) M29. In an embodiment, the invention relates to a method for identifying a maize plant or plant part, such as a maize plant or plant part having increased pathogen resistance and/or tolerance, comprising screening for the presence of molecular marker (marker allele) M30. In an embodiment, the invention relates to a method for identifying a maize plant or plant part, such as a maize plant or plant part having increased pathogen resistance and/or tolerance, comprising screening for the presence of molecular marker (marker allele) M31. In an embodiment, the invention relates to a method for identifying a maize plant or plant part, such as a maize plant or plant part having increased pathogen resistance and/or tolerance, comprising screening for the presence of molecular marker (marker allele) M32. In an embodiment, the invention relates to a method for identifying a maize plant or plant part, such as a maize plant or plant part having increased pathogen resistance and/or tolerance, comprising screening for the presence of molecular marker (marker allele) M33. In an embodiment, the invention relates to a method for identifying a maize plant or plant part, such as a maize plant or plant part having increased pathogen resistance and/or tolerance, comprising screening for the presence of molecular marker (marker allele) M34. In an embodiment, the invention relates to a method for identifying a maize plant or plant part, such as a maize plant or plant part having increased pathogen resistance and/or tolerance, comprising screening for the presence of molecular marker (marker allele) M35. In an embodiment, the invention relates to a method for identifying a maize plant or plant part, such as a maize plant or plant part having increased pathogen resistance and/or tolerance, comprising screening for the presence of molecular marker (marker allele) M36. In an embodiment, the invention relates to a method for identifying a maize plant or plant part, such as a maize plant or plant part having increased pathogen resistance and/or tolerance, comprising screening for the presence of molecular marker (marker allele) M37. In an embodiment, the invention relates to a method for identifying a maize plant or plant part, such as a maize plant or plant part having increased pathogen resistance and/or tolerance, comprising screening for the presence of molecular marker (marker allele) M38. In an embodiment, the invention relates to a method for identifying a maize plant or plant part, such as a maize plant or plant part having increased pathogen resistance and/or tolerance, comprising screening for the presence of molecular marker (marker allele) M39. In an embodiment, the invention relates to a method for identifying a maize plant or plant part, such as a maize plant or plant part having increased pathogen resistance and/or tolerance, comprising screening for the presence of molecular marker (marker allele) M40. In an embodiment, the invention relates to a method for identifying a maize plant or plant part, such as a maize plant or plant part having increased pathogen resistance and/or tolerance, comprising screening for the presence of molecular marker (marker allele) M41. In an embodiment, the invention relates to a method for identifying a maize plant or plant part, such as a maize plant or plant part having increased pathogen resistance and/or tolerance, comprising screening for the presence of molecular marker (marker allele) M42. In an embodiment, the invention relates to a method for identifying a maize plant or plant part, such as a maize plant or plant part having increased pathogen resistance and/or tolerance, comprising screening for the presence of molecular marker (marker allele) M43. In an embodiment, the invention relates to a method for identifying a maize plant or plant part, such as a maize plant or plant part having increased pathogen resistance and/or tolerance, comprising screening for the presence of molecular marker (marker allele) M44. In an embodiment, the invention relates to a method for identifying a maize plant or plant part, such as a maize plant or plant part having increased pathogen resistance and/or tolerance, comprising screening for the presence of molecular marker (marker allele) M45. In an embodiment, the invention relates to a method for identifying a maize plant or plant part, such as a maize plant or plant part having increased pathogen resistance and/or tolerance, comprising screening for the presence of molecular marker (marker allele) M46. In an embodiment, the invention relates to a method for identifying a maize plant or plant part, such as a maize plant or plant part having increased pathogen resistance and/or tolerance, comprising screening for the presence of molecular marker (marker allele) M47. In an embodiment, the invention relates to a method for identifying a maize plant or plant part, such as a maize plant or plant part having increased pathogen resistance and/or tolerance, comprising screening for the presence of molecular marker (marker allele) M48. In an embodiment, the invention relates to a method for identifying a maize plant or plant part, such as a maize plant or plant part having increased pathogen resistance and/or tolerance, comprising screening for the presence of molecular marker (marker allele) M49. In an embodiment, the invention relates to a method for identifying a maize plant or plant part, such as a maize plant or plant part having increased pathogen resistance and/or tolerance, comprising screening for the presence of molecular marker (marker allele) M50. In an embodiment, the invention relates to a method for identifying a maize plant or plant part, such as a maize plant or plant part having increased pathogen resistance and/or tolerance, comprising screening for the presence of molecular marker (marker allele) M51. In an embodiment, the invention relates to a method for identifying a maize plant or plant part, such as a maize plant or plant part having increased pathogen resistance and/or tolerance, comprising screening for the presence of molecular marker (marker allele) M52. In an embodiment, the invention relates to a method for identifying a maize plant or plant part, such as a maize plant or plant part having increased pathogen resistance and/or tolerance, comprising screening for the presence of molecular marker (marker allele) M53. In an embodiment, the invention relates to a method for identifying a maize plant or plant part, such as a maize plant or plant part having increased pathogen resistance and/or tolerance, comprising screening for the presence of molecular marker (marker allele) M54. In an embodiment, the invention relates to a method for identifying a maize plant or plant part, such as a maize plant or plant part having increased pathogen resistance and/or tolerance, comprising screening for the presence of molecular marker (marker allele) M55. In an embodiment, the invention relates to a method for identifying a maize plant or plant part, such as a maize plant or plant part having increased pathogen resistance and/or tolerance, comprising screening for the presence of molecular marker (marker allele) M56.

In an embodiment, the invention relates to a method for identifying a maize plant or plant part, such as a maize plant or plant part having increased pathogen resistance and/or tolerance, comprising screening for the presence of one or more molecular markers (marker alleles) selected from M1 to M12.

In an embodiment, the invention relates to a method for identifying a maize plant or plant part, such as a maize plant or plant part having increased pathogen resistance and/or tolerance, comprising screening for the presence of one or more molecular markers (marker alleles) selected from M13 to M17.

In an embodiment, the invention relates to a method for identifying a maize plant or plant part, such as a maize plant or plant part having increased pathogen resistance and/or tolerance, comprising screening for the presence of one or more molecular markers (marker alleles) selected from M18 to M30.

In an embodiment, the invention relates to a method for identifying a maize plant or plant part, such as a maize plant or plant part having increased pathogen resistance and/or tolerance, comprising screening for the presence of one or more molecular markers (marker alleles) selected from M31 to M48.

In an embodiment, the invention relates to a method for identifying a maize plant or plant part, such as a maize plant or plant part having increased pathogen resistance and/or tolerance, comprising screening for the presence of one or more molecular markers (marker alleles) selected from M49 to M56.

In an aspect, the invention relates to a method for identifying a maize plant or plant part, such as a maize plant or plant part having increased pathogen resistance and/or tolerance, comprising screening for the presence of Zm00001d049121 comprising a coding sequence as set forth in SEQ ID NO: 1, or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049113 comprising a coding sequence as set forth in SEQ ID NO: 3, or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049145 comprising a coding sequence as set forth in SEQ ID NO: 5, or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049148 comprising a coding sequence as set forth in SEQ ID NO: 7, or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049159 comprising a coding sequence as set forth in SEQ ID NO: 9, or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049121 encoding a protein having a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049113 encoding a protein having a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049145 encoding a protein having a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049148 encoding a protein having a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence.

In an aspect, the invention relates to a method for identifying a maize plant or plant part, such as a maize plant or plant part having increased pathogen resistance and/or tolerance, comprising screening for the presence of Zm00001d049159 encoding a protein having a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

In an aspect, the invention relates to a method for identifying a maize plant or plant part, such as a maize plant or plant part having increased pathogen resistance and/or tolerance, comprising screening for the presence of one or more molecular marker (marker allele) selected from M1 to M12, preferably all, in Zm00001d049121, wherein Zm00001d049121 comprises
(i) a nucleotide sequence having the cDNA of SEQ ID NO: 1;
(ii) a nucleotide sequence encoding for an amino acid sequence having the amino acid sequence of SEQ ID NO: 2;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 1, preferably over the entire length of the sequence;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 2, preferably over the entire length of the sequence;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s).

In an aspect, the invention relates to a method for identifying a maize plant or plant part, such as a maize plant or plant part having increased pathogen resistance and/or tolerance, comprising screening for the presence of one or more molecular marker (alleles) selected from M13 to M17, preferably all, in Zm00001d049113, wherein Zm00001d049113 comprises
(i) a nucleotide sequence having the cDNA of SEQ ID NO 3;
(ii) a nucleotide sequence encoding for an amino acid sequence having the amino acid sequence of SEQ ID NO: 4;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 3, preferably over the entire length of the sequence;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 4, preferably over the entire length of the sequence;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s).

In an aspect, the invention relates to a method for identifying a maize plant or plant part, such as a maize plant or plant part having increased pathogen resistance and/or tolerance, comprising screening for the presence of one or more molecular marker (alleles) selected from M18 to M30, preferably all, in Zm00001d049145, wherein Zm00001d049145 comprises
(i) a nucleotide sequence having the cDNA of SEQ ID NO: 5;
(ii) a nucleotide sequence encoding for an amino acid sequence having the amino acid sequence of SEQ ID NO: 6;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 5, preferably over the entire length of the sequence;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 6, preferably over the entire length of the sequence;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s).

In an aspect, the invention relates to a method for identifying a maize plant or plant part, such as a maize plant or plant part having increased pathogen resistance and/or tolerance, comprising screening for the presence of one or more molecular marker (alleles) selected from M31 to M48, preferably all, in Zm00001d049148, wherein Zm00001d049148 comprises
(i) a nucleotide sequence having the cDNA of SEQ ID NO: 7;
(ii) a nucleotide sequence encoding for an amino acid sequence having the amino acid sequence of SEQ ID NO: 8;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 7, preferably over the entire length of the sequence;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 8, preferably over the entire length of the sequence;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s).

In an aspect, the invention relates to a method for identifying a maize plant or plant part, such as a maize plant or plant part having increased pathogen resistance and/or tolerance, comprising screening for the presence of one or more molecular marker (alleles) selected from M49 to M56, preferably all, in Zm00001d049159, wherein Zm00001d049159 comprises
(i) a nucleotide sequence having the cDNA of SEQ ID NO: 9;
(ii) a nucleotide sequence encoding for an amino acid sequence having the amino acid sequence of SEQ ID NO: 10;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 9, preferably over the entire length of the sequence;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 10, preferably over the entire length of the sequence;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s).

In certain embodiments, a maize plant or plant part is identified, such as is identified as having increased pathogen resistance and/or tolerance, if the QTL allele, one or more of marker allele selected from M1 to M56, one or more polynucleic acid having a coding sequence selected from SEQ ID NOs: 1, 3, 5, 7, or 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 1, 3, 5, 7, or 9, preferably over the entire length of the sequence, or one or more polynucleic acid encoding a sequence of any of SEQ ID NOs: 2, 4, 6, 8, or 10 or encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 2, 4, 6, 8, or 10, preferably over the entire length of the sequence, is identified or present.

In certain embodiments, the method for identifying a maize plant or plant part, such as having increased pathogen resistance and/or tolerance, further comprises the step of selecting a maize plant or plant part having the QTL allele, one or more of marker allele selected from M1 to M56, one or more polynucleic acid having a coding sequence selected from SEQ ID NOs: 1, 3, 5, 7, or 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 1, 3, 5, 7, or 9, preferably over the entire length of the sequence, or one or more polynucleic acid encoding a sequence of any of SEQ ID NOs: 2, 4, 6, 8, or 10 or encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 2, 4, 6, 8, or 10, preferably over the entire length of the sequence.

In certain embodiments, the QTL allele and/or one or more of the molecular marker (marker alleles) are heterozygous.

In certain embodiments, the QTL allele and/or one or more of the molecular marker (marker alleles) are homozygous.

In certain embodiments,
- endogenous Zm00001d049121 comprises a coding sequence as set forth in SEQ ID NO: 11 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 11, preferably over the entire length of the sequence;
- endogenous Zm00001d049113 comprises a coding sequence as set forth in SEQ ID NO: 13 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 13, preferably over the entire length of the sequence;
- endogenous Zm00001d049145 comprises a coding sequence as set forth in SEQ ID NO: 15 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 15, preferably over the entire length of the sequence;
- endogenous Zm00001d049148 comprises a coding sequence as set forth in SEQ ID NO: 17 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 17, preferably over the entire length of the sequence; and/or
- endogenous Zm00001d049159 comprises a coding sequence as set forth in SEQ ID NO: 19 or a coding sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 19, preferably over the entire length of the sequence.

In certain embodiments,
- endogenous Zm00001d049121 encodes a protein sequence as set forth in SEQ ID NO: 12 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 12, preferably over the entire length of the sequence;
- endogenous Zm00001d049113 encodes a protein sequence as set forth in SEQ ID NO: 14 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 14, preferably over the entire length of the sequence;
- endogenous Zm00001d049145 encodes a protein sequence as set forth in SEQ ID NO: 16 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 16, preferably over the entire length of the sequence;
- endogenous Zm00001d049148 encodes a protein sequence as set forth in SEQ ID NO: 18 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 18, preferably over the entire length of the sequence; and/or
- endogenous Zm00001d049159 encodes a protein sequence as set forth in SEQ ID NO: 20 or a protein sequence having a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to SEQ ID NO: 20, preferably over the entire length of the sequence.

In an aspect, the invention relates to a method for generating a maize plant or plant part, such as having increased pathogen resistance and/or tolerance, comprising introducing into the genome of a maize plant or a plant part a QTL allele according to the invention as defined herein elsewhere.

In an aspect, the invention relates to a method for generating a maize plant or plant part, such as having increased pathogen resistance and/or tolerance, comprising introducing into the genome of a maize plant or a plant part one or more molecular marker (marker allele) according to the invention as defined herein elsewhere.

In an aspect, the invention relates to a method for generating a maize plant or plant part, such as having increased pathogen resistance and/or tolerance, comprising introducing into the genome of a maize plant or a plant part and/or expressing in a maize plant or plant part one or more polynucleic acid having a coding sequence as set forth in any one or more sequence selected from SEQ ID NOs: 1, 3, 5, 7, and 9, or comprising a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 1, 3, 5, 7, and 9, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049121 comprising a coding sequence as set forth in SEQ ID NO: 1 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049113 comprising a coding sequence as set forth in SEQ ID NO: 3 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049145 comprising a coding sequence as set forth in SEQ ID NO: 5 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049148 comprising a coding sequence as set forth in SEQ ID NO: 7 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049159 comprising a coding sequence as set forth in SEQ ID NO: 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part one or more one or more polynucleic acid encoding a sequence as set forth in any of SEQ ID NOs: 2, 4, 6, 8, or 10 or encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence of any of SEQ ID NOs: 2, 4, 6, 8, or 10, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049121 encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049113 encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049145 encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049148 encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part a polynucleic acid encoding exogenous Zm00001d049159 encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

In an aspect, the invention relates to a method for generating a maize plant or plant part, such as having increased pathogen resistance and/or tolerance, comprising introducing into the genome of a maize plant or a plant part and/or expressing in a maize plant or plant part
(i) a nucleotide sequence having the cDNA of SEQ ID NO: 1;
(ii) a nucleotide sequence encoding for an amino acid sequence having the amino acid sequence of SEQ ID NO: 2;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 1;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 2;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein said nucleotide sequence comprises one or more molecular markers (marker alleles) M1 to M12, preferably M6 and M7, preferably all.

In an aspect, the invention relates to a method for generating a maize plant or plant part, such as having increased pathogen resistance and/or tolerance, comprising introducing into the genome of a maize plant or a plant part and/or expressing in a maize plant or plant part
(i) a nucleotide sequence having the cDNA of SEQ ID NO 3;
(ii) a nucleotide sequence encoding for an amino acid sequence having the amino acid sequence of SEQ ID NO: 4;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 3;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 4;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M13 to M17, preferably all.

In an aspect, the invention relates to a method for generating a maize plant or plant part, such as having increased pathogen resistance and/or tolerance, comprising introducing into the genome of a maize plant or a plant part and/or expressing in a maize plant or plant part
(i) a nucleotide sequence having the cDNA of SEQ ID NO: 5;
(ii) a nucleotide sequence encoding for an amino acid sequence having the amino acid sequence of SEQ ID NO: 6;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 5
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 6;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M18 to M30, preferably all.

In an aspect, the invention relates to a method for generating a maize plant or plant part, such as having increased pathogen resistance and/or tolerance, comprising introducing into the genome of a maize plant or a plant part and/or expressing in a maize plant or plant part
(i) a nucleotide sequence having the cDNA of SEQ ID NO: 7;
(ii) a nucleotide sequence encoding for an amino acid sequence having the amino acid sequence of SEQ ID NO: 8;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 7
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 8;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M31 to M48, preferably all.

In an aspect, the invention relates to a method for generating a maize plant or plant part, such as having increased pathogen resistance and/or tolerance, comprising introducing into the genome of a maize plant or a plant part and/or expressing in a maize plant or plant part
(i) a nucleotide sequence having the cDNA of SEQ ID NO: 9;
(ii) a nucleotide sequence encoding for an amino acid sequence having the amino acid sequence of SEQ ID NO: 10;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 9;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 10;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M49 to M56, preferably all.

In certain embodiments, introduction into the genome is effected by homologous recombination. In this way, the endogenous sequence is replaced with the introduced (exogenous)sequence.

In certain embodiments, introduction into the genome is effected by random integration. In this way, the endogenous sequence may be maintained in addition to the introduced (exogenous) sequence. In certain embodiments, the endogenous sequence is knocked out or knocked down.

Besides introducing and expressing (exogenous) nucleotide sequences as described above, mutagenesis of endogenous sequences is also contemplated.

In an aspect, the invention relates to a method for generating a maize plant or plant part, such as having increased pathogen resistance and/or tolerance, comprising mutating endogenous Zm00001d049121 to a coding sequence as set forth in SEQ ID NO: 1 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence.

In an aspect, the invention relates to a method for generating a maize plant or plant part, such as having increased pathogen resistance and/or tolerance, comprising mutating endogenous Zm00001d049113 to a coding sequence as set forth in SEQ ID NO: 3 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence.

In an aspect, the invention relates to a method for generating a maize plant or plant part, such as having increased pathogen resistance and/or tolerance, comprising mutating endogenous Zm00001d049145 to a coding sequence as set forth in SEQ ID NO: 5 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence.

In an aspect, the invention relates to a method for generating a maize plant or plant part, such as having increased pathogen resistance and/or tolerance, comprising mutating endogenous Zm00001d049148 to a coding sequence as set forth in SEQ ID NO: 7 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence.

In an aspect, the invention relates to a method for generating a maize plant or plant part, such as having increased pathogen resistance and/or tolerance, comprising mutating endogenous Zm00001d049159 to a coding sequence as set forth in SEQ ID NO: 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence.

In an aspect, the invention relates to a method for generating a maize plant or plant part, such as having increased pathogen resistance and/or tolerance, comprising mutating endogenous Zm00001d049121 to a sequence encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence.

In an aspect, the invention relates to a method for generating a maize plant or plant part, such as having increased pathogen resistance and/or tolerance, comprising mutating endogenous Zm00001d049113 to a sequence encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence.

In an aspect, the invention relates to a method for generating a maize plant or plant part, such as having increased pathogen resistance and/or tolerance, comprising mutating endogenous Zm00001d049145 to a sequence encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence.

In an aspect, the invention relates to a method for generating a maize plant or plant part, such as having increased pathogen resistance and/or tolerance, comprising mutating endogenous Zm00001d049148 to a sequence encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence.

In an aspect, the invention relates to a method for generating a maize plant or plant part, such as having increased pathogen resistance and/or tolerance, comprising mutating endogenous Zm00001d049159 to a sequence encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

In an aspect, the invention relates to a method for generating a maize plant or plant part, such as having increased pathogen resistance and/or tolerance, comprising mutating endogenous Zm00001d049121 to
(i) a nucleotide sequence having the cDNA of SEQ ID NO: 1;
(ii) a nucleotide sequence encoding for an amino acid sequence having the amino acid sequence of SEQ ID NO: 2;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 1;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 2;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein said nucleotide sequence comprises one or more molecular markers M1 to M12, preferably M6 and M7, preferably all.

In an aspect, the invention relates to a method for generating a maize plant or plant part, such as having increased pathogen resistance and/or tolerance, comprising mutating endogenous Zm00001d049113 to
(i) a nucleotide sequence having the cDNA of SEQ ID NO 3;
(ii) a nucleotide sequence encoding for an amino acid sequence having the amino acid sequence of SEQ ID NO: 4;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 3;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 4;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M13 to M17, preferably all.

In an aspect, the invention relates to a method for generating a maize plant or plant part, such as having increased pathogen resistance and/or tolerance, comprising mutating endogenous Zm00001d049145 to
(i) a nucleotide sequence having the cDNA of SEQ ID NO: 5;
(ii) a nucleotide sequence encoding for an amino acid sequence having the amino acid sequence of SEQ ID NO: 6;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 5
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 6;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M18 to M30, preferably all.

In an aspect, the invention relates to a method for generating a maize plant or plant part, such as having increased pathogen resistance and/or tolerance, comprising mutating endogenous Zm00001d049148 to
(i) a nucleotide sequence having the cDNA of SEQ ID NO: 7;
(ii) a nucleotide sequence encoding for an amino acid sequence having the amino acid sequence of SEQ ID NO: 8;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 7
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 8;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M31 to M48, preferably all.

In an aspect, the invention relates to a method for generating a maize plant or plant part, such as having increased pathogen resistance and/or tolerance, comprising mutating endogenous Zm00001d049159 to
(i) a nucleotide sequence having the cDNA of SEQ ID NO: 9;
(ii) a nucleotide sequence encoding for an amino acid sequence having the amino acid sequence of SEQ ID NO: 10;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 9;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 10;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M49 to M56, preferably all.

In an aspect, the invention relates to a method for generating a maize plant or plant part, such as having increased pathogen resistance and/or tolerance, comprising introducing (and expressing) or mutating into the genome of a plant or a plant part a polynucleic acid encoding Zm00001d049121 comprising one or more molecular marker selected from M1 to M12, preferably M6 and M7, preferably all.

In an aspect, the invention relates to a method for generating a maize plant or plant part, such as having increased pathogen resistance and/or tolerance, comprising introducing (and expressing) or mutating into the genome of a plant or a plant part a polynucleic acid encoding Zm00001d049121 comprising one or more molecular marker selected from M13 to M17, preferably all.

In an aspect, the invention relates to a method for generating a maize plant or plant part, such as having increased pathogen resistance and/or tolerance, comprising introducing (and expressing) or mutating into the genome of a plant or a plant part a polynucleic acid encoding Zm00001d049121 comprising one or more molecular marker selected from M18 to M30, preferably all.

In an aspect, the invention relates to a method for generating a maize plant or plant part, such as having increased pathogen resistance and/or tolerance, comprising introducing (and expressing) or mutating into the genome of a plant or a plant part a polynucleic acid encoding Zm00001d049121 comprising one or more molecular marker selected from M31 to M48, preferably all.

In an aspect, the invention relates to a method for generating a maize plant or plant part, such as having increased pathogen resistance and/or tolerance, comprising introducing (and expressing) or mutating into the genome of a plant or a plant part a polynucleic acid encoding Zm00001d049121 comprising one or more molecular marker selected from M49 to M56, preferably all.

In certain embodiments, introducing into the genome as referred to herein comprises transgenesis.

In certain embodiments, introducing into the genome as referred to herein comprises transformation.

In certain embodiments, introducing into the genome as referred to herein comprises recombination, such as homologous recombination.

In certain embodiments, introducing into the genome as referred to herein comprises mutagenesis. Preferably, mutagenesis is based on TILLING, CRISPR-mediated gene editing and/or base editing.

In certain embodiments, introducing into the genome as referred to herein comprises introgression. In certain embodiments, introducing into the genome as referred to herein does not comprise introgression.

In certain embodiments, introducing into the genome as referred to herein comprises introducing into the genome in a plant part. In certain embodiments, the plant part is a plant organ. In certain embodiments, the plant part is a plant tissue. In certain embodiments, the plant part is a plant cell. In certain embodiments, the plant part is a protoplast. In certain embodiments, the plant part is a seed.

In certain embodiments, introducing into the genome as referred to herein comprises introducing into the genome in vitro. In certain embodiments, introducing into the genome as referred to herein comprises introducing into the genome in vivo.

In certain embodiments, the method for generating a maize plant or plant part, such as a maize plant or plant part having increased pathogen resistance and/or tolerance, comprises transforming a plant or plant part, preferably a plant cell or a plant tissue, more preferably a protoplast, a callus, an immature or mature embryo or an inflorescence, with a polynucleic acid comprising a QTL allele as described herein elsewhere, and optionally regenerating a plant or plant part from said plant cell or said plant tissue, preferably said protoplast, said callus, said immature or mature embryo or said inflorescence.

In certain embodiments, the method for generating a maize plant or plant part, such as a maize plant or plant part having increased pathogen resistance and/or tolerance, comprises transforming a plant or plant part, preferably a plant cell or a plant tissue, more preferably a protoplast, a callus, an immature or mature embryo or an inflorescence, with one or more polynucleic acid comprising one or more coding sequence selected from SEQ ID NOs: 1, 3, 5, 7, and 9, or with one or more polynucleic acid comprising a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 1, 3, 5, 7, and 9, preferably over the entire length of the sequence, and optionally regenerating a plant or plant part from said plant cell or said tissue, preferably said protoplast, said callus, said immature or mature embryo or said inflorescence.

In certain embodiments, the method for generating a maize plant or plant part, such as a maize plant or plant part having increased pathogen resistance and/or tolerance, comprises transforming a plant or plant part, preferably a plant cell or a plant tissue, more preferably a protoplast, a callus, an immature or mature embryo or an inflorescence, with one or more polynucleic acid encoding one or more sequence selected from SEQ ID NOs: 2, 4, 6, 8, and 10 or with a polynucleic acid encoding a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 2, 4, 6, 8, and 10, preferably over the entire length of the sequence, and optionally regenerating a plant or plant part from said plant cell or said tissue, preferably said protoplast, said callus, said immature or mature embryo or said inflorescence.

In certain embodiments, the method for generating a maize plant or plant part, such as a maize plant or plant part having increased pathogen resistance and/or tolerance, comprises transforming a plant or plant part, preferably a plant cell or a plant tissue, more preferably a protoplast, a callus, an immature or mature embryo or an inflorescence, with a polynucleic acid encoding one or more of Zm00001d049121, Zm00001d049113, Zm00001d049145, Zm00001d049148, and Zm00001d049159, and optionally regenerating a plant or plant part from said plant cell or said tissue, preferably said protoplast, said callus, said immature or mature embryo or said inflorescence, preferably wherein
- Zm00001d049121 comprises one or more, preferably all, of molecular markers (marker alleles) M1 to M12, preferably M6 and M7;
- Zm00001d049113 comprises one or more, preferably all, of molecular markers (marker alleles) M13 to M17;
- Zm00001d049145 comprises one or more, preferably all, of molecular markers (marker alleles) M18 to M30;
- Zm00001d049148 comprises one or more, preferably all, of molecular markers (marker alleles) M31 to M48; and/or
- Zm00001d049159 comprises one or more, preferably all, of molecular markers (marker alleles) M49 to M56.

In certain embodiments, the transformed plant or plant part does not endogenously comprise the QTL allele according to the invention as described herein.

In certain embodiments, the transformed plant or plant part does not endogenously comprise the one or more molecular marker (marker alleles) according to the invention as described herein.

In certain embodiments, the transformed plant or plant part does not endogenously comprise the polynucleic acid according to the invention as described herein.

In certain embodiments, the methods for obtaining or generating plants or plant parts as described herein according to the invention involve or comprise transgenesis and/or gene editing, such as including CRISPR/Cas, TALEN, ZFN, base editing, prime editing, meganucleases; (induced) mutagenesis, which may or may not be random mutagenesis, such as TILLING.

In certain embodiments, the methods for obtaining plants or plant parts as described herein according to the invention do not involve or comprise transgenesis, gene editing, and/or mutagenesis.

In certain embodiments, the methods for obtaining plants or plant parts as described herein according to the invention involve, comprise or consist of breeding and selection.

In certain embodiments, the methods for obtaining plants or plant parts as described herein according to the invention do not involve, comprise or consist of breeding and selection.

In certain embodiments of the methods, plants, polynucleic acids, or uses of the invention as described herein, the endogenous Zm00001d049121 comprises
(i) a nucleotide sequence having the cDNA of SEQ ID NO: 11;
(ii) a nucleotide sequence encoding for an amino acid sequence having the amino acid sequence of SEQ ID NO: 12;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 11;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 12;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein any one or more, preferably all of molecular markers M1 to M12 are absent and/or wherein the coding sequence of Zm00001d049121 does not comprise SEQ ID NO: 1 or Zm00001d049121 does not encode a protein having a sequence as set forth in SEQ ID NO: 2.

In certain embodiments of the methods, plants, polynucleic acids, or uses of the invention as described herein, the endogenous Zm00001d049113 comprises
(i) a nucleotide sequence having the cDNA of SEQ ID NO 13;
(ii) a nucleotide sequence encoding for an amino acid sequence having the amino acid sequence of SEQ ID NO: 14;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 13;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 14;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein any one or more, preferably all of molecular markers M13 to M17 are absent and/or wherein the coding sequence of Zm00001d049113 does not comprise SEQ ID NO: 3 or Zm00001d049113 does not encode a protein having a sequence as set forth in SEQ ID NO: 4.

In certain embodiments of the methods, plants, polynucleic acids, or uses of the invention as described herein, the endogenous Zm00001d049145 comprises
(i) a nucleotide sequence having the cDNA of SEQ ID NO: 15;
(ii) a nucleotide sequence encoding for an amino acid sequence having the amino acid sequence of SEQ ID NO: 16;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 15
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 16;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein any one or more, preferably all of molecular markers M18 to M30 are absent and/or wherein the coding sequence of Zm00001d049145 does not comprise SEQ ID NO: 5 or Zm00001d049145 does not encode a protein having a sequence as set forth in SEQ ID NO: 6.

In certain embodiments of the methods, plants, polynucleic acids, or uses of the invention as described herein, the endogenous Zm00001d049148 comprises
(i) a nucleotide sequence having the cDNA of SEQ ID NO: 17;
(ii) a nucleotide sequence encoding for an amino acid sequence having the amino acid sequence of SEQ ID NO: 18;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 17
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 18;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein any one or more, preferably all of molecular markers M31 to M48 are absent and/or wherein the coding sequence of Zm00001d049148 does not comprise SEQ ID NO: 7 or Zm00001d049148 does not encode a protein having a sequence as set forth in SEQ ID NO: 8.

In certain embodiments of the methods, plants, polynucleic acids, or uses of the invention as described herein, the endogenous Zm00001d049159 comprises
(i) a nucleotide sequence having the cDNA of SEQ ID NO: 19;
(ii) a nucleotide sequence encoding for an amino acid sequence having the amino acid sequence of SEQ ID NO: 20;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 19;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 20;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein any one or more, preferably all of molecular markers M49 to M56 are absent and/or wherein the coding sequence of Zm00001d049159 does not comprise SEQ ID NO: 9 or Zm00001d049159 does not encode a protein having a sequence as set forth in SEQ ID NO: 10.

In an aspect, the invention relates to a method for generating a plant or plant part, comprising (a) providing a first plant identified or generated according to the invention as described herein, (b) crossing said first plant with a second plant, (c) selecting progeny plants comprising the QTL allele of the invention, any one or more of molecular markers A, B, or C, any one or more of molecular markers (marker alleles) selected from M1 to M56, a polynucleotide sequence as set forth in any one or more sequence selected from SEQ ID NOs: 1, 3, 5, 7, and 9 or comprising a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 1, 3, 5, 7, and 9, preferably over the entire length of the sequence, and/or a polynucleotide sequence encoding a protein as set forth in any one or more sequence selected from SEQ ID NOs: 2, 4, 6, 8, and 10 or encoding a protein comprising a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to one or more sequence selected from SEQ ID NOs: 2, 4, 6, 8, and 10, preferably over the entire length of the sequence; and optionally (d) harvesting or obtaining said maize plant or plant part from said progeny.

The term "pathogen" as used herein generally refers to any type of infectious agent capable of causing an (infectious) disease, and includes without limitation a virus, bacterium, protozoan, prion, viroid, or fungus (including yeasts). Also parasites, such as insects or worms, but also parasitic plants or algae are generally encompassed by the term pathogen as used herein.

In certain embodiments, the term pathogen as used herein refers to a fungal pathogen. In certain embodiments, the term pathogen as used herein refers to a fungal pathogen of the genus *Exserohilum* (synonymous with *Helminthosporium*). In certain embodiments, the term pathogen as used herein refers to a fungal pathogen of the species *Exserohilum turcicum* (synonymous with *Helminthosporium turcicum*). In certain embodiments, the term pathogen as used herein refers to a pathogen causing Northern Corn Leaf Blight.

As used herein, *Exserohilum turcicum* can be used interchangeably with *Helminthosporium turcicum. Exserohilum turcicum* is the anamorph of *Setosphaeria turcica.. Exserohilum turcicuml Setosphaeria turcica* belong to the phylum Ascomycota, the order of Pleosporales, and the family of Pleosporaceae. *Exserohilum turcicum* causes the plant disease Northern corn leaf blight. The most common diagnostic symptom of Northern corn leaf blight on maize is cigar-shaped (elliptical) necrotic gray-green lesions on the leaves of several cm long.

In an aspect, the invention relates to a maize plant or plant part obtained or obtainable by the methods according to the invention as described herein, such as the methods for identifying a maize plant or plant part or the methods for generating a maize plant or plant part. The invention also relates to the progeny of such plants.

In an aspect, the invention relates to a maize plant or plant part comprising the QTL allele according to the invention as described herein. In certain embodiments, the QTL allele is homozygous. In certain embodiments, the QTL allele is heterozygous.

In an aspect, the invention relates to a maize plant or plant part comprising any one or more molecular marker (marker allele) according to the invention as described herein.

In certain embodiments, the molecular marker (marker allele) is homozygous. In certain embodiments, the molecular marker (allele) allele is heterozygous.

In an aspect, the invention relates to a maize plant or plant part comprising any one or more polynucleic acid according to the invention as described herein. In certain embodiments, the polynucleic acid is homozygous. In certain embodiments, the polynucleic acid is heterozygous.

In certain embodiments, the maize plant or plant part thereof is not maize line H102 or a plant part thereof.

In certain embodiments, the maize plant is not a maize variety.

In certain embodiments, the maize plant or plant part is transgenic, gene-edited, or mutagenized. In certain embodiments, the maize plant or plant part is transgenic, gene-edited, or mutagenized in order to comprise the QTL allele, one or more molecular marker (allele), or one or more of the polynucleic acids according to the invention as described herein.

In an aspect, the invention relates to a maize plant or plant part, which may be transgenic or mutagenized, (exogenously) comprising and/or (recombinantly) expressing Zm00001d049121, wherein Zm00001d049121 encodes
(i) a nucleotide sequence having the cDNA of SEQ ID NO: 1;
(ii) a nucleotide sequence encoding for an amino acid sequence having the amino acid sequence of SEQ ID NO: 2;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 1;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 2;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein said nucleotide sequence comprises one or more molecular markers M1 to M12, preferably M6 and M7, preferably all.

In an aspect, the invention relates to a maize plant or plant part, which may be transgenic or mutagenized, (exogenously) comprising and/or (recombinantly) expressing Zm00001d049113, wherein Zm00001d049113 encodes
(i) a nucleotide sequence having the cDNA of SEQ ID NO 3;
(ii) a nucleotide sequence encoding for an amino acid sequence having the amino acid sequence of SEQ ID NO: 4;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 3;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 4;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M13 to M17, preferably all.

In an aspect, the invention relates to a maize plant or plant part, which may be transgenic or mutagenized, (exogenously) comprising and/or (recombinantly) expressing Zm00001d049145, wherein Zm00001d049145 encodes
(i) a nucleotide sequence having the cDNA of SEQ ID NO: 5;
(ii) a nucleotide sequence encoding for an amino acid sequence having the amino acid sequence of SEQ ID NO: 6;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 5
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 6;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M18 to M30, preferably all.

In an aspect, the invention relates to a maize plant or plant part, which may be transgenic or mutagenized, (exogenously) comprising and/or (recombinantly) expressing Zm00001d049148, wherein Zm00001d049148 encodes
(i) a nucleotide sequence having the cDNA of SEQ ID NO: 7;
(ii) a nucleotide sequence encoding for an amino acid sequence having the amino acid sequence of SEQ ID NO: 8;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 7
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 8;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M31 to M48, preferably all.

In an aspect, the invention relates to a maize plant or plant part, which may be transgenic or mutagenized, (exogenously) comprising and/or (recombinantly) expressing Zm00001d049159, wherein Zm00001d049159 encodes
(i) a nucleotide sequence having the cDNA of SEQ ID NO: 9;
(ii) a nucleotide sequence encoding for an amino acid sequence having the amino acid sequence of SEQ ID NO: 10;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 9;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 10;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M49 to M56, preferably all.

As described herein elsewhere, in certain embodiments such maize plant or plant part does not comprise endogenously the recited polynucleic acids.

In an aspect, the invention relates to a (isolated) polynucleic acid comprising the QTL allele according to the invention as described herein.

In an aspect, the invention relates to a (isolated) polynucleic acid comprising one or more molecular marker (marker allele) according to the invention as described herein.

In an aspect, the invention relates to a (isolated) polynucleic acid comprising
(i) a nucleotide sequence having the cDNA of SEQ ID NO: 1;
(ii) a nucleotide sequence encoding for an amino acid sequence having the amino acid sequence of SEQ ID NO: 2;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 1;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 2;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s);
preferably wherein said nucleotide sequence comprises one or more molecular markers M1 to M12, preferably M6 and M7, preferably all.

In an aspect, the invention relates to a (isolated) polynucleic acid comprising
(i) a nucleotide sequence having the cDNA of SEQ ID NO 3;
(ii) a nucleotide sequence encoding for an amino acid sequence having the amino acid sequence of SEQ ID NO: 4;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 3;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 4;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M13 to M17, preferably all.

In an aspect, the invention relates to a (isolated) polynucleic acid comprising
(i) a nucleotide sequence having the cDNA of SEQ ID NO: 5;
(ii) a nucleotide sequence encoding for an amino acid sequence having the amino acid sequence of SEQ ID NO: 6;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 5
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 6;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M18 to M30, preferably all.

In an aspect, the invention relates to a (isolated) polynucleic acid comprising
(i) a nucleotide sequence having the cDNA of SEQ ID NO: 7;
(ii) a nucleotide sequence encoding for an amino acid sequence having the amino acid sequence of SEQ ID NO: 8;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 7
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 8;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M31 to M48, preferably all.

In an aspect, the invention relates to a (isolated) polynucleic acid comprising
(i) a nucleotide sequence having the cDNA of SEQ ID NO: 9;
(ii) a nucleotide sequence encoding for an amino acid sequence having the amino acid sequence of SEQ ID NO: 10;
(iii) a nucleotide sequence having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 9;
(iv) a nucleotide sequence encoding for a polypeptide having at least 98.5%, 98.6%, 98.7%, 98.8%, 98.9%, or 99%, preferably at least 99.1%, 99.2%, 99.3%, 99.4%, or 99.5%, more preferably at least 99.6%, 99.7%, 99.8% or 99.9% identity to the sequence of SEQ ID NO: 10;
(v) a nucleotide sequence hybridizing with the reverse complement of a nucleotide sequence as defined in (i) or (ii) under stringent hybridization conditions; or
(vi) a nucleotide sequence encoding a protein derived from the amino acid sequence encoded by the nucleotide sequence of (i) to (v) by way of substitution, deletion and/or addition of one or more amino acid(s)
preferably wherein said nucleotide sequence comprises one or more molecular markers M49 to M56, preferably all.

In an aspect, the invention relates to a polynucleic acid comprising a coding sequence selected from SEQ ID NOs: 1, 3, 5, 7, and 9 or which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 1, 3, 5, 7, and 9; or which encodes a polypeptide selected from SEQ ID NOs: 2, 4, 6, 8, and 10 or a polypeptide which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 2, 4, 6, 8, and 10.

In an aspect, the invention relates to a polynucleic acid specifically hybridizing with the nucleotide sequence of any one of SEQ ID NOs: 1, 3, 5, 7, and 9 or a nucleotide sequence encoding a protein sequence of any of SEQ ID NOs: 2, 4, 6, 8, and 10, or the complement thereof, or the reverse complement thereof. In certain embodiments, the polynucleic acid is a (PCR) primer or (hybridization) probe. In certain embodiments, the polynucleic acid is an allele-specific primer or probe. In certain embodiments, the polynucleic acid is a KASP primer.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides.

In certain embodiments, the polynucleic acid comprises at most 1500 nucleotides, such as 1200, 1000, 800, 600, 400, 200 nucleotides, such as at most 100, 80, 60, 50, 40, or 30 nucleotides.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides, and the polynucleic acid comprises at most 1500 nucleotides, such as 1200, 1000, 800, 600, 400, 200 nucleotides, such as at most 100, 80, 60, 50, 40, or 30 nucleotides.

In an aspect, the invention relates to a (isolated) polynucleic acid comprising a (molecular) marker (allele) of the invention, or the complement or the reverse complement of a (molecular) marker (allele) of the invention. In certain embodiments, the invention relates to a polynucleic acid comprising at least 10 contiguous nucleotides, preferably at least 15 contiguous nucleotides or at least 20 contiguous nucleotides of a (molecular) marker (allele) of the invention, or the complement or the reverse complement of a (molecular) marker (allele) of the invention. In certain embodiments, the invention relates to a polynucleic acid comprising at least 10 contiguous nucleotides, preferably at least 15 contiguous nucleotides or at least 20 contiguous nucleotides of any of SEQ ID NOs: 1, 3, 5, 7, or 9, or the complement or the reverse complement of any of SEQ ID NOs: 1, 3, 5, 7, or 9. In certain embodiments, the polynucleic acid is capable of discriminating between a (molecular) marker (allele) of the invention and a non-molecular marker allele, such as to specifically hybridise with a (molecular) marker allele of the invention. In certain embodiments, the polynucleic acid is capable of hybridising with a unique nucleotide fragment or section of any of SEQ ID NOs: 1, 3, 5, 7, or 9, or the complement or the reverse complement of any of SEQ ID NOs: 1, 3, 5, 7, or 9. It will be understood that a unique section or fragment preferably refers to a section or fragment comprising the SNP or the respective marker alleles (e.g. InDel) of the invention (such as marker alleles A, B, C, or M1-M56), or a section or fragment comprising the 5' or 3' junction of the insert of a marker allele of the invention or a section or fraction comprised within the insert of a marker allele of the invention (such as marker allele M7), or a section or fragment comprising the junction of the deletion of a marker allele of the invention (such as marker allele M6). In certain embodiments, the polynucleic acid or the complement or reverse complement thereof does not (substantially) hybridise with or bind to (genomic) DNA originating from maize inbred line B73. In certain embodiments, the sequence of the polynucleic acid or the complement or reverse complement thereof does not occur or is not present in maize inbred line B73.

It will be understood that "specifically hybridizing" means that the polynucleic acid hybridises with the (molecular) marker allele (such as under stringent hybridisation conditions, as defined herein elsewhere), but does not (substantially) hybridise with a polynucleic acid not comprising the marker allele or is (substantially) incapable of being used as a PCR primer. By means of example, in a suitable readout, the hybridization signal with the marker allele or PCR amplification of the marker allele is at least 5 times, preferably at least 10 times stronger or more than the hybridisation signal with a non-marker allele, or any other sequence.

In an aspect, the invention relates to a kit comprising such polynucleic acids, such as primers (comprising forward and/or reverse primers) and/or probes. The kit may further comprise instructions for use.

In will be understood that in embodiments relating to a set of forward and reverse primers, only one of both primers (forward or reverse) may need to be capable of discriminating between a (molecular) marker allele of the invention and a non-marker allele, and hence may be unique. The other primer may or may not be capable of discriminating between a (molecular) marker allele of the invention and a non-marker allele, and hence may be unique.

In an aspect, the invention relates to a vector comprising a (isolated) polynucleic acid according to the invention as described herein. In certain embodiments, the vector is a (plant) expression vector. In certain embodiments, the vector is an inducible (plant) expression vector. In certain embodiments, the expression is tissue- or organ-specific. In certain embodiments, the expression is developmentally specific. In certain embodiments, the expression is tissue- or organ-specific and developmentally specific.

As used herein, a "vector" has its ordinary meaning in the art, and may for instance be a plasmid, a cosmid, a phage or an expression vector, a transformation vector, shuttle vector, or cloning vector; it may be double- or single-stranded, linear or circular; or it may transform a prokaryotic or eukaryotic host, either via integration into its genome or extrachromosomally. The nucleic acid according to the invention is preferably operatively linked in a vector with one or more regulatory sequences which allow the transcription, and, optionally, the expression, in a prokaryotic or eukaryotic host cell. A regulatory sequence-preferably, DNA-may be homologous or heterologous to the nucleic acid according to the invention. For example, the nucleic acid is under the control of a suitable promoter or terminator. Suitable promoters may be promoters which are constitutively induced (example: 35S promoter from the "Cauliflower mosaic virus" (Odell et al., 1985); those promoters which are tissue-specific are especially suitable (example: Pollen-specific promoters, Chen et al. (2010), Zhao et al. (2006), or Twell et al. (1991)), or are development-specific (example: blossom-specific promoters). Suitable promoters may also be synthetic or chimeric promoters which do not occur in nature, are composed of multiple elements, and contain a minimal promoter, as well as-upstream of the minimum promoter-at least one cis-regulatory element which serves as a binding location for special transcription factors. Chimeric promoters may be designed according to the desired specifics and are induced or repressed via different factors. Examples of such promoters are found in Gurr & Rushton (2005) or Venter (2007). For example, a suitable terminator is the nos-terminator (Depicker et al., 1982). The vector may be introduced via conjugation, mobilization, biolistic transformation, agrobacteria-mediated transformation, transfection, transduction, vacuum infiltration, or electroporation. The vector may be a plasmid, a cosmid, a phage or an expression vector, a transformation vector, shuttle vector, or cloning vector; it may be double- or single-stranded, linear or circular. The vector may transform a prokaryotic or eukaryotic host, either via integration into its genome or extrachromosomally.

As used herein, the term "operatively linked" or "operably linked" means connected in a common nucleic acid molecule in such a manner that the connected elements are positioned and oriented relative to one another such that a transcription of the nucleic acid molecule may occur. A DNA which is operatively linked with a promoter is under the transcriptional control of this promoter. In an aspect, the invention relates to the use of the polynucleic acid or vector according to the invention as described herein for generating a maize plant or plant part, such as a maize plant or plant part having increased pathogen resistance and/or tolerance.

In certain embodiments, the vector is an expression vector. The nucleic acid is preferably operatively linked in a vector with one or more regulatory sequences which allow the transcription, and optionally the expression, in a prokaryotic or eukaryotic host cell. A regulatory sequence may be homologous or heterologous to the nucleic acid. For example, the nucleic acid is under the control of a suitable promoter or terminator. Suitable promoters may be promoters which are constitutively induced, for example, the 35S promoter from the "Cauliflower mosaic virus" (Odell et al., 1985. Identification of DNA sequences required for activity of the cauliflower mosaic virus 35S promoter.) Tissue-specific promoters, e.g. pollen-specific promoters as described in Chen et al. (2010. Molecular Biology Reports 37(2):737-744), Zhao et al. (2006. Planta 224(2): 405-412), or Twell et al. (1991. Genes & Development 5(3): 496-507), are particularly suitable, as are development-specific promoters, e.g. blossom-specific promoters. Suitable promoters may also be synthetic or chimeric promoters which do not occur in nature, and which are composed of multiple elements. Such synthetic or chimeric promoter may contain a minimal promoter, as well as at least one cis-regulatory element which serves as a binding location for special transcription factors. Chimeric promoters may be designed according to the desired specifics and can be induced or repressed via different factors. Examples of such promoters are found in Gurr & Rushton (2005. Trends in Biotechnology 23(6): 275-282) or Venter (2007. Trends in Plant Science: 12(3):, 118-124). For example, a suitable terminator is the nos-terminator (Depicker et al., 1982. Journal of Molecular and Applied Genetics 1(6): 561-573).

In certain embodiments, the vector is a conditional expression vector. In certain embodiments, the vector is a constitutive expression vector. In certain embodiments, the vector is a tissue-specific expression vector, such as a pollen-specific expression vector. In certain embodiments, the vector is an inducible expression vector. All such vectors are well-known in the art.

Methods for preparation of the described vectors are commonplace to the person skilled in the art (Sambrook et al., 2001).

Also envisaged herein is a host cell, such as a plant cell, which comprises a nucleic acid as described herein, preferably an induction-promoting nucleic acid or a nucleic acid encoding a double-stranded RNA as described herein, or a vector as described herein. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

The host cell may be a prokaryotic (for example, bacterial) or eukaryotic cell (for example, a plant cell or a yeast cell). For example, the host cell may be an agrobacterium, such as Agrobacterium tumefaciens or Agrobacterium rhizogenes. Preferably, the host cell is a plant cell.

In an aspect, the invention relates to the use of the polynucleic acid or vector according to the invention as described herein for identifying a maize plant or plant part, such as a maize plant or plant part having increased pathogen resistance and/or tolerance.

A nucleic acid described herein or a vector described herein may be introduced in a host cell via well-known methods, which may depend on the selected host cell, including, for example, conjugation, mobilization, biolistic transformation, agrobacteria-mediated transformation, transfection, transduction, vacuum infiltration, or electroporation. In particular, methods for introducing a nucleic acid or a vector in an agrobacterium cell are well-known to the skilled person and may include conjugation or electroporation methods. Also methods for introducing a nucleic acid or a vector into a plant cell are known (Sambrook et al., 2001) and may include diverse transformation methods such as biolistic transformation and agrobacterium-mediated transformation.

In particular embodiments, the present invention relates to a transgenic plant cell which comprises a nucleic acid as described herein, in particular an induction-promoting nucleic acid or a nucleic acid encoding a double-stranded RNA as described herein, as a transgene or a vector as described herein. In further embodiments, the present invention relates to a transgenic plant or a part thereof which comprises the transgenic plant cell.

For example, such a transgenic plant cell or transgenic plant is a plant cell or plant which is, preferably stably, transformed with a nucleic acid as described herein, in particular an induction-promoting nucleic acid or a nucleic acid encoding a double-stranded RNA as described herein, or a vector as described herein.

Preferably, the nucleic acid in the transgenic plant cell is operatively linked with one or more regulatory sequences which allow the transcription, and optionally the expression, in the plant cell. A regulatory sequence may be homologous or heterologous to the nucleic acid. The total structure made up of the nucleic acid according to the invention and the regulatory sequence(s) may then represent the transgene.

In an aspect, the invention relates to the use of one or more of the (molecular) marker (allele) described herein for identifying a plant or plant part having increased pathogen resistance and/or tolerance. In an aspect, the invention relates to the use of one or more of the (molecular) marker (allele) described herein which are able to detect at least one diagnostic marker allele for identifying a plant or plant part, such as having increased pathogen resistance and/or tolerance. In an aspect, the invention relates to the detection of one or more of the (molecular) marker alleles described herein for identifying a plant or plant part having increased pathogen resistance and/or tolerance.

The marker alleles of the invention as described herein may be diagnostic marker alleles which are useable for identifying plants or plant parts having increased pathogen resistance and/or tolerance.

In an aspect, the invention relates to a maize plant or plant part, such as a protoplast, comprising the polynucleic acid or vector according to the invention as described herein.

In an aspect, the invention relates to a method for controlling pathogen infestation in a maize plant (population) comprising
a) Providing (a) maize plant(s) according to the invention as described herein or growing from seeds (a) maize plant(s) according to the invention as described herein,
b) Cultivating the plant(s) of a) under conditions of pathogen infestation.

In certain embodiments, pathogen infestation is reduced. In certain embodiments, pathogen infestation is delayed. In certain embodiments, pathogen symptoms are reduced. In certain embodiments, pathogen symptoms are delayed. In certain embodiments, infestation and symptoms are reduced or delayed. In certain embodiments, the number of lesions is reduced, such as the average or mean number of lesions per plant or the average or mean number of lesions in a plant population (such as for instance expressed in number of plants or per growth area, such as per hectare). A reduction in number of lesions can be at least 5%, preferably at least 10%, more preferably at least 20%. A delay in pathogen infestation or symptoms can be at least one week, preferably at least two weeks, more preferably at least one month.

The plants, plant parts or plant populations as described herein having increased pathogen resistance or tolerance can be used to control pathogen infestation or infection. Accordingly, in an aspect, the invention relates to the use of such plants for controlling pathogen infestation or infection. Preferably pathogen infestation or infection is controlled by reduction of pathogen infestation or infection or as reduction in the symptoms of pathogen infestation or infection, at the plant, plant part, or plant population level, such as further described below.

In certain embodiments, an increased resistance or tolerance may present itself as a reduction of infection or infestation (e.g. the amount of pathogens (e.g. per plant area or per plant biomass), the multiplication (rate) or spread (rate)/distribution of pathogens, as well as the speed of pathogen spreading such as at a specific time during the (growth) season) at the (sub) plant level (such as for instance particular cells, organs, or tissues, for instance harvestable parts of a plant or for instance leaves, stalks, fruits, or seeds) or at the population level, such as a reduction of at least 5%, preferably at least 10%, such as at least 20%, at least 30%, at least 40%, at least 50%, at least 60%, at least 70%, at least 80%, at least 90%, or (about) 100%. At the population level, an increased resistance or tolerance may present itself as a reduction of infection or infestation as described above, but also for instance as a reduction in the amount of infected plants (or a combination), such as a reduction of at least 5%, preferably at least 10%, such as at least 20%, at least 30%, at least 40%, at least 50%, at least 60%, at least 70%, at least 80%, at least 90%, or (about) 100%. It will be understood that such reduction of infection or infestation can be relative to a reference plant (part) or population (such as a corresponding wild type plant not according to the invention).

In certain embodiments, an increased resistance or tolerance may present itself as a reduction in the loss of biomass or yield in general or of a particular (harvestable) plant part (such as seed or fruit amount or weight) due to or as a consequence of pathogen infection. In certain embodiments, the resistant or tolerant plants exhibit a loss in biomass production (such as expressed in g/day or kg/ha or kg/ha/day, such as expressed as dry matter for instance expressed as weight percent) under pathogen infection which is at least 1%, preferably at least 2%, such as at least 3%, at least 4%, at least 5%, such as at least 10%, at least 15%, or at least 20% or more, lower than corresponding control plants, such as plants which are less resistant or tolerant, or plants not according to the invention as described herein. In certain embodiments, the resistant or tolerant plants exhibit biomass production (such as expressed in g/day or kg/ha or kg/ha/day, such as expressed as dry matter for instance expressed as weight percent) under pathogen infection which is at least 1%, preferably at least 2%, such as at least 3%, at least 4%, at least 5%, such as at least 10%, at least 15%, or at least 20% or more, higher than corresponding control plants, such as plants which are less resistant or tolerant, or plants not according to the invention as described herein.

As used herein, the term "yield potential" refers to maximum yield obtainable at harvest.

In an aspect, the invention relates to the use of a maize plant according to the invention as described herein for controlling pathogen infestation in a maize plant (population).

In an aspect, the invention relates to the use of a maize plant according to the invention as described herein for increasing the yield (potential) of a plant, preferably under conditions of pathogen infestation. In certain embodiments, the yield is biomass or seed yield. In certain embodiments, said biomass is whole plant biomass or biomass of a plant part. In certain embodiments, said plant part is a tissue, organ, fruit, or seed.

In certain embodiments of the methods, plants, polynucleic acids, or uses as described herein, the maize plant or plant part comprises molecular marker (allele) M1. In certain embodiments of the methods, plants, polynucleic acids, or uses as described herein, the maize plant or plant part comprises molecular marker (allele) M2. In certain embodiments of the methods, plants, polynucleic acids, or uses as described herein, the maize plant or plant part comprises molecular marker (allele) M3. In certain embodiments of the methods, plants, polynucleic acids, or uses as described herein, the maize plant or plant part comprises molecular marker (allele) M4. In certain embodiments of the methods, plants, polynucleic acids, or uses as described herein, the maize plant or plant part comprises molecular marker (allele) M5. In certain embodiments of the methods, plants, polynucleic acids, or uses as described herein, the maize plant or plant part comprises molecular marker (allele) M6. In certain embodiments of the methods, plants, polynucleic acids, or uses as described herein, the maize plant or plant part comprises molecular marker (allele) M7. In certain embodiments of the methods, plants, polynucleic acids, or uses as described herein, the maize plant or plant part comprises molecular marker (allele) M6 and M7.ln certain embodiments of the methods, plants, polynucleic acids, or uses as described herein, the maize plant or plant part comprises molecular marker (allele) M8. In certain embodiments of the methods, plants, polynucleic acids, or uses as described herein, the maize plant or plant part comprises molecular marker (allele) M9. In certain embodiments of the methods, plants, polynucleic acids, or uses as described herein, the maize plant or plant part comprises molecular marker (allele) M10. In certain embodiments of the methods, plants, polynucleic acids, or uses as described herein, the maize plant or plant part comprises molecular marker (allele) M11. In certain embodiments of the methods, plants, polynucleic acids, or uses as described herein, the maize plant or plant part comprises molecular marker (allele) M12. In certain embodiments of the methods, plants, polynucleic acids, or uses as described herein, the maize plant or plant part comprises molecular marker (allele) M13. In certain embodiments of the methods, plants, polynucleic acids, or uses as described herein, the maize plant or plant part comprises molecular marker (allele) M14. In certain embodiments of the methods, plants, polynucleic acids, or uses as described herein, the maize plant or plant part comprises molecular marker (allele) M15. In certain embodiments of the methods, plants, polynucleic acids, or uses as described herein, the maize plant or plant part comprises molecular marker (allele) M16. In certain embodiments of the methods, plants, polynucleic acids, or uses as described herein, the maize plant or plant part comprises molecular marker (allele) M17. In certain embodiments of the methods, plants, polynucleic acids, or uses as described herein, the maize plant or plant part comprises molecular marker (allele) M18. In certain embodiments of the methods, plants, polynucleic acids, or uses as described herein, the maize plant or plant part comprises molecular marker (allele) M19. In certain embodiments of the methods, plants, polynucleic acids, or uses as described herein, the maize plant or plant part comprises molecular marker (allele) M20. In certain embodiments of the methods, plants, polynucleic acids, or uses as described herein, the maize plant or plant part comprises molecular marker (allele) M21. In certain embodiments of the methods, plants, polynucleic acids, or uses as described herein, the maize plant or plant part comprises molecular marker (allele) M22. In certain embodiments of the methods, plants, polynucleic acids, or uses as described herein, the maize plant or plant part comprises molecular marker (allele) M23. In certain embodiments of the methods, plants, polynucleic acids, or uses as described herein, the maize plant or plant part comprises molecular marker (allele) M24. In certain embodiments of the methods, plants, polynucleic acids, or uses as described herein, the maize plant or plant part comprises molecular marker (allele) M25. In certain embodiments of the methods, plants, polynucleic acids, or uses as described herein, the maize plant or plant part comprises molecular marker (allele) M25. In certain embodiments of the methods, plants, polynucleic acids, or uses as described herein, the maize plant or plant part comprises molecular marker (allele) M27. In certain embodiments of the methods, plants, polynucleic acids, or uses as described herein, the maize plant or plant part comprises molecular marker (allele) M28. In certain embodiments of the methods, plants, polynucleic acids, or uses as described herein, the maize plant or plant part comprises molecular marker (allele) M29. In certain embodiments of the methods, plants, polynucleic acids, or uses as described herein, the maize plant or plant part comprises molecular marker (allele) M30. In certain embodiments of the methods, plants, polynucleic acids, or uses as described herein, the maize plant or plant part comprises molecular marker (allele) M31. In certain embodiments of the methods, plants, polynucleic acids, or uses as described herein, the maize plant or plant part comprises molecular marker (allele) M32. In certain embodiments of the methods, plants, polynucleic acids, or uses as described herein, the maize plant or plant part comprises molecular marker (allele) M33. In certain embodiments of the methods, plants, polynucleic acids, or uses as described herein, the maize plant or plant part comprises molecular marker (allele) M34. In certain embodiments of the methods, plants, polynucleic acids, or uses as described herein, the maize plant or plant part comprises molecular marker (allele) M35. In certain embodiments of the methods, plants, polynucleic acids, or uses as described herein, the maize plant or plant part comprises molecular marker (allele) M36. In certain embodiments of the methods, plants, polynucleic acids, or uses as described herein, the maize plant or plant part comprises molecular marker (allele) M37. In certain embodiments of the methods, plants, polynucleic acids, or uses as described herein, the maize plant or plant part comprises molecular marker (allele) M38. In certain embodiments of the methods, plants, polynucleic acids, or uses as described herein, the maize plant or plant part comprises molecular marker (allele) M39. In certain embodiments of the methods, plants, polynucleic acids, or uses as described herein, the maize plant or plant part comprises molecular marker (allele) M40. In certain embodiments of the methods, plants, polynucleic acids, or uses as described herein, the maize plant or plant part comprises molecular marker (allele) M41. In certain embodiments of the methods, plants, polynucleic acids, or uses as described herein, the maize plant or plant part comprises molecular marker (allele) M42. In certain embodiments of the methods, plants, polynucleic acids, or uses as described herein, the maize plant or plant part comprises molecular marker (allele) M43. In certain embodiments of the methods, plants, polynucleic acids, or uses as described herein, the maize plant or plant part comprises molecular marker (allele) M44. In certain embodiments of the methods, plants, polynucleic acids, or uses as described herein, the maize plant or plant part comprises molecular marker (allele) M45. In certain embodiments of the methods, plants, polynucleic acids, or uses as described herein, the maize plant or plant part comprises molecular marker (allele) M46. In certain embodiments of the methods, plants, polynucleic acids, or uses as described herein, the maize plant or plant part comprises molecular marker (allele) M47. In certain embodiments of the methods, plants, polynucleic acids, or uses as described herein, the maize plant or plant part comprises molecular marker (allele) M48. In certain embodiments of the methods, plants, polynucleic acids, or uses as described herein, the maize plant or plant part comprises molecular marker (allele) M49. In certain embodiments of the methods, plants, polynucleic acids, or uses as described herein, the maize plant or plant part comprises molecular marker (allele) M50. In certain embodiments of the methods, plants, polynucleic acids, or uses as described herein, the maize plant or plant part comprises molecular marker (allele) M51. In certain embodiments of the methods, plants, polynucleic acids, or uses as described herein, the maize plant or plant part comprises molecular marker (allele) M52. In certain embodiments of the methods, plants, polynucleic acids, or uses as described herein, the maize plant or plant part comprises molecular marker (allele) M53. In certain embodiments of the methods, plants, polynucleic acids, or uses as described herein, the maize plant or plant part comprises molecular marker (allele) M54. In certain embodiments of the methods, plants, polynucleic acids, or uses as described herein, the maize plant or plant part comprises molecular marker (allele) M55. In certain embodiments of the methods, plants, polynucleic acids, or uses as described herein, the maize plant or plant part comprises molecular marker (allele) M56.

In certain embodiments of the methods, plants, polynucleic acids, or uses as described herein, the maize plant or plant part comprises molecular marker (alleles) M1 to M12.

In certain embodiments of the methods, plants, polynucleic acids, or uses as described herein, the maize plant or plant part comprises molecular marker (alleles) M13 to M17.

In certain embodiments of the methods, plants, polynucleic acids, or uses as described herein, the maize plant or plant part comprises molecular marker (alleles) M18 to M30.

In certain embodiments of the methods, plants, polynucleic acids, or uses as described herein, the maize plant or plant part comprises molecular marker (alleles) M31 to M48.

In certain embodiments of the methods, plants, polynucleic acids, or uses as described herein, the maize plant or plant part comprises molecular marker (alleles) M49 to M56.

The aspects and embodiments of the invention are further supported by the following non-limiting examples. The following examples, including the experiments conducted and the results achieved, are provided for illustrative purposes only and are not constructed as limiting the present invention.

### EXAMPLES

### EXAMPLE 1: Fine mapping and cloning of the resistance gene HTM on chromosome 4

### QTL mapping and development of recombinants

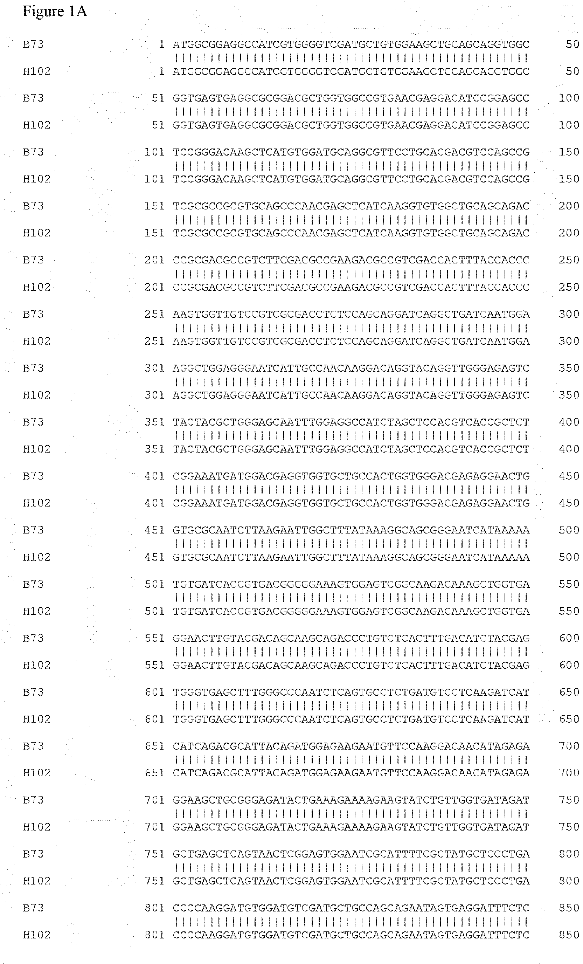

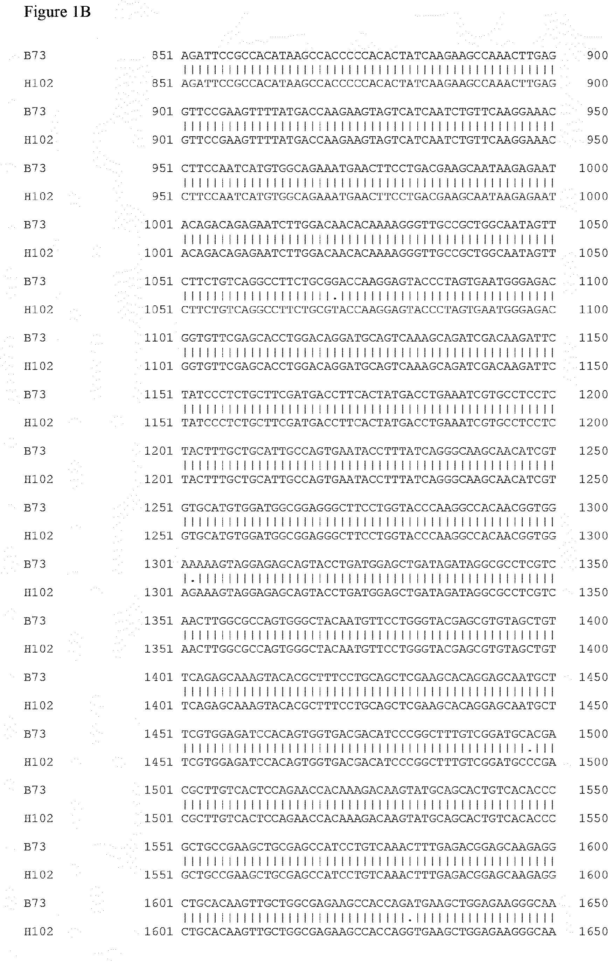

The donor line H102 (Welz, H.G. 1998) was crossed and backcrossed with the KWS proprietary lines 5FX and 9QX to create two F2 populations for QTL mapping. The F2 populations were planted in the field with 538 + 489 individuals. QTL mapping resulted in a peak (LOD value for population with 5FX = 62.66 (Figure 1A); LOD value for population with 9QX = 25.45 (Figure 1B)) for both populations on chromosome 4. Additional QTL peaks could be detected on chromosome 1 (LOD-values 12.4/14.6) and chromosome 8 (LOD-values 9.7/4.7).

For recombinant plant development for the QTL on chromosome 4 two additional mapping populations with 1GX and 1AX were developed. The generation BC3S3 was genotyped and the generation BC3S4 was phenotyped with 66 and 103 recombinants respectively at 4 locations. These recombinants only harbour the donor fragment on chromosome 4 and have the recurrent parent fragment on the QTL regions on chromosome 1 and 8. Therefore the effect of QTL region on chromosome 4 could be validated. The validated region in the 1AX-population encompasses 67.4 Mbp/20.6 putcM and in the 1GX-population 2.36 Mbp/4.21 putcM. The effect of the donor allele on chromosome 4 versus the recurrent parent allele is 4.3 scoring notes.

### Molecular analysis of target region

For the donor line H102 genomic resources were established:
a. whole genome sequencing with 40x Illumina and de novo assembly;
b. a BAC library was established at CNRGV.

The validated region in the 1GX-population 2.36 Mbp/4.21 putcM was further investigated for putative candidate genes. The region encompasses in total 42 genes (based on B73AGPv04 annotation) from which five genes were selected for further evaluation:
Zm00001d049121 is annotated as Nucleotide-binding site-leucine-rich repeat protein (NBS-LRR protein). At cDNA-level the resistant H102 genotype shares 98.3% identity with susceptible B73 genotype and at protein level 97.7%. The amino acid sequences differ in particular in the second part (C-terminal) of the protein. Several amino acid substitutions are detectable (positions with reference to B73 allele of Zm00001d049121). Amino acid substitutions with conservation between groups of weakly similar properties roughly equivalent to scoring =< 0.5 and > 0 in the Gonnet PAM 250 matrix are: D544G, M564T, K603S, P604L, A605E and C821R. Amino acid substitutions with conservation between groups of strongly similar properties as below - roughly equivalent to scoring > 0.5 in the Gonnet PAM 250 matrix are: N682S, E844K and H895N. Apart of these substitutions the H102 genotype shows an insertion of 10 amino acids (-Asn-Leu-Gln-Tyr-Val-Arg-Val-Thr-Ser-Cys-) between positions 602 and 603 referenced to Zm00001d049121 derived from B73, and a small deletion of 2 amino acids (-Arg-Asp-) at positions 809 and 810 referenced to Zm00001d049121 derived from B73.

Zm00001d049113 annotated as Papain like cysteine protease (C1 type). At cDNA-level the resistant H102 genotype shares 99.4% identity with susceptible B73 genotype and at protein level 99.7%. The amino acid sequences differ only in position 138 referenced to Zm00001d049113 derived from B73 where the B73 allele carries an alanine (A) which is missing in resistant H102 genotype at this position.

Zm00001d049145 is annotated as CRINKLY4-like protein kinase. At cDNA-level the resistant H102 genotype shares 99.4% identity with susceptible B73 genotype and at protein level 99.1%. Several amino acid substitutions are detectable (positions with reference to the B73 allele). Amino acid substitutions with conservation between groups of weakly similar properties roughly equivalent to scoring =< 0.5 and > 0 in the Gonnet PAM 250 matrix are: N83A, A366V and P428A. Amino acid substitutions with conservation between groups of strongly similar properties as below - roughly equivalent to scoring > 0.5 in the Gonnet PAM 250 matrix are: V368L, A387S and K414R. Apart of these substitutions the H102 genotype shows one additional amino acid, namely Asparagine (N) between positions 414 and 415 referenced to B73 allele.

Zm00001d049148 is annotated as Serine (or cysteine) protease inhibitor. At cDNA-level the resistant H102 genotype shares 98.7% identity with susceptible B73 genotype and at protein level 98.7%. Several amino acid substitutions are detectable (positions with reference to the B73 allele). Amino acid substitutions with conservation between groups of weakly similar properties roughly equivalent to scoring =< 0.5 and > 0 in the Gonnet PAM 250 matrix are: V113A, G170V and L198Q. Amino acid substitutions with conservation between groups of strongly similar properties as below - roughly equivalent to scoring > 0.5 in the Gonnet PAM 250 matrix are: V138I, T368S and V434L.

Zm00001d049159 is annotated as Leucine-rich repeat receptor-like protein kinase. At cDNA-level the resistant H102 genotype shares 99.3% identity with susceptible B73 genotype and at protein level 99.5%. Several amino acid substitutions are detectable (positions with reference to the B73 allele). Amino acid substitutions with conservation between groups of weakly similar properties roughly equivalent to scoring =< 0.5 and > 0 in the Gonnet PAM 250 matrix are: F8L and A16V. Amino acid substitutions with conservation between groups of strongly similar properties as below - roughly equivalent to scoring > 0.5 in the Gonnet PAM 250 matrix are: V64I and Q672E. Apart of these substitutions the H102 genotype shows a small insertion of 2 amino acids (-Gly-Pro-) between positions 18 and 19 referenced to B73 allele.

**Table 2: Sequences (SEQ ID NOs) of selected genes and corresponding B73 references:**

| **gene identifier** | **H102** | | **B73** | |
|---|---|---|---|---|
| | **cDNA** | **protein** | **cDNA** | **protein** |
| Zm00001d049121 | 1 | 2 | 11 | 12 |
| Zm00001d049113 | 3 | 4 | 13 | 14 |
| Zm00001d049145 | 5 | 6 | 15 | 16 |
| Zm00001d049148 | 7 | 8 | 17 | 18 |
| Zm00001d049159 | 9 | 10 | 19 | 20 |

### Validation of the selected genes

For all selected genes probes for BAC library screening were developed in order to obtain a larger genomic fragment of the donor line H102. The functional validation was performed by transformation of the H102 allele located on the fragments into the susceptible maize genotype A188.

In a first expression cassette, the coding sequence of Zm00001d049121 is fused to the sequence of the constitutively active OsActin promoter including the intronic sequence and a terminator. Additionally, expression cassettes with the genomic fragment of Zm00001d049121 under control of either its native promoter or the constitutively active BdEF1 promoter and a terminator were designed. For the selected genes Zm00001d049113, Zm00001d049145, Zm00001d049148, Zm00001d049159 expression cassettes with the BdEF1 promoter and the respective genomic fragments of the selected genes as well as a terminator sequence were prepared. Each of these expression cassettes was transformed into a binary vector containing a herbicide gene (e.g.: BASTA resistance, glyphosate resistance or ALS inhibitor resistance) for subsequent transformation into Agrobacterium tumefaciens for Agrobacterium mediated plant transformation into maize (Zea mays) genotype A188. T0 plants have been multiplied and progenies grown in greenhouse are tested for resistance to NCLB.

For that, 20 individuals per genotype were grown in pots. The controls were untransformed A188 genotype as well as A188 genotype transformed with an empty vector. 14 days after sowing, an artificial infection was carried out using infected and ground leaf material which was administered to the plants to be tested. This type of inoculation allowed a comparable H. turcicum infestation to be simulated in different test years and at different locations independently of the prevailing natural infestation conditions there. After a further 2 to 3 weeks, the first symptoms of disease developed. From the time of the appearance of the first symptoms, every other day the classification scores of the HT resistance feature as well as the number of plants with symptoms were determined. From this, the AUDPC (area under disease progress curve) was determined. The infestation frequency (as the %/time x period) was used to classify the plants under investigation; here, an AUDPC from 0 - 100 was resistant, 101 - 450 was heterozygous, and > 450 was vulnerable.

### Marker development

With several further proprietary and publicly available sequences alignments of the selected genes Zm00001d049113, Zm00001d049121, and Zm00001d049159 have been performed and analysed for diagnostic marker development. For the genes Zm00001d049113 and Zm00001d049121 three diagnostic SNPs (specific for the donor line) were converted to KASP marker assays (Table 3).

**Table 3: KASP marker sequences**

| **Gene identifier** | **marker sequence with SNP in** 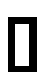 | **donor allele** | **SEQ ID NO:** |
|---|---|---|---|
| Zm00001d049121 | AAGACCTTCA[A/G]CCACATGATC | G | 21 |
| Zm00001d049121 | GGCACTTCCC[A/G]AGCTTGGGGA | A | 22 |
| Zm00001d049113 | GTATTAAAAM[A/T]TCGGTACTGG | A | 23 |

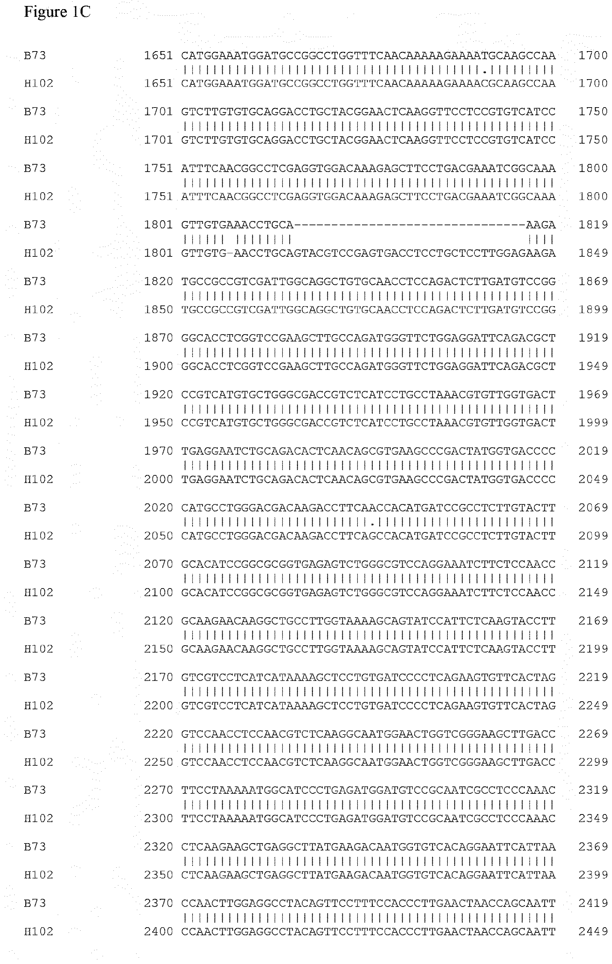

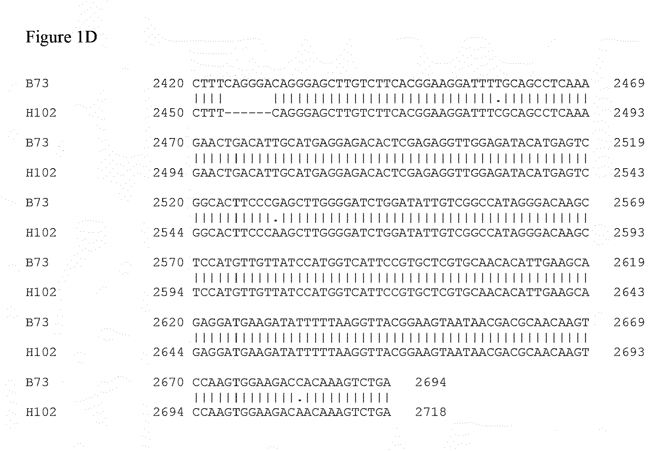

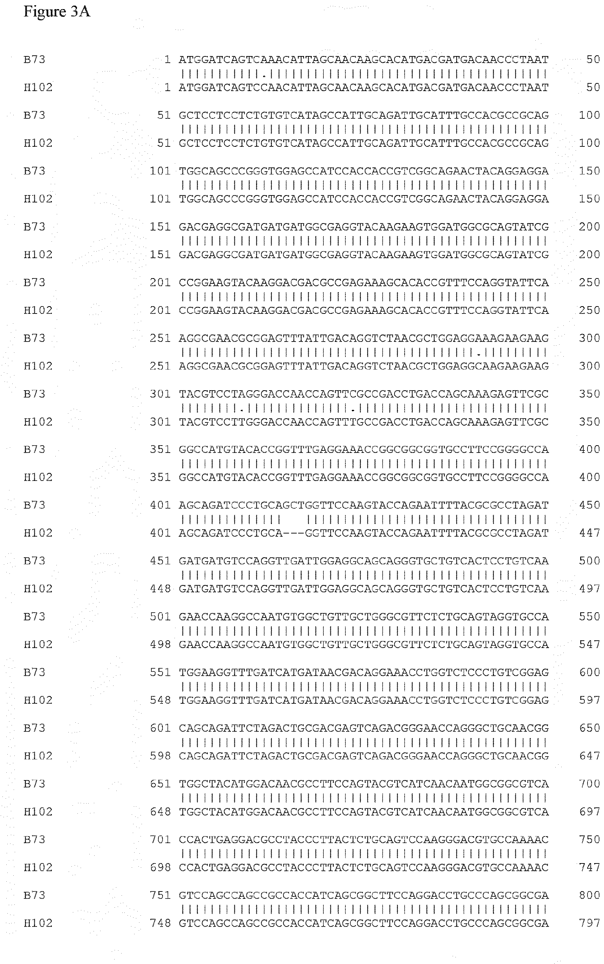

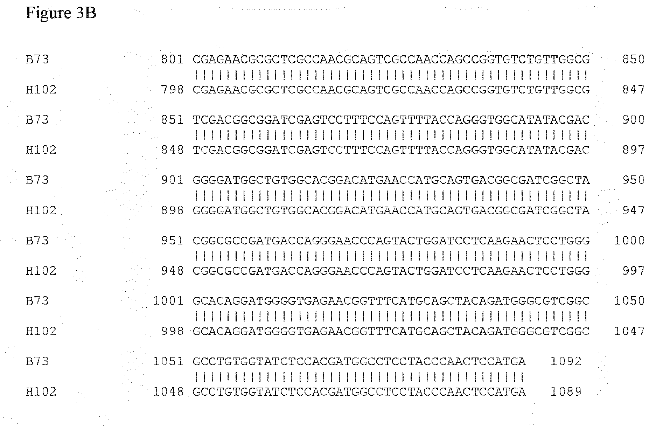

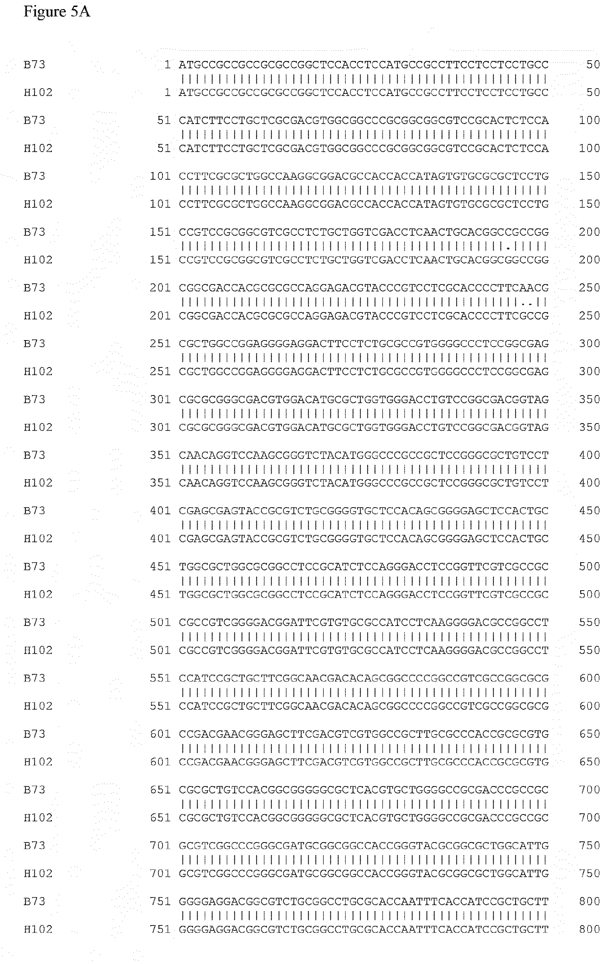

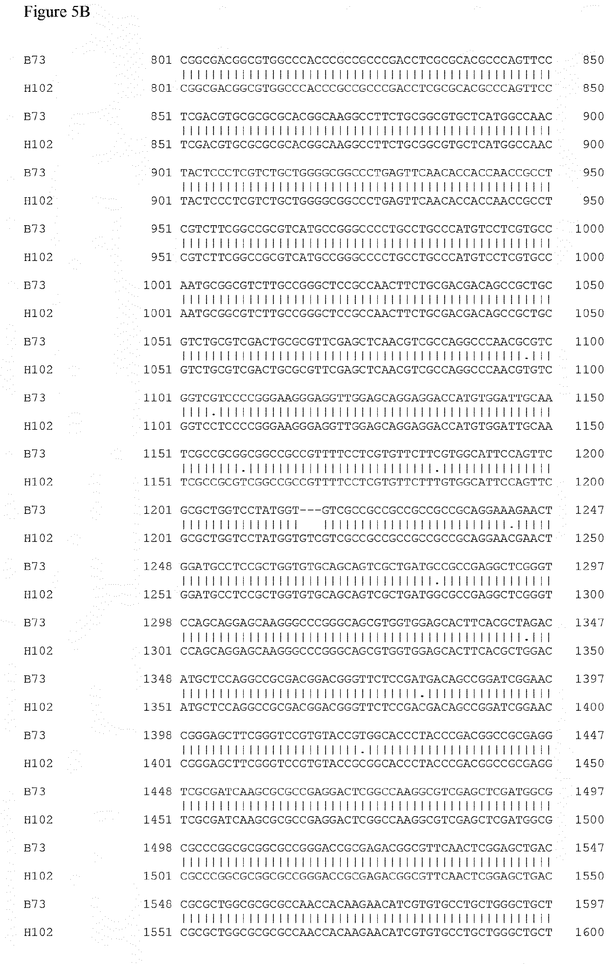

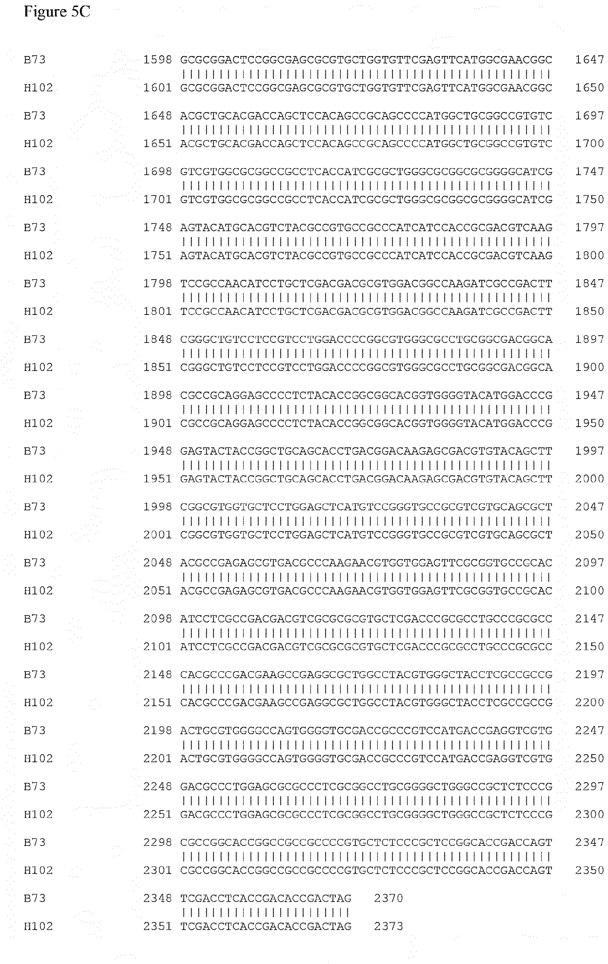

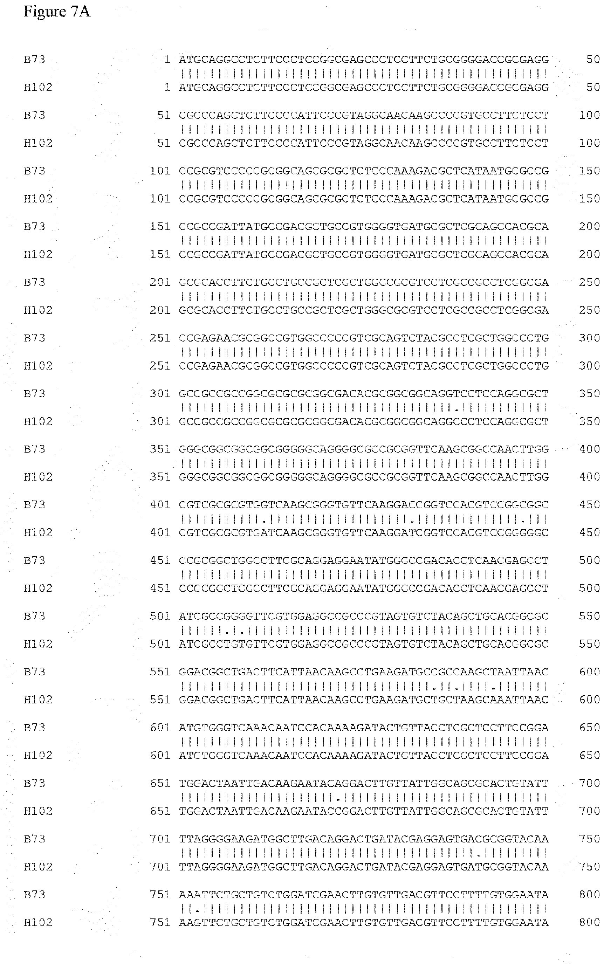

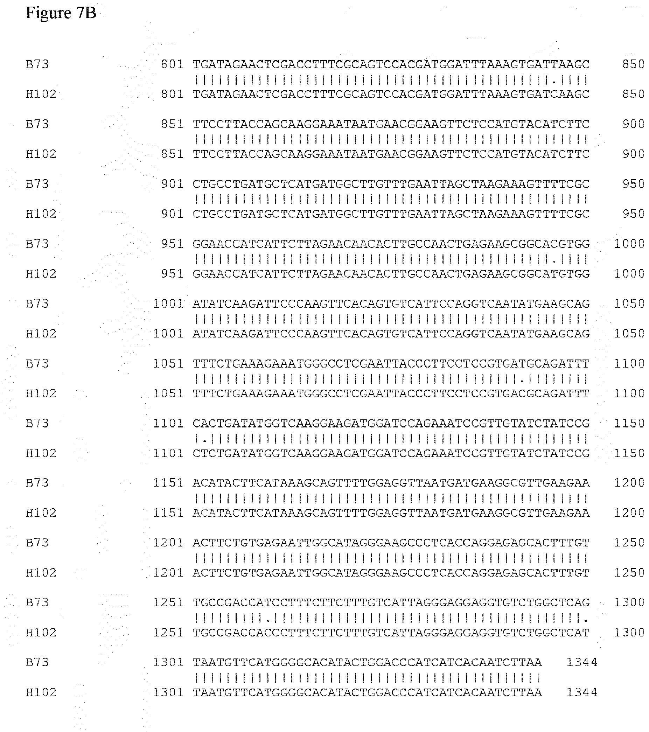

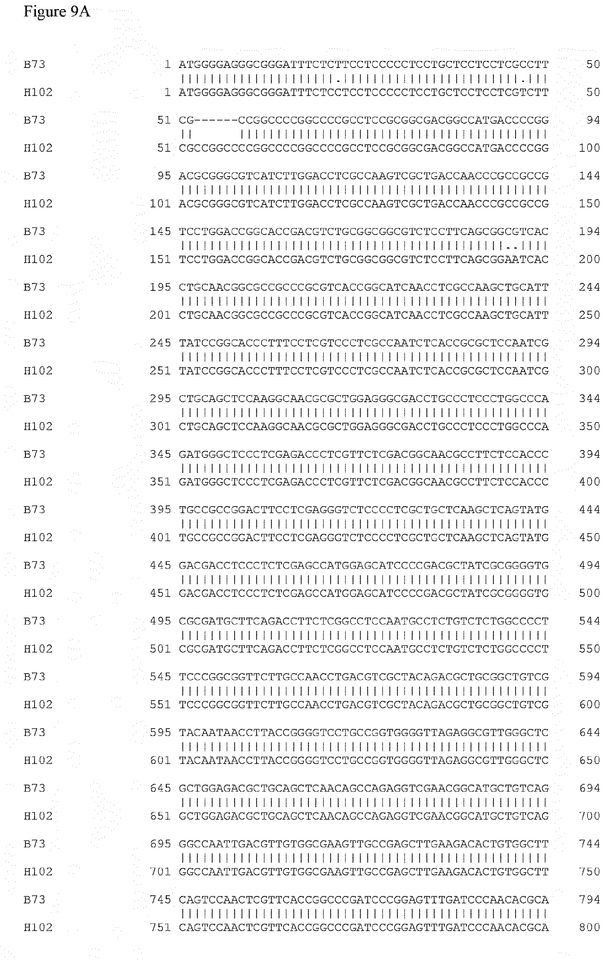

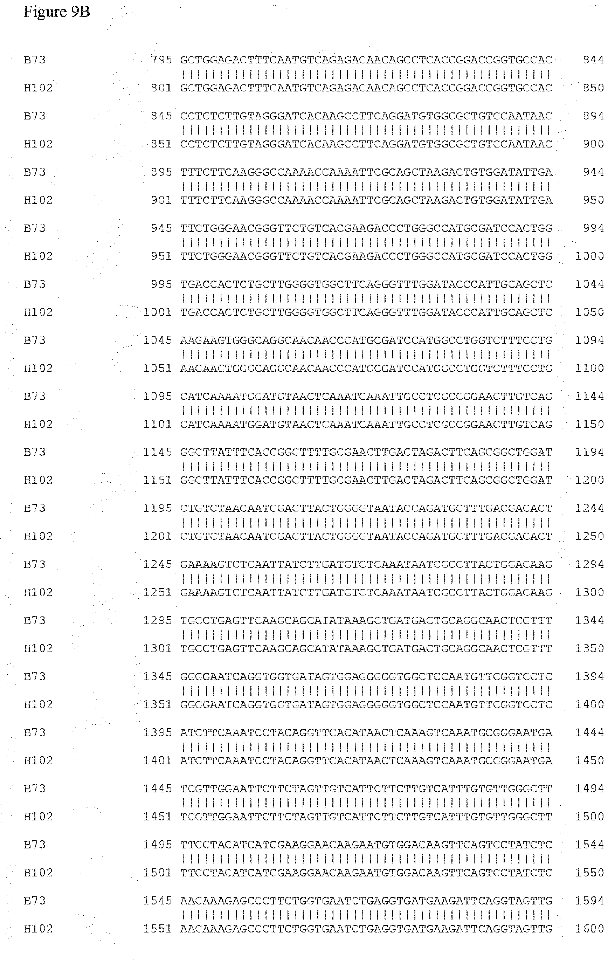

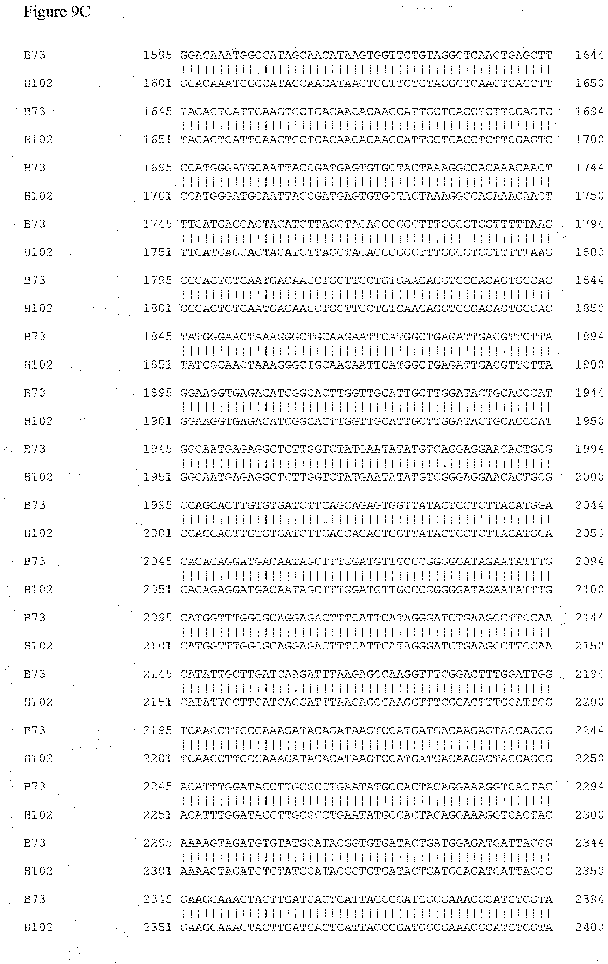

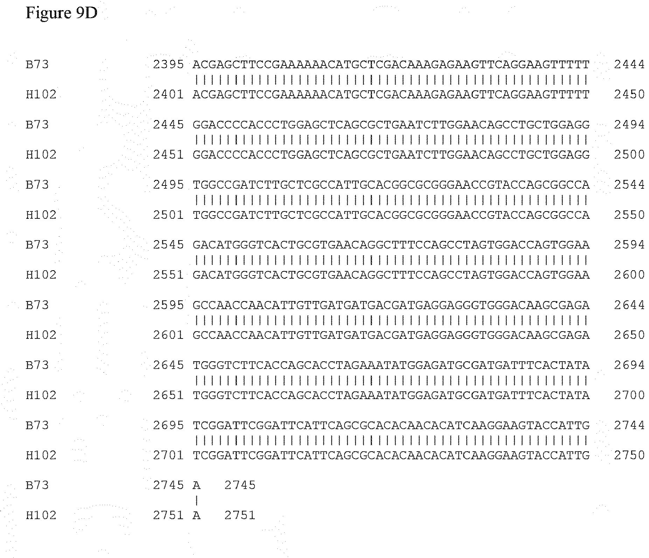

All other SNPs or InDels which can be derived from the sequence alignments of the cDNA (Figures 1, 3, 5, 7, and 9) might be used for the development of molecular markers for the detection of the individual genes and thus for detecting NCLB resistant corn plant carrying the HtM locus. All identified polymorphisms in the cDNAs of the selected genes are shown in the following Table 4.

**Table 4**

| **Gene identifier** | **Poly-morphism** | **H102 (donor) allele** | **B73 allele** | **Nucleotide position referenced to B73 allele** |
|---|---|---|---|---|
| Zm00001d049121 | SNP | T | G | 1071 |
| | SNP | G | A | 1302 |
| | SNP | C | A | 1497 |
| | SNP | G | A | 1631 |
| | SNP | C | T | 1691 |
| | InDel | - | A | 1807 |
| | InDel | GTACGTCCGAGTG ACCTCCTGCTCCT TGGAG | - | between 1815 and 1816 |
| | SNP | G | A | 2045 |
| | InDel | - | CAGGGA | 2424 - 2429 |
| | SNP | C | T | 2458 |
| | SNP | A | G | 2530 |
| | SNP | A | C | 2683 |
| Zm00001d049113 | SNP | C | A | 12 |
| | SNP | C | A | 291 |
| | SNP | T | A | 309 |
| | SNP | T | C | 324 |
| | InDel | - | GCT | 415 - 417 |
| Zm00001d049145 | SNP | G | C | 195 |
| | SNP | G | A | 247 |
| | SNP | C | A | 248 |
| | SNP | T | C | 1097 |
| | SNP | C | G | 1105 |
| | SNP | T | G | 1159 |
| | SNP | T | C | 1185 |
| | InDel | GTC | - | between 1216 and 1217 |
| | SNP | C | A | 1242 |
| | SNP | G | C | 1282 |
| | SNP | G | A | 1344 |
| | SNP | C | T | 1380 |
| | SNP | C | T | 1422 |
| Zm00001d049148 | SNP | C | T | 338 |
| | SNP | A | G | 412 |
| | SNP | T | C | 432 |
| | SNP | G | C | 447 |
| | SNP | T | G | 507 |
| | SNP | T | G | 509 |
| | SNP | T | C | 585 |
| | SNP | T | C | 588 |
| | SNP | A | T | 593 |
| | SNP | C | A | 672 |
| | SNP | T | C | 741 |
| | SNP | G | A | 753 |
| | SNP | C | T | 846 |
| | SNP | T | C | 996 |
| | SNP | C | T | 1092 |
| | SNP | T | A | 1102 |
| | SNP | C | T | 1260 |
| | SNP | T | G | 1300 |
| Zm00001d049159 | SNP | C | T | 22 |
| | SNP | T | C | 47 |
| | InDel | CCGGCC | - | between 52 and 53 |
| | SNP | A | C | 189 |
| | SNP | A | G | 190 |
| | SNP | G | A | 1980 |
| | SNP | G | C | 2014 |
| | SNP | G | A | 2160 |

## Claims

1. A method for identifying a maize plant or plant part, comprising screening for the presence of a QTL allele located on chromosome 4 in a maize plant or plant part, wherein said QTL allele is located on a chromosomal interval flanked by marker alleles A and B, wherein marker alleles A and B are SNPs which are respectively adenine (A) at the position 16107190 bp on reference genome B73 AGPv04 and cytosine (C) at the position 18470371 bp on reference genome B73 AGPv04.

2. The method according to claims 1, wherein said QTL allele comprises one or more of marker alleles M1 to M56, wherein
- M1 is a SNP which is thymine (T) at the position 1071 of the coding sequence of Zm00001d049121 referenced to the B73 reference genome AGPv4;
- M2 is a SNP which is guanine (G) at the position 1302 of the coding sequence of Zm00001d049121 referenced to the B73 reference genome AGPv4;
- M3 is a SNP which is cytosine (C) at the position 1497 of the coding sequence of Zm00001d049121 referenced to the B73 reference genome AGPv4;
- M4 is a SNP which is guanine (G) at the position 1631 of the coding sequence of Zm00001d049121 referenced to the B73 reference genome AGPv4;
- M5 is a SNP which is cytosine (C) at the position 1691 of the coding sequence of Zm00001d049121 referenced to the B73 reference genome AGPv4;
- M6 is an indel which is deletion of the nucleotide at the position 1807 of the coding sequence of Zm00001d049121 referenced to the B73 reference genome AGPv4;
- M7 is an indel which is an insertion of one or more nucleotides between nucleotides at positions 1815 and 1816 of the coding sequence of Zm00001d049121 referenced to the B73 reference genome AGPv4;
- M8 is a SNP which is guanine (G) at the position 2045 of the coding sequence of Zm00001d049121 referenced to the B73 reference genome AGPv4;
- M9 is an indel which is deletion of nucleotides at positions 2424-2429 of Zm00001d049121 referenced to the B73 reference genome AGPv4;
- M10 is a SNP which is cytosine (C) at the position 2458 of the coding sequence of Zm00001d049121 referenced to the B73 reference genome AGPv4;
- M11 is a SNP which is adenine (A) at the position 2530 of the coding sequence of Zm00001d049121 referenced to the B73 reference genome AGPv4;
- M12 is a SNP which is adenine (A) at the position 2683 of the coding sequence of Zm00001d049121 referenced to the B73 reference genome AGPv4;
- M13 is a SNP which is cytosine (C) at the position 12 of the coding sequence of Zm00001d049113 referenced to the B73 reference genome AGPv4;
- M14 is a SNP which is cytosine (C) at the position 291 of the coding sequence of Zm00001d049113 referenced to the B73 reference genome AGPv4;
- M15 is a SNP which is thymine (T) at the position 309 of the coding sequence of Zm00001d049113 referenced to the B73 reference genome AGPv4;
- M16 is a SNP which is thymine (T) at the position 324 of the coding sequence of Zm00001d049113 referenced to the B73 reference genome AGPv4;
- M17 is an indel which is deletion of nucleotides at positions 415-417 of the coding sequence of Zm00001d049113 referenced to the B73 reference genome AGPv4;
- M18 is a SNP which is guanine (G) at the position 195 of the coding sequence of Zm00001d049145 referenced to the B73 reference genome AGPv4;
- M19 is a SNP which is guanine (G) at the position 247 of the coding sequence of Zm00001d049145 referenced to the B73 reference genome AGPv4;
- M20 is a SNP which is cytosine (C) at the position 248 of the coding sequence of Zm00001d049145 referenced to the B73 reference genome AGPv4;
- M21 is a SNP which is thymine (T) at the position 1097 of the coding sequence of Zm00001d049145 referenced to the B73 reference genome AGPv4;
- M22 is a SNP which is cytosine (C) at the position 1105 of the coding sequence of Zm00001d049145 referenced to the B73 reference genome AGPv4;
- M23 is a SNP which is thymine (T) at the position 1159 of the coding sequence of Zm00001d049145 referenced to the B73 reference genome AGPv4;
- M24 is a SNP which is thymine (T) at the position 1185 of the coding sequence of Zm00001d049145 referenced to the B73 reference genome AGPv4;
- M25 is an indel which is an insertion of one or more nucleotides between nucleotides at positions 1216 and 1217 of the coding sequence of Zm00001d049145 referenced to the B73 reference genome AGPv4;
- M26 is a SNP which is cytosine (C) at the position 1242 of the coding sequence of Zm00001d049145 referenced to the B73 reference genome AGPv4;
- M27 is a SNP which is guanine (G) at the position 1282 of the coding sequence of Zm00001d049145 referenced to the B73 reference genome AGPv4;
- M28 is a SNP which is guanine (G) at the position 1344 of the coding sequence of Zm00001d049145 referenced to the B73 reference genome AGPv4;
- M29 is a SNP which is cytosine (C) at the position 1380 of the coding sequence of Zm00001d049145 referenced to the B73 reference genome AGPv4;
- M30 is a SNP which is cytosine (C) at the position 1422 of the coding sequence of Zm00001d049145 referenced to the B73 reference genome AGPv4;
- M31 is a SNP which is cytosine (C) at the position 338 of the coding sequence of Zm00001d049148 referenced to the B73 reference genome AGPv4;
- M32 is a SNP which is adenine (A) at the position 412 of the coding sequence of Zm00001d049148 referenced to the B73 reference genome AGPv4;
- M33 is a SNP which is thymine (T) at the position 432 of the coding sequence of Zm00001d049148 referenced to the B73 reference genome AGPv4;
- M34 is a SNP which is guanine (G) at the position 447 of the coding sequence of Zm00001d049148 referenced to the B73 reference genome AGPv4;
- M35 is a SNP which is thymine (T) at the position 507 of the coding sequence of Zm00001d049148 referenced to the B73 reference genome AGPv4;
- M36 is a SNP which is thymine (T) at the position 509 of the coding sequence of Zm00001d049148 referenced to the B73 reference genome AGPv4;
- M37 is a SNP which is thymine (T) at the position 585 of the coding sequence of Zm00001d049148 referenced to the B73 reference genome AGPv4;
- M38 is a SNP which is thymine (T) at the position 588 of the coding sequence of Zm00001d049148 referenced to the B73 reference genome AGPv4;
- M39 is a SNP which is adenine (A) at the position 593 of the coding sequence of Zm00001d049148 referenced to the B73 reference genome AGPv4;
- M40 is a SNP which is cytosine (C) at the position 672 of the coding sequence of Zm00001d049148 referenced to the B73 reference genome AGPv4;
- M41 is a SNP which is thymine (T) at the position 741 of the coding sequence of Zm00001d049148 referenced to the B73 reference genome AGPv4;
- M42 is a SNP which is guanine (G) at the position 753 of the coding sequence of Zm00001d049148 referenced to the B73 reference genome AGPv4;
- M43 is a SNP which is cytosine (C) at the position 846 of the coding sequence of Zm00001d049148 referenced to the B73 reference genome AGPv4;
- M44 is a SNP which is thymine (T) at the position 996 of the coding sequence of Zm00001d049148 referenced to the B73 reference genome AGPv4;
- M45 is a SNP which is cytosine (C) at the position 1092 of the coding sequence of Zm00001d049148 referenced to the B73 reference genome AGPv4;
- M46 is a SNP which is thymine (T) at the position 1102 of the coding sequence of Zm00001d049148 referenced to the B73 reference genome AGPv4;
- M47 is a SNP which is cytosine (C) at the position 1260 of the coding sequence of Zm00001d049148 referenced to the B73 reference genome AGPv4;
- M48 is a SNP which is thymine (T) at the position 1300 of the coding sequence of Zm00001d049148 referenced to the B73 reference genome AGPv4;
- M49 is a SNP which is cytosine (C) at the position 22 of the coding sequence of Zm00001d049159 referenced to the B73 reference genome AGPv4;
- M50 is a SNP which is thymine (T) at the position 47 of the coding sequence of Zm00001d049159 referenced to the B73 reference genome AGPv4;
- M51 is an indel which is an insertion of one or more nucleotides between nucleotides at positions 52 and 53 of the coding sequence of Zm00001d049159 referenced to the B73 reference genome AGPv4;
- M52 is a SNP which is adenine (A) at the position 189 of the coding sequence of Zm00001d049159 referenced to the B73 reference genome AGPv4;
- M53 is a SNP which is adenine (A) at the position 190 of the coding sequence of Zm00001d049159 referenced to the B73 reference genome AGPv4;
- M54 is a SNP which is guanine (G) at the position 1980 of the coding sequence of Zm00001d049159 referenced to the B73 reference genome AGPv4;
- M55 is a SNP which is guanine (G) at the position 2014 of the coding sequence of Zm00001d049159 referenced to the B73 reference genome AGPv4;
- M56 is a SNP which is guanine (G) at the position 2160 of the coding sequence of Zm00001d049159 referenced to the B73 reference genome AGPv4;
preferably wherein
- the B73 coding sequence of Zm00001d049121 comprises a sequence as set forth in SEQ ID NO: 11;
- the B73 coding sequence of Zm00001d049113 comprises a sequence as set forth in SEQ ID NO: 13;
- the B73 coding sequence of Zm00001d049145 comprises a sequence as set forth in SEQ ID NO: 15;
- the B73 coding sequence of Zm00001d049148 comprises a sequence as set forth in SEQ ID NO: 17; and/or
- the B73 coding sequence of Zm00001d049159 comprises a sequence as set forth in SEQ ID NO: 19.

3. The method according to any of claims 1 to 2, wherein screening for the presence of said QTL allele comprises identifying any one or more of molecular markers A or B and/or identifying any one or more of marker alleles selected from M1 to M56 as defined in claim 2.

4. A method for identifying a maize plant or plant part, comprising screening for the presence of one or more marker alleles selected from M1 to M56 as defined in claim 2.

5. The method according to any of claims 1 to 4, further comprising selecting a plant or plant part comprising said QTL allele or one or more of marker alleles M1 to M56 as defined in claim 2.

6. The method according to any of claims 1 to 5, wherein a plant or plant part is identified as having increased resistance to a pathogen if said QTL or one or more of said marker alleles are present.

7. A method for generating a maize plant, comprising introducing into the genome of a plant or a plant part
- a QTL allele as defined in any of claims 1 to 3;
- one or more marker allele selected from M1 to M56 as defined in claim 2;
- a polynucleic acid encoding exogenous Zm00001d049121 comprising one or more, preferably all, molecular marker M1 to M12; preferably having a coding sequence as set forth in SEQ ID NO: 1 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 1, preferably over the entire length of the sequence; or encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence;
- a polynucleic acid encoding exogenous Zm00001d049113 comprising one or more, preferably all, molecular marker M13 to M17; preferably having a sequence as set forth in SEQ ID NO: 3 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 3, preferably over the entire length of the sequence, or encoding a sequence as set forth in SEQ ID NO: 4or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence;
- a polynucleic acid encoding exogenous Zm00001d049145 comprising one or more, preferably all, molecular marker M18 to M30; preferably having a sequence as set forth in SEQ ID NO: 5 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 5, preferably over the entire length of the sequence, or encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence;
- a polynucleic acid encoding exogenous Zm00001d049148 comprising one or more, preferably all, molecular marker M31 to M48; preferably having a sequence as set forth in SEQ ID NO: 7 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 7, preferably over the entire length of the sequence, or encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence; and/or
- a polynucleic acid encoding exogenous Zm00001d049159 comprising one or more, preferably all, molecular marker M49 to M56; preferably having a sequence as set forth in SEQ ID NO: 9 or a coding sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 9, preferably over the entire length of the sequence, or encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence;
preferably wherein introducing into the genome comprises mutagenesis or transgenesis.

8. The method according to claim 7, comprising transforming a plant or plant part, preferably a plant cell or a plant tissue, more preferably a protoplast, a callus, an immature or mature embryo or an inflorescence, with the QTL allele as defined in claims 1 to 3 or the polynucleic acid as defined in claim 7, and optionally regenerating a plant or plant part from said plant cell or plant tissue, preferably said protoplast, said callus, said immature or mature embryo or said inflorescence.

9. The method according to any of claims 1 to 8, wherein said plant or plant part has increased resistance to a pathogen, preferably a fungal pathogen, more preferably wherein said pathogen is an *Exserohilum sp,* most preferably wherein said pathogen is *Exserohilum turcicum.*

10. A method for generating a maize plant, preferably a maize plant having increased pathogen resistance and/or tolerance, or a part thereof, preferably wherein said pathogen is *Exserohilum turcicum,* comprising
- mutating endogenous Zm00001d049121 to a sequence encoding a sequence as set forth in SEQ ID NO: 2 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 2, preferably over the entire length of the sequence;
- mutating endogenous Zm00001d049113 to a sequence encoding a sequence as set forth in SEQ ID NO: 4 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 4, preferably over the entire length of the sequence;
- mutating endogenous Zm00001d049145 to a sequence encoding a sequence as set forth in SEQ ID NO: 6 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 6, preferably over the entire length of the sequence;
- mutating endogenous Zm00001d049148 to a sequence encoding a sequence as set forth in SEQ ID NO: 8 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 8, preferably over the entire length of the sequence; and/or
- mutating endogenous Zm00001d049159 to a sequence encoding a sequence as set forth in SEQ ID NO: 10 or a sequence having an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence as set forth in SEQ ID NO: 10, preferably over the entire length of the sequence.

11. A maize plant comprising a QTL allele as defined in any of claims 1 to 3, one or more marker allele selected from M1 to M56 as defined in claim 2, one or more polynucleic acid having a coding sequence selected from SEQ ID NOs: 1, 3, 5, 7, and 9, one or more polynucleic acid having a coding sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 1, 3, 5, 7, and 9, preferably over the entire length of the sequence, one or more polynucleic acid encoding a sequence selected from SEQ ID NOs: 2, 4, 6, 8, and 10 and/or one or more polynucleic acid encoding a sequence with an identity of at least 98.5%, 98.6%, 98.7%, 98.8%, 98.9%, 99%, 99.1%, 99.2%, 99.3%, 99.4%, 99.5%, 99.6%, 99.7%, 99.8% or 99.9% to a sequence selected from SEQ ID NOs: 2, 4, 6, 8, and 10, preferably over the entire length of the sequence, or a plant part thereof, or the progeny thereof.

12. The maize plant or plant part according to claim 11, which is transgenic, gene-edited, or mutagenized.

13. An isolated polynucleic acid comprising a coding sequence selected from SEQ ID NOs: 1, 3, 5, 7, and 9 or which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 1, 3, 5, 7, and 9; or which encodes a polypeptide selected from SEQ ID NOs: 2, 4, 6, 8, and 10 or a polypeptide which is at least 98.5%, 98.6%, 98.7%, 98.8%, 98.9%, 99%, 99.1%, 99.2%, 99.3%, 99.4%, 99.5%, 99.6%, 99.7%, 99.8% or 99.9% identical, preferably over its entire length, to a sequence of any of SEQ ID NOs: 2, 4, 6, 8, and 10.

14. The isolated polynucleic acid according to claim 13, wherein said polynucleic acid comprises
- one or more molecular marker selected from M1 to M12;
- one or more molecular marker selected from M13 to M17;
- one or more molecular marker selected from M18 to M30;
- one or more molecular marker selected from M31 to M48; and/or
- one or more molecular marker selected from M49 to M56.

15. An isolated polynucleic acid specifically hybridizing with the polynucleic acid of claim 13 or 14, the complement thereof, or the reverse complement thereof, preferably wherein said polynucleic acid is a primer or a probe.
